(19) Europäisches Patentamt
European Patent Office
Office européen des brevets

(11) EP 4 317 160 A1

(12) EUROPEAN PATENT APPLICATION
published in accordance with Art. 153(4) EPC

(43) Date of publication:
**07.02.2024 Bulletin 2024/06**

(21) Application number: **22781209.6**

(22) Date of filing: **31.03.2022**

(51) International Patent Classification (IPC):
***C07D 471/04** (2006.01)* ***A01P 7/04** (2006.01)*
***C07D 487/04** (2006.01)* ***A01N 43/90** (2006.01)*
***A01N 47/02** (2006.01)*

(52) Cooperative Patent Classification (CPC):
**A01N 43/90; A01N 47/02; A01P 7/04; C07D 471/04; C07D 487/04**

(86) International application number:
**PCT/JP2022/016390**

(87) International publication number:
**WO 2022/210999 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2021 JP 2021063247**

(71) Applicant: **Kumiai Chemical Industry Co., Ltd.**
**Taito-ku**
**Tokyo 110-0008 (JP)**

(72) Inventors:
- **ARISUE Kaoru**
**Tokyo 110-0008 (JP)**
- **YASHIRO Kazuki**
**Tokyo 110-0008 (JP)**
- **KOIKE Shusuke**
**Tokyo 110-0008 (JP)**
- **MASUI Noboru**
**Tokyo 110-0008 (JP)**

(74) Representative: **Comoglio, Elena et al**
**Jacobacci & Partners S.p.A.**
**Corso Emilia 8**
**10152 Torino (IT)**

# (54) HETEROCYCLIC COMPOUND AND USE THEREOF

(57) The present invention provides heterocyclic compounds or salts thereof having excellent pest control effects, pest control agents containing the same as active ingredients, and production intermediates thereof.

The present invention provides heterocyclic compounds represented by the general formula [I]

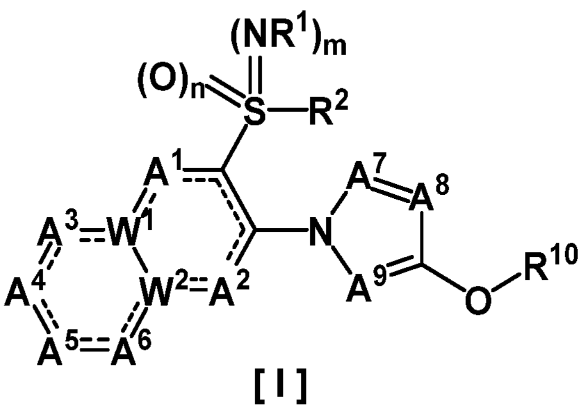

[ I ]

(wherein, $R^1$ represents a hydrogen atom, cyano group, or $C_1$-$C_7$ acyl group which is unsubstituted or substituted with a halogen atom, $R^2$ represents a $C_1$-$C_6$ alkyl group, etc., each of $A^1$ and $A^2$ independently represents a nitrogen atom, CH or bond, either $W^1$ or $W^2$ represents a nitrogen atom, and the other represents a carbon atom, each of $A^3$, $A^4$, $A^5$, $A^6$, $A^7$, $A^8$, and $A^9$ independently represents a nitrogen atom or a carbon atom substituted with $R^3$, $R^4$, $R^5$, $R^6$, $R^7$, $R^8$,

and $R^9$, respectively, each of $R^3$, $R^4$, $R^5$, $R^6$, $R^7$, $R^8$, and $R^9$ represents hydrogen atoms, $C_1$-$C_6$ haloalkyl groups, etc., $R^{10}$ represents a hydrogen atom, $C_1$-$C_{12}$ alkyl group, $C_1$-$C_6$ alkoxy $C_1$-$C_6$ alkyl group, $C_1$-$C_{12}$ haloalkyl group, etc., and $R^{11}$ represents a halogen atom, azido group, cyano group, etc.) or pesticide-acceptable salts thereof, and pest control agents characterized by containing the same as active ingredients.

## Description

### Technical field

The present invention relates to pest control agents containing novel heterocyclic compounds or salts thereof, derivatives or salts thereof as active ingredients and production intermediates thereof.

### Technical background

Compounds having insecticidal activity are described in patent document 1.
Compounds having insecticidal activity and coupled with monocyclic groups are described in patent document 2.
Compounds having insecticidal activity and coupled with monocyclic groups by nitrogen-carbon bonds are described in patent document 3.
Diarylazole compounds having insecticidal activity are described in patent document 4.
Alkoxypyrazole compounds having insecticidal activity are described in patent document 5.
Fused ring compounds wherein a sulfur-containing substituent is coupled at a position adjacent to a nitrogen-containing five-membered ring bonded by a nitrogen-carbon bond are described in patent document 6. However, there is no description regarding insecticidal activity.
In the above patent documents, there is no disclosure of the heterocyclic compounds of the present invention.

### Prior art documents

### Patent documents

1. International patent publication No. 2017/061497
2. International patent publication No. 2018/052119
3. International patent publication No. 2019/124548
4. International patent publication No. 2016/204270
5. Japanese patent publication No. 2021-024859
6. International patent publication No. 03/048081


### Summary of the invention

### Problems to be solved by the invention

The pest control agents used for useful crops are expected to exhibit sufficient pest control effect at a low dose when applied to soil or foliage. Besides, since there are increasing demands for the safety of chemical substances and their impact on the environment, development of safer pest control agents is expected. Furthermore, in recent years, due to long term use of pest control agents such as insecticides, acaricides, and nematicides, pests that have acquired resistance to the pest control agents have emerged. Therefore, it becomes difficult to control pests completely. In addition, the use of pest control agents having high toxicity to humans and animals is also a problem in terms of safety for workers and the like.
The subject of the present invention is to solve the above-mentioned problems of the conventional pest control agents under such circumstances, and further, to provide pest control agents having excellent safety, control effect, residual effects and the like, and novel compounds for the purpose.

### Means for solving problems

To develop the pest control agents having the above-mentioned preferable properties, the present inventors have synthesized various heterocyclic compounds and diligently investigated their physiological activities. As a result, it was found out that the heterocyclic compounds represented by the following general formula [I] (hereinafter, also referred to as the compounds of the present invention) have excellent control effects on various pests. The present inventors continued further research and completed the present invention.
That is, the present invention relates to the following aspects.

(1) A compound represented by the general formula [I] or salt thereof, wherein,

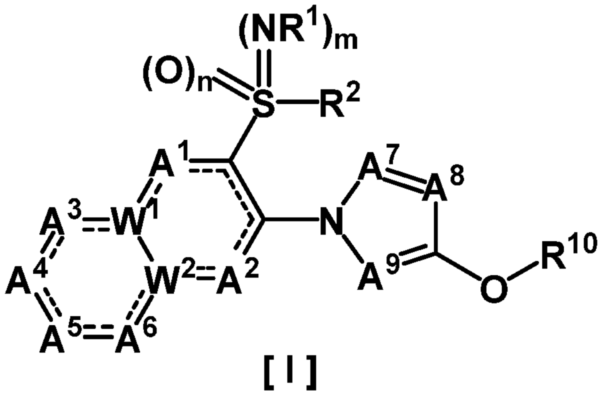

[ I ]

each of m and n independently represents an integer of 0, 1 or 2, and the sum thereof is an integer in the range of 0 to 2,

$R^1$ represents a hydrogen atom, cyano group, or $C_1$-$C_7$ acyl group which is unsubstituted or substituted with a halogen atom,

$R^2$ represents a $C_1$-$C_6$ alkyl group, $C_1$-$C_6$ alkoxy $C_1$-$C_6$ alkyl group, $C_1$-$C_6$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_3$-$C_6$ cycloalkyl group, $C_3$-$C_6$ halocycloalkyl group, $C_3$-$C_6$ cycloalkyl $C_1$-$C_6$ alkyl group, $C_3$-$C_6$ halocycloalkyl $C_1$-$C_6$ alkyl group, cyano $C_1$-$C_6$ alkyl group, cyano $C_3$-$C_6$ cycloalkyl group, $C_7$-$C_{14}$ aralkyl group which is unsubstituted or optionally substituted with $R^{11}$, or $C_6$-$C_{14}$ heteroaralkyl group which is unsubstituted or optionally substituted with $R^{11}$,

$W^1$ represents a nitrogen atom,

$W^2$ represents a carbon atom, or

$W^1$ represents a carbon atom,

$W^2$ represents a nitrogen atom,

$A^1$ represents a nitrogen atom, a carbon atom substituted with $R^a$, or a bond,

$A^2$ represents a nitrogen atom, a carbon atom substituted with $R^b$, or a bond,

$A^3$ represents a nitrogen atom, a carbon atom substituted with $R^3$, or a bond,

any one of $A^1$, $A^2$ or $A^3$ is a bond,

each of $A^4$, $A^5$, $A^6$, $A^7$, $A^8$ and $A^9$ independently represents a nitrogen atom, or a carbon atom substituted with $R^4$, $R^5$, $R^6$, $R^7$, $R^8$ and $R^9$, respectively,

each of $R^a$, $R^b$, $R^3$, $R^4$, $R^5$, $R^6$, $R^7$, $R^8$ and $R^9$ independently represents a hydrogen atom, halogen atom, azido group, cyano group, nitro group, $C_1$-$C_6$ alkyl group, cyano $C_1$-$C_6$ alkyl group, hydroxy $C_1$-$C_6$ alkyl group, $C_1$-$C_6$ haloalkyl group, $C_2$-$C_6$ alkenyl group, $C_2$-$C_6$ alkynyl group, $C_3$-$C_6$ cycloalkyl group, cyano $C_3$-$C_6$ cycloalkyl group, hydroxy $C_3$-$C_6$ cycloalkyl group, $C_3$-$C_6$ halocycloalkyl group, $C_3$-$C_6$ cycloalkenyl group, $C_3$-$C_6$ cycloalkyl $C_1$-$C_6$ alkyl group, $C_3$-$C_6$ halocycloalkyl $C_1$-$C_6$ alkyl group, $C_3$-$C_6$ cycloalkyl $C_2$-$C_6$ alkenyl group, $C_3$-$C_6$ cycloalkyl $C_2$-$C_6$ alkynyl group, heterocyclic group which is unsubstituted or optionally substituted with $R^{11}$, $C_6$-$C_{12}$ aryl group which is unsubstituted or optionally substituted with $R^{11}$, $C_5$-$C_{12}$ heteroaryl group which is unsubstituted or optionally substituted with $R^{11}$, $C_7$-$C_{14}$ aralkyl group which is unsubstituted or optionally substituted with $R^{11}$, $C_6$-$C_{14}$ heteroaralkyl group which is unsubstituted or optionally substituted with $R^{11}$, hydroxy group, $C_1$-$C_6$ alkoxy group, $C_1$-$C_6$ haloalkoxy group, cyano $C_1$-$C_6$ alkoxy group, hydroxy $C_1$-$C_6$ alkoxy group, $C_1$-$C_6$ haloalkoxy $C_1$-$C_6$ alkoxy group, $C_1$-$C_6$ alkoxy $C_1$-$C_6$ haloalkoxy group, $C_1$-$C_6$ haloalkoxy $C_1$-$C_6$ haloalkoxy group, $C_3$-$C_6$ cycloalkyloxy group, $C_3$-$C_6$ cycloalkyl $C_1$-$C_6$ alkoxy group, $C_3$-$C_6$ halocycloalkyloxy group, cyano $C_3$-$C_6$ cycloalkyloxy group, $C_3$-$C_6$ halocycloalkyl $C_1$-$C_6$ alkoxy group, (cyano $C_3$-$C_6$ cycloalkyl) $C_1$-$C_6$ alkoxy group, $C_1$-$C_6$ haloalkylthio $C_1$-$C_6$ alkoxy group, $C_1$-$C_6$ haloalkylsulfinyl $C_1$-$C_6$ alkoxy group, $C_1$-$C_6$ haloalkylsulfonyl $C_1$-$C_6$ alkoxy group, $C_7$-$C_{14}$ aralkyloxy group which is unsubstituted or optionally substituted with $R^{11}$, $C_6$-$C_{14}$ heteroaralkyloxy group which is unsubstituted or optionally substituted with $R^{11}$, $C_6$-$C_{12}$ aryloxy group which is unsubstituted or optionally substituted with $R^{11}$, $C_5$-$C_{12}$ heteroaryloxy group which is unsubstituted or optionally substituted with $R^{11}$, sulfanyl group, pentahalosulfanyl group, $C_1$-$C_6$ alkylthio group, $C_1$-$C_6$ alkylsulfinyl group, $C_1$-$C_6$ alkylsulfonyl group, $C_1$-$C_6$ haloalkylthio group, $C_1$-$C_6$ haloalkylsulfinyl group, $C_1$-$C_6$ haloalkylsulfonyl group, $C_3$-$C_6$ cycloalkylthio group, $C_3$-$C_6$ cycloalkylsulfinyl group, $C_3$-$C_6$ cycloalkylsulfonyl group, $C_3$-$C_6$ halocycloalkylthio group, $C_3$-$C_6$ halocycloalkylsulfinyl group, $C_3$-$C_6$ halocycloalkylsulfonyl group, $C_3$-$C_6$ cycloalkyl $C_1$-$C_6$ alkylthio group, $C_3$-$C_6$ cycloalkyl $C_1$-$C_6$ alkylsulfinyl group, $C_3$-$C_6$ cycloalkyl $C_1$-$C_6$ alkylsulfonyl group, $C_3$-$C_6$ halocycloalkyl $C_1$-$C_6$ alkylthio group, $C_3$-$C_6$ halocycloalkyl $C_1$-$C_6$ alkylsulfinyl group, $C_3$-$C_6$ halocycloalkyl $C_1$-$C_6$ alkylsulfonyl group, group-S ($R^{12}$) = NH, group-S ($R^{12}$) = N-C≡N, group-S ($R^{12}$) = N-C (= O) $R^{13}$, group-S ($R^{12}$) (= O) = NH, group-S ($R^{12}$) (= O) = N-C≡N, group-S ($R^{12}$) (= O) = N-C (= O) $R^{13}$, $C_6$-$C_{12}$ arylthio group,

$C_6$-$C_{12}$ arylsulfinyl group, $C_6$-$C_{12}$ arylsulfonyl group, $C_5$-$C_{12}$ heteroarylthio group, $C_5$-$C_{12}$ heteroarylsulfinyl group, $C_5$-$Cl_2$ heteroarylsulfonyl group, amino group, mono($C_1$-$C_6$ alkyl)amino group, di($C_1$-$C_6$ alkyl)amino group, (di($C_1$-$C_6$ alkyl)sulfinylidene)amino group, di ($C_1$-$C_6$ alkyl) phosphoryl group, $C_1$-$C_7$ acyl group, $C_1$-$C_7$ haloacyl group, hydroxyimino $C_1$-$C_6$ alkyl group, hydroxyimino(amino) $C_1$-$C_7$ alkyl group, $C_1$-$C_6$ alkoxyimino $C_1$-$C_6$ alkyl group, $C_1$-$C_6$ haloalkoxyimino $C_1$-$C_6$ alkyl group, carboxyl group, $C_1$-$C_6$ alkoxycarbonyl group, aminocarbonyl group, mono($C_1$-$C_6$ alkyl)aminocarbonyl group, di($C_1$-$C_6$ alkyl)aminocarbonyl group, mono($C_1$-$C_6$ haloalkyl)aminocarbonyl group, mono($C_3$-$C_6$ cycloalkyl)aminocarbonyl group, mono (cyano $C_3$-$C_6$ cycloalkyl) aminocarbonyl group, mono ($C_1$-$C_6$ alkoxyimino $C_1$-$C_6$ alkyl) aminocarbonyl group, di ($C_1$-$C_6$ alkyl) aminomethylidene aminocarbonyl group, aminothiocarbonyl group, mono($C_1$-$C_6$ alkyl)aminothiocarbonyl group, di($C_1$-$C_6$ alkyl)aminothiocarbonyl group, or N-($C_1$-$C_6$ alkoxy)-N-($C_1$-$C_6$ alkyl)aminocarbonyl group,

$R^{10}$ represents a hydrogen atom, $C_1$-$C_{12}$ alkyl group, $C_1$-$C_6$ alkoxy $C_1$-$C_6$ alkyl group, $C_1$-$C_{12}$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_2$-$C_6$ alkenyl group, $C_2$-$C_6$ haloalkenyl group, $C_2$-$C_6$ alkynyl group, $C_3$-$C_6$ cycloalkyl $C_2$-$C_6$ alkynyl group, $C_2$-$C_6$ haloalkynyl group, $C_3$-$C_6$ cycloalkyl group, $C_3$-$C_6$halocycloalkyl group, $C_3$-$C_6$ cycloalkyl $C_1$-$C_6$ alkyl group, $C_3$-$C_6$ halocycloalkyl $C_1$-$C_6$ alkyl group, heterocyclyl $C_1$-$C_6$ alkyl group which is unsubstituted or optionally substituted with $R^{11}$, $C_7$-$C_{14}$ aralkyl group which is unsubstituted or optionally substituted with $R^{11}$, $C_6$-$C_{14}$ heteroaralkyl group which is unsubstituted or optionally substituted with $R^{11}$, $C_6$-$C_{12}$ aryl group which is unsubstituted or optionally substituted with $R^{11}$, or $C_5$-$C_{12}$ heteroaryl group which is unsubstituted or optionally substituted with R 11, and

R 11 represents a halogen atom, azido group, cyano group, nitro group, $C_1$-$C_6$ alkyl group, $C_1$-$C_6$ haloalkyl group, $C_1$-$C_6$ alkoxy group, $C_1$-$C_6$ haloalkoxy group, $C_3$-$C_6$ cycloalkyl group, pentahalosulfanyl group, $C_1$-$C_6$ alkylthio group, $C_1$-$C_6$ alkylsulfinyl group, $C_1$-$C_6$ alkylsulfonyl group, $C_1$-$C_6$ haloalkylthio group, $C_1$-$C_6$ haloalkylsulfinyl group, $C_1$-$C_6$ haloalkylsulfonyl group, amino group, mono($C_1$-$C_6$ alkyl)amino group, or di($C_1$-$C_6$ alkyl)amino group,

$R^{12}$ and $R^{13}$ independently represent a $C_1$-$C_6$ alkyl group, $C_1$-$C_6$ alkoxy $C_1$-$C_6$ alkyl group, $C_1$-$C_6$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_3$-$C_6$ cycloalkyl group, $C_3$-$C_6$ halocycloalkyl group, $C_3$-$C_6$ cycloalkyl $C_1$-$C_6$ alkyl group, $C_3$-$C_6$ halocycloalkyl $C_1$-$C_6$ alkyl group, cyano $C_1$-$C_6$ alkyl group, $C_7$-$C_{14}$ aralkyl group which is unsubstituted or optionally substituted by $R^{11}$, or $C_6$-$C_{14}$ heteroaralkyl group which ais unsubstituted or optionally substituted by $R^{11}$.(2) The compound or salt thereof according to (1) described above, wherein $A^7$ represents a nitrogen atom, and each of $A^8$ and $A^9$ represents a carbon atom substituted with $R^8$ and $R^9$, respectively.

(3) The compound or salt thereof according to (1) described above, wherein $A^1$ represents a bond, and each of $A^2$ and $A^7$ represents a nitrogen atom,

$W^1$ represents a nitrogen atom,
$W^2$ represents a carbon atom,
each of $A^3$, $A^4$, $A^5$, $A^6$, $A^8$, and $A^9$ represents a carbon atom substituted with $R^3$, $R^4$, $R^5$, $R^6$, $R^8$ and $R^9$, respectively.

(4) The compound or salt thereof according to (3) described above, wherein each of $R^3$, $R^4$, $R^5$, $R^6$, $R^8$ and $R^9$ independently represents a hydrogen atom, halogen atom, $C_1$-$C_6$ alkyl group, $C_1$-$C_6$ haloalkyl group, cyano $C_3$-$C_6$ cycloalkyl group, $C_1$-$C_6$ haloalkoxy group, or cyano $C_1$-$C_6$ alkoxy group.
(5) The compound or salt thereof according to any one of (1) to (4) described above, wherein $R^2$ represents a $C_1$-$C_6$ alkyl group.
(6) The compound or salt thereof according to any one of (1) to (5) described above, wherein $R^{10}$ represents a $C_1$-$C_{12}$ haloalkyl group.
(7) The compound or salt thereof according to (1) described above, wherein the compound represented by the general formula [I] is a compound represented by the general formula [Ia], wherein

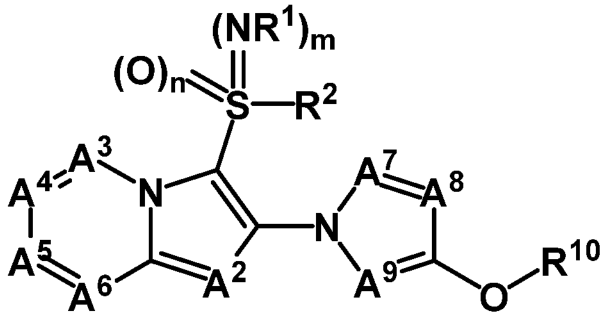

[ Ia ]

$A^2$, $A^3$, $A^4$, $A^5$, $A^6$, $A^7$, $A^8$, $A^9$, m, n, $R^1$, $R^2$, and $R^{10}$ have the same meaning as in (1) described above.

(8) The compound or salt thereof according to (1) described above, wherein the compound represented by the general formula [I] is a compound represented by the general formula (A), wherein

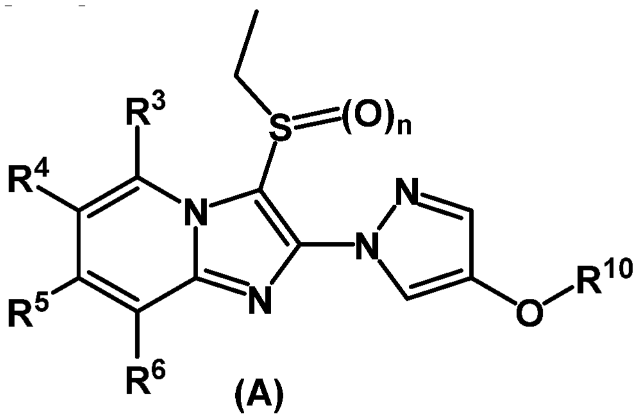

(A)

n, $R^3$, $R^4$, $R^5$, $R^6$ and $R^{10}$ have the same meaning as in (1) described above.

(9) The compound or salt thereof according to (8) described above, wherein n is 2, each of $R^3$, $R^5$ and $R^6$ is a hydrogen atom, $R^4$ is a $C_1$-$C_6$ haloalkyl group, cyano $C_3$-$C_6$ cycloalkyl group, $C_1$-$C_6$ haloalkoxy group, or cyano $C_1$-$C_6$ alkoxy group, and $R^{10}$ is a $C_1$-$C_{12}$ haloalkyl group.

(10) The compound or salt thereof according to (1) described above, wherein the compound represented by the general formula [1] is a compound represented by the general formula (B),

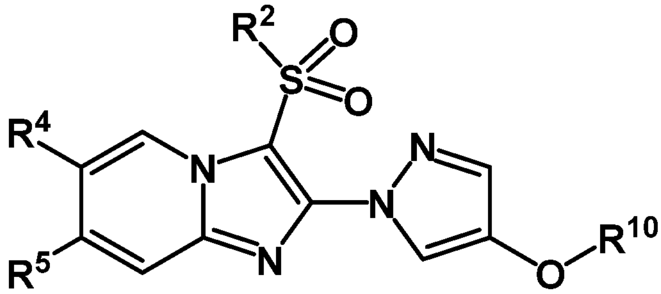

(B)

wherein $R^2$, $R^4$, $R^5$ and $R^{10}$ have the same meaning as in (1) described above.

(11) The compound or salt thereof according to (1) described above, wherein the compound represented by the general formula [1] is a compound represented by the general formula (C),

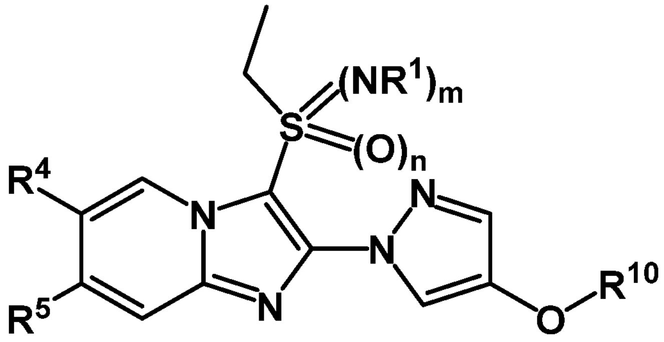

**(C)**

wherein m, n, $R^1$, $R^4$, $R^5$ and $R^{10}$ have the same meaning as in (1) described above. (12) The compound or salt thereof according to (1) described above, wherein the compound represented by the general formula [1] is a compound represented by the general formula (D),

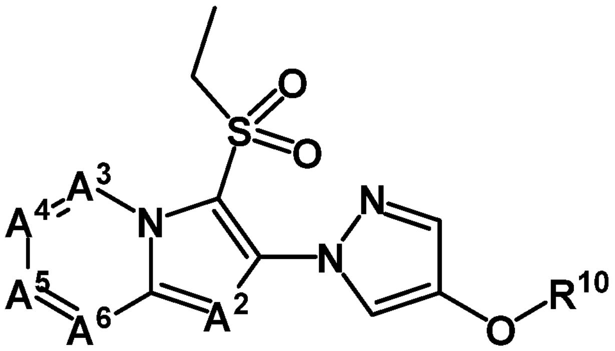

**(D)**

wherein $A^2$, $A^3$, $A^4$, $A^5$, $A^6$ and $R^{10}$ have the same meaning as in (1) described above.

(13) The compound or salt thereof according to (12) described above, wherein $A^2$ represents a nitrogen atom or a carbon atom substituted with $R^b$, $A^3$ represents a nitrogen atom or a carbon atom substituted with $R^3$, and each of $A^4$, $A^5$ and $A^6$ independently represents a nitrogen atom or a carbon atom substituted with $R^4$, $R^5$, and $R^6$, respectively.
(14) A pesticide composition comprising the compound or salt thereof according to any one of (1) to (13) described above as an active ingredient.
(15) The pesticide composition according to (14) described above, wherein the pesticide composition further comprises a surfactant.
(16) A pest control agent comprising the compound or salt thereof according to any one of (1) to (13) described above as an active ingredient.
(17) The pest control agent according to (16) described above, wherein the pest control agent is an insecticide, a nematicide, or an acaricide.
(18) The pest control agent according to (17) described above, which has control effects against pests in paddy fields, farmland, turf, orchards, non-agricultural land, greenhouses, nursery facilities, and plant factories for cultivating agricultural and horticultural plants.
(19) The pest control agent according to (18) described above, wherein the agricultural and horticultural plants are plants to which resistance has been given by breeding methods or gene recombination techniques.
(20) A method for controlling pests using an effective amount of the compound or salt thereof according to any one of (1) to (13) described above.
(21) A method for controlling pests by allowing a pesticide composition comprising the compound or salt thereof according to any one of (1) to (13) described above as an active ingredient to act at the same time or in a divided manner on places where agricultural or horticultural crops are to be grown or growing.

(22) The method for controlling pests according to (20) or (21) described above, wherein the places where the pest control agents are applied are paddy fields, farmland, turf, orchards, non-agricultural land, greenhouses, nursery facilities, or plant factories.
(23) The method for controlling pests according to any one of (20) to (22) described above, wherein the compound or salt thereof according to any one of (1) to (13) described above is used as an insecticide, a nematicide, or an acaricide.
(24) A use of the pest control agent according to any one of (16) to (19) described above for controlling pests on agricultural and horticultural crops.
(25) The compound represented by the general formula [I] or salt thereof according to (1) described above, wherein $R^{10}$ is a hydrogen atom in the general formula [I].
(26) A method for producing the compound represented by the general formula [I] according to (1) described above, comprising using the compound or salt thereof according to (25) described above as an intermediate.

**Effects of the invention**

The pest control agents containing the compounds of the present invention can exhibit excellent control effects on a wide range of pests such as hemiptera pests, lepidoptera pests, coleoptera pests, diptera pests, hymenoptera pests, orthoptera pests, isoptera pests, thysanoptera pests, mites, and plant-parasitic nematodes, and can also control the pests that have acquired pesticide resistance.

**Embodiments for carrying out the invention**

The symbols and terms described herein will be described.
In the present invention, the "pest control agent" means insecticides, nematicides, or acaricides used in agricultural or horticultural fields and animals such as livestocks and pets for household or epidemic prevention.
In the present invention, the "halogen atom" refers to a fluorine atom, chlorine atom, bromine atom, and iodine atom.
In the present invention, the notations such as "$C_1$-$C_6$" indicate that the numbers following the element symbols represent the number of carbon atoms. For example, the number of carbon atoms may be in the range of 1 to 6 in this case.
In the present invention, the "$C_1$-$C_2$ alkyl group" refers to a linear alkyl group having 1 to 2 carbon atoms, unless otherwise specified. For example, methyl and ethyl groups, and the like can be mentioned..
In the present invention, the "$C_1$-$C_6$ alkyl group" refers to a linear or branched alkyl group having 1 to 6 carbon atoms, unless otherwise specified. For example, in addition to the examples in the above-mentioned "$C_1$-$C_2$ alkyl group", n-propyl, isopropyl, n-butyl, sec-butyl, isobutyl, tert-butyl, n-pentyl, 1-methylbutyl, 2-methylbutyl, 3-methylbutyl, 1-ethylpropyl, 1,1-dimethylpropyl, 1,2-dimethylpropyl, neopentyl, n-hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethyl-1-methylpropyl, 1-ethyl-2-methylpropyl groups, and the like can be mentioned.
In the present invention, the "$C_1$-$C_{12}$ alkyl group" refers to a linear or branched alkyl group having 1 to 12 carbon atoms, unless otherwise specified. For example, in addition to the examples in the above-mentioned "$C_1$-$C_6$ alkyl group", heptyl, 1-methylhexyl, 2-methylhexyl, 3-methylhexyl, 4-methylhexyl, isoheptyl, 1,1-dimethylpentyl, 2,2-dimethylpentyl, 4,4-dimethylpentyl, 1-ethylpentyl, 2-ethylpentyl, 1,1,3-trimethylbutyl, 1,2,2-trimethylbutyl, 1,3,3-trimethylbutyl, 2,2,3-trimethylbutyl, 2,3,3-trimethylbutyl, 1-propylbutyl, 1,1,2,2-tetramethylpropyl, octyl, 1-methylheptyl, 3-methylheptyl, isooctyl, 2-ethylhexyl, 5,5-dimethylhexyl, 2,4,4-trimethylpentyl, 1-ethyl-1-methylpentyl, 1-propylpentyl, nonyl, 1-methyloctyl, 2-methyloctyl, 3-methyloctyl, isononyl, 1-ethylheptyl, 1,1-dimethylheptyl, 6,6-dimethylheptyl, decyl, 1-methylnonyl, 2-methylnonyl, 6-methylnonyl, isodecyl, 1-ethyloctyl, 1-propylheptyl, undecyl, 1-methyldecyl, isoundecyl, dodecyl, 1-methylundecyl, isododecyl groups, and the like can be mentioned.
In the present invention, the "$C_1$-$C_6$ haloalkyl group" refers to a linear or branched haloalkyl group having 1 to 6 carbon atoms substituted with 1 to 13 same or different halogen atoms, unless otherwise specified. For example, fluoromethyl, difluoromethyl, trifluoromethyl, chloromethyl, dichloromethyl, trichloromethyl, bromomethyl, dibromomethyl, tribromomethyl, iodomethyl, chlorodifluoromethyl, dichlorofluoromethyl, 1-fluoroethyl, 2-fluoroethyl, 1,1-difluoroethyl, 2,2-difluoroethyl, 2,2,2-trifluoroethyl, 1,1,2,2-tetrafluoroethyl, pentafluoroethyl, 1-chloroethyl, 2-chloroethyl, 1,1-dichloroethyl, 2,2-dichloroethyl, 2,2,2-trichloroethyl, 1,1,2,2-tetrachloroethyl, pentachloroethyl, 1-bromoethyl, 2-bromoethyl, 2,2,2-tribromoethyl, 1-iodoethyl, 2-iodoethyl, 2-chloro-2,2-difluoroethyl, 2,2-dichloro-2-fluoroethyl, 2-trichloroethyl, 1-fluoropropyl, 2-fluoropropyl, 3-fluoropropyl, 1,1-difluoropropyl, 2,2-difluoropropyl, 3,3-difluoropropyl, 3,3,3-trifluoropropyl, 2,2,3,3,3-pentafluoropropyl, heptafluoropropyl, 1-fluoropropan-2-yl, 2-fluoropropan-2-yl, 1,1-difluoropropan-2-yl, 1,2-difluoropropan-2-yl, 1,3-difluoropropan-2-yl, 1,2,3-trifluoropropan-2-yl, 1,1,3,3-tetrafluoropropan-2-yl, 1,1,1,3,3,3-hexafluoropropan-2-yl, heptafluoropropan-2-yl, 1-chloropropyl, 2-chloropropyl, 3-chloropropyl, 1,1-dichloropropyl, 2,2-dichloropropyl, 3,3-dichloropropyl, 3,3,3-trichloropropyl, 2,2,3,3,3-pentachloropropyl, heptachloropropyl, 1-chloropropan-2-yl, 2-chloropropan-2-yl, 1,1-dichloropropan-2-yl, 1,2-dichloropropan-2-yl, 1,3-dichloropropan-2-yl, 1,2,3-trichloropropan-2-yl, 1,1,3,3-tetrachloropropan-2-yl, 1,1,1,3,3,3-hexachloropropan-2-yl, heptachloropropan-2-yl, 1-bromopropyl, 2-bromopropyl, 3-bromopropyl, 1-bromopropan-2-yl, 2-bromopropan-2-yl, 1-iodopropyl, 2-iodopropyl, 3-iodopropyl, 1-iodopropan-2-yl, 2-iodopropan-2-yl, 1-fluorobutyl, 2-fluorobutyl, 3-fluorobutyl, 4-fluorobutyl, 4,4-difluorobutyl, 4,4,4-trifluorobutyl, 3,3,4,4,4-pentafluorobutyl, 2,2,3,3,4,4,4-heptafluorobutyl, nonafluorobutyl, 1,1,1-trifluorobutan-2-yl, 4,4,4-trifluorobutan-2-yl, 3,3,4,4,4-pentafluorobutan-2-yl, nonafluorobutan-2-yl, 1,1,1,3,3,3-hexafluoro-2- (trifluoromethyl) propan-2-yl, 1-chlorobutyl, 2-chlorobutyl, 3-chlorobutyl, 4-chlorobutyl, 4,4-dichlorobutyl, 4,4,4-trichlorobutyl, nonachlorobutyl, 1,1,1-trichlorobutan-2-yl, 4,4,4-trichlorobutan-2-yl, nonachlorobutan-2-yl, 1-bromobutyl, 2-bromobutyl, 3-bromobutyl, 4-bromobutyl, 1-iodobutyl, 2-iodobutyl, 3-iodobutyl, 4-iodobutyl, 4-chloro-1,1,2,2,3,3,4,4-octafluorobutyl, 4-bromo-1,1,2,2,3,3,4,4-octafluorobutyl, 1-fluoropentyl, 2-fluoropentyl, 3-fluoropentyl, 4-fluoropentyl, 5-fluoropentyl, 5,5,5-trifluoropentyl, 4,4,5,5,5-pentafluoropentyl, 3,3,4,4,5,5,5-heptafluoropentyl, 2,2,3,3,4,4,5,5,5-nonafluoropentyl, undecafluoropentyl, 1-chloropentyl, 2-chloropentyl, 3-chloropentyl, 4-chloropentyl, 5-chloropentyl, 5,5,5-trichloropentyl, 4,4,5,5,5-pentachloropentyl, 3,3,4,4,5,5,5-heptachloropentyl, 2,2,3,3,4,4,5,5,5-nonachloropentyl, undecachloropentyl, 1-bromopentyl, 2-bromopentyl, 3-bromopentyl, 4-bromopentyl, 5-bromopentyl, 5-iodopentyl, 1-fluorohexyl, 2-fluorohexyl, 3-fluorohexyl, 4-fluorohexyl, 5-fluorohexyl, 6-fluorohexyl, 6,6,6-trifluorohexyl, 5,5,6,6,6-pentafluorohexyl, 4,4,5,5,6,6,6-heptafluorohexyl, 3,3,4,4,5,5,6,6,6-nonafluorohexyl, 2,2,3,3,4,4,5,5,6,6,6-undecafluorohexyl, tridecafluorohexyl, 1-chlorohexyl, 2-chlorohexyl, 3-chlorohexyl, 4-chlorohexyl, 5-chlorohexyl, 6-chlorohexyl, 5-bromohexyl, 6-bromohexyl, 5-iodohexyl, or 6-iodohexyl groups, and the like can be mentioned.

In the present invention, the "$C_1$-$C_{12}$ haloalkyl group" refers to a linear or branched alkyl group having 1 to 12 carbon atoms substituted with 1 to 25 same or different halogen atoms, unless otherwise specified. For example, in addition to the examples in the above-mentioned "$C_1$-$C_6$ haloalkyl group", 2,2,3,4,4,6,6,6-octafluoro-3,5,5-tris(trifluoromethyl)hexyl, 1H,1H-perfluoroheptyl, 1H,1H,2H,2H-perfluoroheptyl, 1H, 1H,2H,2H,3H,3H - perfluoroheptyl, 1H, 1H, 7H-perfluoroheptyl, perfluoroheptyl, 2-(perfluoro-3-methylbutyl) ethyl, 1H,1H-perfluorooctyl, 1H,1H,2H,2H-perfluorooctyl, 1H,1H,2H,2H,3H,3H-perfluorooctyl, 6-(perfluorohexyl) ethyl, 1H,1H,8H-perfluorooctyl, perfluorooctyl, 1H,1H-perfluorononyl, 1H,1H,2H,2H-perfluorononyl, 1H,1H,2H,2H,3H,3H-perfluorononyl, 6-(perfluoro-1-methylethyl) hexyl, 1H,1H,9H-perfluorononyl, perfluorononyl, 1H,1H-perfluorodecyl, 1H,1H,2H,2H-perfluorodecyl, 1H,1H,2H,2H,3H,3H-perfluorodecyl, 6-(perfluorobutyl)hexyl, 1H,1H,9H-perfluorodecyl, or perfluorodecyl groups, and the like can be mentioned.

In the present invention, the "$C_3$-$C_6$ cycloalkyl group" refers to a cycloalkyl group having 3 to 6 carbon atoms, unless otherwise specified. For example, cyclopropyl, cyclobutyl, cyclopentyl, and cyclohexyl groups, and the like can be mentioned.

In the present invention, the "$C_3$-$C_6$ halocycloalkyl group" refers to a cycloalkyl group having 3 to 6 carbon atoms substituted with 1 to 11 same or different halogen atoms, unless otherwise specified. For example, 1-fluorocyclopropyl, 2-fluorocyclopropyl, 2,2-difluorocyclopropyl, 2,2,3,3-tetrafluorocyclopropyl, 1-chlorocyclopropyl, 2-chlorocyclopropyl, 2,2-dichlorocyclopropyl, 2,2,3,3-tetrachlorocyclopropyl, 2,2-dibromocyclopropyl, 2,2-diiodocyclopropyl, 1-fluorocyclobutyl, 2-fluorocyclobutyl, 3-fluorocyclobutyl, 3,3-difluorocyclobutyl, heptafluorocyclobutyl, 2-chlorocyclobutyl, 3-chlorocyclobutyl, 3,3-dichlorocyclobutyl, 3,3-dibromocyclobutyl, 3,3-diiodocyclobutyl, 1-fluorocyclopentyl, 2-fluorocyclopentyl, 3-fluorocyclopentyl, 2,2-difluorocyclopentyl, 3,3-difluorocyclopentyl, nonafluorocyclopentyl, 2,2-dichlorocyclopentyl, 3,3-dichlorocyclopentyl, 2,2-dibromocyclopentyl, 3,3-dibromocyclopentyl, 2,2-diiodocyclopentyl, 3,3-diiodocyclopentyl, 1-fluorocyclohexyl, 2-fluorocyclohexyl, 3-fluorocyclohexyl, 4-fluorocyclohexyl, 2,2-difluorocyclohexyl, 3,3-difluorocyclohexyl, 4,4-difluorocyclohexyl, 1-chlorocyclohexyl, 2-chlorocyclohexyl, 3-chlorocyclohexyl, 4-chlorocyclohexyl, 2,2-dichlorocyclohexyl, 3,3-dichlorocyclohexyl, 4,4-dichlorocyclohexyl, 3,3-dibromocyclohexyl, 4,4-dibromocyclohexyl, 3,3-diiodocyclohexyl, 4,4-diiodocyclohexyl, or perfluorocyclohexyl groups, and the like can be mentioned.

In the present invention, the "$C_2$-$C_6$ alkenyl group" refers to a linear or branched alkenyl group having 2 to 6 carbon atoms, unless otherwise specified. For example, vinyl, 1-propenyl, isopropenyl, 2-propenyl, 1-butenyl, 1-methyl-1-propenyl, 2-butenyl, 1-methyl-2-propenyl, 3-butenyl , 2-methyl-1-propenyl, 2-methyl-2-propenyl, 1,3-butadienyl, 1-pentenyl, 1-ethyl-2-propenyl, 2-pentenyl, 1-methyl-1-butenyl, 3-pentenyl, 1-methyl-2-butenyl, 4-pentenyl, 1-methyl-3-butenyl, 3-methyl-1-butenyl, 1,2-dimethyl-2-propenyl, 1,1-dimethyl-2-propenyl, 2-methyl-2-butenyl, 3-methyl-2-butenyl, 1,2-dimethyl-1-propenyl, 2-methyl-3-butenyl, 3-methyl-3-butenyl, 1,3-pentadienyl, 1-vinyl-2-propenyl, 1-hexenyl, 1-propyl-2-propenyl, 2-hexenyl, 1-methyl-1-pentenyl, 1-ethyl-2-butenyl, 3-hexenyl, 4-hexenyl, 5-hexenyl, 1-methyl-4-pentenyl, 1-ethyl-3-butenyl, 1- (isobutyl) vinyl, 1-ethyl-1-methyl-2-propenyl, 1-ethyl-2-methyl-2-propenyl, 1-(isopropyl) -2-propenyl, 2-methyl-2-pentenyl, 3-methyl-3-pentenyl, 4-methyl-3-pentenyl, 1,3-dimethyl-2-butenyl, 1,1-dimethyl-3-butenyl, 3-methyl-4-pentenyl, 4-methyl-4-pentenyl, 1,2-dimethyl-3-butenyl, 1,3-dimethyl-3-butenyl, 1,1,2-trimethyl-2-propenyl, 1,5-hexadienyl, 1-vinyl-3-butenyl, 2,4-hexadienyl, or 3,3-dimethyl-1-butenyl groups, and the like can be mentioned.

In the present invention, the "$C_2$-$C_6$ haloalkenyl group" refers to a linear or branched alkenyl group having 2 to 6 carbon atoms substituted with 1 to 11 same or different halogen atoms, unless otherwise specified. For example, 1-fluorovinyl, 2-fluorovinyl, 1,2-difluorovinyl, 2,2-difluorovinyl, trifluorovinyl, 1-chlorovinyl, 2-chlorovinyl, 1,2-dichlorovinyl,

2,2-dichlorovinyl, trichlorovinyl, 1,2-dibromovinyl, 2,2-dibromovinyl, tribromovinyl, 1,2-diiodomovinyl, 2,2-diiodovinyl, triiodovinyl, 1-fluoro-2-propenyl, 2-fluoro-2-propenyl, 3-fluoro-2-propenyl, 2,3-difluoro-2-propenyl, 3,3-difluoro-2-propenyl, 3,3-difluoro-1-propenyl, 2,3,3-trifluoro-2-propenyl, 3,3,3-trifluoro-1-propenyl, 2-chloro-3,3,3-trifluoro-1-propenyl, 1,2,3,3,3-pentafluoro-1-propenyl, 1-chloro-2-propenyl, 2-chloro-2-propenyl, 3-chloro-2-propenyl, 2,3-dichloro-2-propenyl, 3,3-dichloro-2-propenyl, 3,3-dichloro-1-propenyl, 2,3,3-trichloro-2-propenyl, 3,3,3-trichloro-1-propenyl, 3-bromo-2-propenyl, 3,3-dibromo-2-propenyl, 3,3-diiodo-2-propenyl, 2,2-difluoro-1-propen-2-yl, 3,3,3-trifluoro-1-propen-2-yl, 3,3,3-trichloro-1-propen-2-yl, 4-fluoro-3-butenyl, 4,4-difluoro-3-butenyl, 4,4-difluoro-3-buten-2-yl, 4,4,4-trifluoro-2-butenyl, 3,4,4-trifluoro-3-butenyl, 2-trifluoromethyl-2-propenyl, 2-trifluoromethyl-3,3-difluoro-2-propenyl, 4,4,4-trifluoro-3-chloro-2-butenyl, 4,4-dichloro-3-butenyl, 4,4,4-trichloro-2-butenyl, 2-trichloromethyl-2-propenyl, 5,5-difluoro-4-pentenyl, 4,5,5-trifluoro-4-pentenyl, 5,5,5-trifluoro-3-pentenyl, 4,4,4-trifluoro-3-methyl-2-butenyl, 4,4,4-trifluoro-3-trifluoromethyl-2-butenyl, 5,5-dichloro-4-pentenyl, 4,4,4-trichloro-3-methyl-2-butenyl, 6,6-difluoro-5-hexenyl, 5,6,6-trifluoro-5-pentenyl, 6,6,6-trifluoro-4-pentenyl, 5,5,5-trifluoro-4-methyl-3-pentenyl, 5,5,5-trifluoro-4-trifluoromethyl-3-pentenyl, 6,6-dichloro-5-hexenyl, or 5,5,5-trichloro-4-methyl-3-pentenyl groups, and the like can be mentioned.

In the present invention, the "$C_3$-$C_6$ cycloalkenyl group" refers to a cycloalkenyl group having 3 to 6 carbon atoms, unless otherwise specified. For example, 1-cyclopropen-1-yl, 1-cyclobuten-1-yl, 1-cyclopenten-1-yl or 1-cyclohexen-1-yl groups, and the like can be mentioned.[0039] In the present invention, the "$C_2$-$C_6$ alkynyl group" refers to a linear or branched alkynyl group having 2 to 6 carbon atoms, unless otherwise specified. For example, ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 1-(n-propyl)-2-propynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, 1-ethyl-3-butynyl, 1-ethyl-1-methyl-2-propynyl, 1-(isopropyl)-2-propynyl, 1,1-dimethyl-2-butynyl, or 2,2-dimethyl-3-butynyl or 3,3-dimethyl-1-butynyl groups, and the like can be mentioned.

In the present invention, the "$C_2$-$C_6$ haloalkynyl group" refers to a linear or branched alkynyl group having 2 to 6 carbon atoms substituted with 1 to 9 same or different halogen atoms, unless otherwise specified. For example, fluoroethynyl, chloroethynyl, bromoethynyl, iodoethynyl, 3-fluoro-2-propynyl, 3-chloro-2-propynyl, 3-bromo-2-propynyl, 3-iodo-2-propynyl, 4-fluoro-3-butynyl, 4-chloro-3-butynyl, 4-bromo-3-butynyl, 4-iodo-3-butynyl, 4,4-difluoro-2-butynyl, 4,4-dichloro-2-butynyl, 4,4,4-trifluoro-2-butynyl, 4,4,4-trichloro-2-butynyl, 3-fluoro-1-methyl-2-propynyl, 3-chloro-1-methyl-2-propynyl, 5-fluoro-4-pentynyl, 5-chloro-4-pentynyl, 5,5,5-trifluoro-3-pentynyl, 5,5,5-trichloro-3-pentynyl, 4-fluoro-2-methyl-3-butynyl, 4-chloro-2-methyl-3-butynyl, 6-fluoro-5-hexynyl, 6-chloro-5-hexynyl, 6,6,6-trifluoro-4-hexynyl, 6,6,6-trichloro-4-hexynyl, 5-fluoro-3-methyl-4-pentynyl, or 5-chloro-3-methyl-4-pentynyl groups, and the like can be mentioned.

In the present invention, the "$C_3$-$C_6$ cycloalkyl $C_2$-$C_6$ alkynyl group" means a group in which any position of the $C_2$-$C_6$ alkynyl group is substituted with a $C_3$-$C_6$ cycloalkyl group, wherein the cycloalkyl moiety and the alkynyl moiety have the above-mentioned meanings, unless otherwise specified. For example, cyclopropylethynyl, cyclobutylethynyl, cyclopentylethynyl, cyclohexylethynyl, 3-cyclopropyl-1-propynyl, 1-cyclopropyl-2-propynyl, 3-cyclopropyl-2-propynyl, 3-cyclopropyl-1,1-dimethyl-2-propynyl, 4-cyclopropyl-1,1-dimethyl-2-butynyl groups, and the like can be mentioned.

In the present invention, the "$C_1$-$C_6$ alkoxy group" means a ($C_1$-$C_6$ alkyl) -O- group, wherein the alkyl moiety has the above-mentioned meaning, unless otherwise specified. For example, methoxy, ethoxy, n-propoxy, isopropoxy, n-butoxy, isobutoxy, sec-butoxy, tert-butoxy, n-pentoxy, 1-methylbutoxy, 2-methylbutoxy, 3-methylbutoxy, 1-ethylpropoxy, 1,1-dimethylpropoxy, 1,2-dimethylpropoxy, or n-hexyloxy groups, and the like can be mentioned.

In the present invention, the "$C_1$-$C_6$ haloalkoxy group" refers to a ($C_1$-$C_6$ haloalkyl) -O- group, wherein the haloalkyl moiety has the above-mentioned meaning, unless otherwise specified. For example, difluoromethoxy, dichloromethoxy, trifluoromethoxy, trichloromethoxy, tribromomethoxy, chlorodifluoromethoxy, bromodifluoromethoxy, 2-fluoroethoxy, 1-chloroethoxy, 2-chloroethoxy, 1-bromoethoxy, 2-bromoethoxy, 2,2-difluoroethoxy, 1,2-dichloroethoxy, 2,2-dichloroethoxy, 2,2,2-trifluoroethoxy, 2,2,2-trichloroethoxy, 1,1,2,2-tetrafluoroethoxy, pentafluoroethoxy, 2-bromo-2-chloroethoxy, 2-chloro-1,1,2,2-tetrafluoroethoxy, 1-chloro-1,2,2,2-tetrafluoroethoxy, 1-chloropropoxy, 2-chloropropoxy, 3-chloropropoxy, 2-bromopropoxy, 3-bromopropoxy, 2-bromo-1-methylethoxy, 3-iodopropoxy, 2,3-dichloropropoxy, 2,3-dibromopropoxy, 3,3,3-trifluoropropoxy, 3,3,3-trifluoro-2-propoxy, 3,3,3-trichloropropoxy, 3-bromo-3,3-difluoropropoxy, 2,2-difluoropropoxy, 3,3-dichloro-3-fluoropropoxy, 2,2,3,3-tetrafluoropropoxy, 1-bromo-3,3,3-trifluoropropoxy, 2,2,3,3,3-pentafluoropropoxy, 2,2,2-trifluoro-1-trifluoromethylethoxy, heptafluoropropoxy, heptafluoro-2-propoxy, 1,2,2,2-tetrafluoro-1-trifluoromethylethoxy, 1,1,2,3,3,3-hexafluoropropoxy, 2-chlorobutoxy, 3-chlorobutoxy, 4-chlorobutoxy, 2-chloro-1,1-dimethylethoxy, 4-bromobutoxy, 3-bromo-2-methylpropoxy, 2-bromo-1, 1-dimethylethoxy, 2,2-dichloro-1,1-dimethylethoxy, 2-chloro-1-chloromethyl-2-methylethoxy, 4,4,4-trifluorobutoxy, 3,3,3-trifluoro-1-methylpropoxy, 3,3,3-trifluoro-2-methylpropoxy, 2,3,4-trichlorobutoxy, 2,2,2-trichloro-1,1-dimethylethoxy, 4-chloro-4,4-difluorobutoxy, 4,4-dichloro-4-fluorobutoxy, 4-bromo-4,4-difluorobutoxy, 2,4-dibromo-4,4-difluorobutoxy, 3,4-dichloro-3,4,4-trifluorobutoxy, 3,3-dichloro-4,4,4-trifluorobutoxy, 4-bromo-3,3,4,4-tetrafluorobutoxy, 4-bromo-3-chloro-3,4,4-trifluorobutoxy, 2,2,3,3,4,4-hexafluorobutoxy, 2,2,3,4,4,4-hexafluorobutoxy, 2,2,2-trifluoro-1-methyl-1-trifluoromethylethoxy, 3,3,3-trifluoro-2-trifluoromethylpropoxy, 2,2,3,3,4,4,4-heptafluorobutoxy, 3,3,4,4,4-pentafluoro-2-butoxy, 2,3,3,3-tetrafluoro-2-trifluoromethylpropoxy, 1,1,2,2,3,3,4,4-octafluorobutoxy, nonafluorobutoxy, perfluoro-tert-butoxy, 4-chloro-1,1,2,2,3,3,4,4-octafluorobutoxy, 5,5,5-trifluoropentoxy, 4,4,5,5,5-pentafluoropentoxy, 3,3,4,4,5,55-heptafluoropentoxy, 3,3,4,4,5,5,5-heptafluoro-2-pentoxy, 2,2,3,3,4,4,5,5,5-nonafluoropentoxy, 2,2,3,3,4,4,5,5-octafluoropentoxy, perfluoropentoxy, 4,4,5,5,5-pentafluoro-2-butoxy, 2,2-bis(triflulomethyl)propoxy, 2,2,3,3,4,4,5,5,6,6,6-undecafluorohexyloxy, 3,3,4,4,5,5,6,6,6-nonafluorohexyloxy, 4,4,5,5,6,6,6-heptafluorohexyloxy, 2,2,3,3,4,4,5,5,6,6-decafluorohexyloxy, 4,4,4-trifluoro-3,3-bis(trifluoromethyl)butyloxy, perfluorohexyloxy groups, and the like can be mentioned.

In the present invention, the "$C_1$-$C_6$ alkoxy $C_1$-$C_6$ alkyl group" means a group in which any position of the $C_1$-$C_6$ alkyl group is substituted with a $C_1$-$C_6$ alkoxy group, wherein the alkoxy moiety and the alkyl moiety have the above-mentioned meanings, unless otherwise specified. For example, methoxymethyl, ethoxymethyl, n-propoxymethyl, isopropoxymethyl, tert-butoxymethyl, 1-methoxyethyl, 1-methoxy-1-methylethyl, 2-methoxyethyl, 1-ethoxyethyl, 2-ethoxyethyl, 2-isopropoxyethyl, 3-methoxypropyl, 2-methoxypropyl, 3-ethoxypropyl, 4-methoxybutyl, or 4-ethoxybutyl groups, and the like can be mentioned.

In the present invention, the "$C_1$-$C_6$ alkoxy $C_1$-$C_6$ haloalkyl group" means a group in which any position of the $C_1$-$C_6$ haloalkyl group is substituted with a $C_1$-$C_6$ alkoxy group, wherein the alkoxy moiety and the haloalkyl moiety have the above-mentioned meanings, unless otherwise specified. For example, any combination of those exemplified by the above-mentioned "$C_1$-$C_6$ alkoxy group" and "$C_1$-$C_6$ haloalkyl group" can be mentioned.

In the present invention, the "$C_1$-$C_6$ haloalkoxy $C_1$-$C_6$ alkyl group" means a group in which any position of the $C_1$-$C_6$ alkyl group is substituted with a $C_1$-$C_6$ haloalkoxy group, wherein the haloalkoxy moiety and the alkyl moiety have the above-mentioned meanings, unless otherwise specified. For example, any combination of those exemplified by the above-mentioned "$C_1$-$C_6$ haloalkoxy group" and "$C_1$-$C_6$ alkyl group" can be mentioned.

In the present invention, the "$C_1$-$C_6$ haloalkoxy $C_1$-$C_6$ haloalkyl group" means a group in which any position of the $C_1$-$C_6$ haloalkyl group is substituted with a $C_1$-$C_6$ haloalkoxy group, wherein the haloalkoxy moiety and the haloalkyl moiety have the above-mentioned meanings, unless otherwise specified. For example, any combination of those exemplified by the above-mentioned "$C_1$-$C_6$ haloalkoxy group" and "$C_1$-$C_6$ haloalkyl group" can be mentioned.

In the present invention, the "$C_3$-$C_6$ cycloalkyl $C_1$-$C_6$ alkyl group" means a group in which any position of the $C_1$-$C_6$ alkyl group is substituted with a $C_3$-$C_6$ cycloalkyl group, wherein the cycloalkyl moiety and the alkyl moiety have the above-mentioned meanings, unless otherwise specified. For example, any combination of those exemplified by the above-mentioned "$C_3$-$C_6$ cycloalkyl group" and "$C_1$-$C_6$ alkyl group" can be mentioned.

In the present invention, the "$C_3$-$C_6$ halocycloalkyl $C_1$-$C_6$ alkyl group" means a group in which any position of the $C_1$-$C_6$ alkyl group is substituted with a $C_3$-$C_6$ halocycloalkyl group, wherein the halocycloalkyl moiety and the alkyl moiety have the above-mentioned meanings, unless otherwise specified. For example, any combination of those exemplified by the above-mentioned "$C_3$-$C_6$ halocycloalkyl group" and "$C_1$-$C_6$ alkyl group" can be mentioned.

In the present invention, the "$C_3$-$C_6$ cycloalkyl $C_2$-$C_6$ alkenyl group" refers to a group in which any position of the $C_2$-$C_6$ alkenyl group is substituted with a $C_3$-$C_6$ cycloalkyl group wherein the cycloalkyl moiety and the alkenyl moiety have the above-mentioned meanings, unless otherwise specified. For example, any combination of those exemplified by the above-mentioned "$C_3$-$C_6$ cycloalkyl group" and "$C_2$-$C_6$ alkenyl group" can be mentioned.

In the present invention, the "$C_1$-$C_6$ haloalkoxy $C_1$-$C_6$ alkoxy group" means a group in which any position of the $C_1$-$C_6$ alkoxy group is substituted with a $C_1$-$C_6$ haloalkoxy group, wherein the haloalkoxy moiety and the alkoxy moiety have the above-mentioned meanings, unless otherwise specified. For example, any combination of those exemplified by the above-mentioned "$C_1$-$C_6$ haloalkoxy group" and "$C_1$-$C_6$ alkoxy group" can be mentioned.

In the present invention, the "$C_1$-$C_6$ alkoxy $C_1$-$C_6$ haloalkoxy group" means a group in which any position of the $C_1$-$C_6$ haloalkoxy group is substituted with a $C_1$-$C_6$ alkoxy group, wherein the alkoxy moiety and the haloalkoxy moiety have the above-mentioned meanings, unless otherwise specified. For example, any combination of those exemplified by the above-mentioned "$C_1$-$C_6$ alkoxy group" and "$C_1$-$C_6$ haloalkoxy group" can be mentioned.

In the present invention, the "$C_1$-$C_6$ haloalkoxy $C_1$-$C_6$ haloalkoxy group" means a group in which any position of the $C_1$-$C_6$ haloalkoxy group is substituted with a $C_1$-$C_6$ haloalkoxy group, wherein the haloalkoxy moiety has the above-mentioned meaning, unless otherwise specified. For example, any combination of those exemplified in the above-mentioned "$C_1$-$C_6$ haloalkoxy group" can be mentioned. The $C_1$-$C_6$ haloalkoxy groups may be the same as each other or different from each other.

In the present invention, the "$C_3$-$C_6$ cycloalkyloxy group" refers to a ($C_3$-$C_6$ cycloalkyl)-O-group in which the cycloalkyl moiety has the above-mentioned meaning, unless otherwise specified. For example, cyclopropoxy, cyclobutoxy, cyclopentyloxy, or cyclohexyloxy groups, and the like can be mentioned.

In the present invention, the "$C_3$-$C_6$ halocycloalkyloxy group" means a ($C_3$-$C_6$ halocycloalkyl) -O- group in which the halocycloalkyl moiety has the above-mentioned meaning, unless otherwise specified. For example, 2,2-difluorocyclopropoxy, 2,2-dichlorocyclopropoxy, 3,3-difluorocyclobutoxy, 3,3-dichlorocyclobutoxy, 3-fluorocyclopentyloxy, 3,3-difluorocyclopentyloxy, nonafluorocyclopentyloxy, 3,3-dichlorocyclopentyloxy, 4,4-difluorocyclohexyloxy, or 4,4-dichlorocyclohexyloxy groups, and the like can be mentioned.

In the present invention, the "$C_3$-$C_6$ cycloalkyl $C_1$-$C_6$ alkoxy group" means a group in which any position of the $C_1$-$C_6$ alkoxy group is substituted with a $C_3$-$C_6$ cycloalkyl group, wherein the cycloalkyl moiety and the alkoxy moiety

have the above-mentioned meanings, unless otherwise specified. For example, cyclopropylmethoxy, cyclobutylmethoxy, cyclopentylmethoxy, or cyclohexylmethoxy groups can be mentioned.

In the present invention, the "$C_3$-$C_6$ halocycloalkyl $C_1$-$C_6$ alkoxy group" means a group in which any position of the $C_1$-$C_6$ alkoxy group is substituted with a $C_3$-$C_6$ halocycloalkyl group, wherein the halocycloalkyl moiety and the alkoxy moiety have the above-mentioned meanings, unless otherwise specified. For example, 1-fluorocyclopropylmethoxy, 1-chlorocyclopropylmethoxy, 1-bromocyclopropylmethoxy, 1-fluorocyclobutylmethoxy, 1-chlorocyclobutylmethoxy, 1-bromocyclobutylmethoxy, 2,2-difluorocyclopropylmethoxy, 2,2-dichlorocyclopropylmethoxy, and 3,3-difluorocyclobutylmethoxy groups can be mentioned.

In the present invention, the "$C_6$-$C_{12}$ aryl group" refers to an aryl group having 6 to 12 carbon atoms, unless otherwise specified. For example, phenyl, 1-naphthyl and 2-naphthyl groups can be mentioned. The 1-naphthyl group is also refered to as naphthalen-1-yl group. The 2-naphthyl group is also refered to as naphthalen-2-yl group.

In the present invention, the "$C_6$-$C_{12}$ aryloxy group" refers to a ($C_6$-$C_{12}$ aryl)-O- group, unless otherwise specified. For example, phenoxy, naphthalen-1-yloxy, naphthalen-2-yloxy groups, and the like can be mentioned.

In the present invention, the "$C_7$-$C_{14}$ aralkyl group" refers to an alkyl group substituted with an aryl group having 7 to 14 carbon atoms, unless otherwise specified. For example, $C_6$-$C_{12}$ aryl $C_1$-$C_2$ alkyl, phenyl $C_1$-$C_2$ alkyl groups, and the like can be mentioned. Specific examples of the $C_7$-$C_{14}$ aralkyl group include benzyl, 1-phenylethyl, 2-phenylethyl, 3-phenylpropyl, 4-phenylbutyl, naphthalen-1-ylmethyl, naphthalen-2-ylmethyl groups, and the like. Besides, the aralkyl group is also refered to as arylalkyl group.

In the present invention, the "$C_7$-$C_{14}$ aralkyloxy group" refers to a ($C_7$-$C_{14}$ aralkyl)-O- group in which the aralkyl moiety has the above-mentioned meaning, unless otherwise specified. For example, benzyloxy, 1-phenylethyloxy, 2-phenylethyloxy, 3-phenylpropyloxy, 4-phenylbutyloxy, (naphthalen-1-yl)methyloxy, (naphthalen-2-yl)methyloxy groups, and the like can be mentioned.

In the present invention, the "$C_5$-$C_{12}$ heteroaryl group" means a monocyclic or fused bicyclic structure having 5 to 12 ring member atoms which are one or more heteroatoms selected from O, S and N. The inclusion of heteroatoms allows aromaticity in 5-membered rings as well as in 6-membered rings. Examples of typical heteroatomic systems include monocyclic $C_5$-$C_6$ aromatic groups, such as pyridyl, pyrimidyl, pyrazinyl, pyridazinyl, triazinyl, thienyl, furanyl, pyrrolyl, pyrazolyl, thiazolyl, isothiazolyl, oxazolyl, isoxazolyl, imidazolyl, triazolyl, thiadiazolyl, oxadiazolyl, or tetrazolyl groups; fused bicyclic moieties formed by fusing one of these monocyclic groups with a phenyl ring or with any heteroaromatic monocyclic group to form a $C_8$-$C_{10}$ bicyclic group, such as indolyl, benzimidazolyl, indazolyl, benzotriazolyl, isoquinolinyl, quinolinyl, benzothiazolyl, benzofuranyl, benzothienyl, benzisoxazolyl, pyrazolopyridyl, quinazolinyl, quinoxalinyl, or cinnolinyl groups, and the like can be mentioned..

Here, the substitutional position is not particularly limited. That is, the heteroaryl group includes all its positional isomers. For example, when the heteroaryl group is a pyridyl group, the pyridyl group may be 2-pyridyl, 3-pyridyl, or 4-pyridyl group.

In the present invention, the "$C_5$-$C_{12}$ heteroaryloxy group" means a ($C_5$-$C_{12}$ heteroaryl)-O-group in which the heteroaryl moiety has the above-mentioned meaning, unless otherwise specified.

In the present invention, the "$C_6$-$C_{14}$ heteroaralkyl group" means an alkyl group in which any position of $C_1$-$C_2$ alkyl group is substituted with a heteroaryl group having 5 to 12 carbon atoms, wherein the heteroaryl moiety has the above-mentioned meaning, and the $C_1$-$C_2$ alkyl moiety refers to a methyl or ethyl group, unless otherwise specified. For example, pyridylmethyl, pyridylethyl, indolylmethyl, furylmethyl, thienylmethyl, pyrrolylmethyl groups, and the like can be mentioned.

In the present invention, the "$C_6$-$C_{14}$ heteroaralkyloxy group" means a ($C_6$-$C_{14}$ heteroaralkyl)-O- group in which the heteroaralkyl moiety has the above-mentioned meaning, unless otherwise specified. For example, pyridylmethyloxy, pyridylethyloxy, indolylmethyloxy, furylmethyloxy, thienylmethyloxy, pyrrolylmethyl, pyrrolylmethyloxy groups, and the like can be mentioned.

In the present invention, the "pentahalosulfanyl group" refers to a $\lambda^6$-sulfanyl group substituted with 5 same or different halogen atoms, unless otherwise specified. For example, chlorotetrafluorosulfanyl, or pentafluorosulfanyl groups, and the like can be mentioned.

In the present invention, the "$C_1$-$C_6$ alkylthio group" refers to a ($C_1$-$C_6$ alkyl)-S- group in which the alkyl moiety has the above-mentioned meaning, unless otherwise specified. For example, methylthio, ethylthio, n-propylthio, isopropylthio, n-butylthio, isobutylthio, sec-butylthio, tert-butylthio, pentylthio, or hexylthio groups, and the like can be mentioned.

In the present invention, the "$C_1$-$C_6$ alkylsulfinyl group" refers to a ($C_1$-$C_6$ alkyl)-S(=O)-group in which the alkyl moiety has the above-mentioned meaning, unless otherwise specified. For example, methylsulfinyl, ethylsulfinyl, n-propylsulfinyl, isopropylsulfinyl, n-butylsulfinyl, isobutylsulfinyl, sec-butylsulfinyl, tert-butylsulfinyl, pentylsulfinyl, or hexylsulfinyl groups, and the like can be mentioned.

In the present invention, the "$C_1$-$C_6$ alkylsulfonyl group" refers to a ($C_1$-$C_6$ alkyl)-$S(=O)_2$- group in which the alkyl moiety has the above-mentioned meaning, unless otherwise specified. For example, methylsulfonyl, ethylsulfonyl, n-propylsulfonyl, isopropylsulfonyl, n-butylsulfonyl, isobutylsulfonyl, sec-butylsulfonyl, tert-butylsulfonyl, pentylsulfonyl,

or hexylsulfonyl groups, and the like can be mentioned.

In the present invention, the "$C_1$-$C_6$ haloalkylthio group" refers to a ($C_1$-$C_6$ haloalkyl)-S-group in which the haloalkyl moiety has the above-mentioned meaning, unless otherwise specified. For example, fluoromethylthio, difluoromethylthio, trifluoromethylthio, trichloromethylthio, 2,2,2-trifluoroethylthio, pentafluoroethylthio, 2,2,2-trichloroethylthio, 3,3,3-trifluoropropylthio, 1,1,2,3,3,3-hexafluoropropylthio, heptafluoropropylthio, (1,1,1,3,3,3-hexafluoropropan-2-yl)thio, (heptafluoropropan-2-yl)thio, or 4,4,4-trifluorobutylthio groups, and the like can be mentioned.

In the present invention, the "$C_1$-$C_6$ haloalkylsulfinyl group" refers to a ($C_1$-$C_6$ haloalkyl)-S(=O)- group in which the haloalkyl moiety has the above-mentioned meaning, unless otherwise specified. For example, difluoromethylsulfinyl, trifluoromethylsulfinyl, trichloromethylsulfinyl, 2,2,2-trifluoroethylsulfinyl, 2,2,2-trichloroethylsulfinyl, pentafluoroethylsulfinyl, 3,3,3-trifluoropropylsulfinyl, heptafluoropropylsulfinyl, or heptafluoro-2-propylsulfinyl groups, and the like can be mentioned.

In the present invention, the "$C_1$-$C_6$ haloalkylsulfonyl group" refers to a ($C_1$-$C_6$ haloalkyl)-$S(=O)_2$- group in which the haloalkyl moiety has the above-mentioned meaning, unless otherwise specified. For example, difluoromethylsulfonyl, trifluoromethylsulfonyl, trichloromethylsulfonyl, 2,2,2-trifluoroethylsulfonyl, pentafluoroethylsulfonyl, 3,3,3-trifluoropropylsulfonyl, heptafluoropropylsulfonyl, heptafluoro-2-propylsulfonyl, or nonafluorobutylsulfonyl groups, and the like can be mentioned.

In the present invention, the "$C_3$-$C_6$ cycloalkylthio group" refers to a ($C_3$-$C_6$ cycloalkyl)-S-group in which the cycloalkyl moiety has the above-mentioned meaning, unless otherwise specified. For example, cyclopropylthio, cyclobutylthio, cyclopentylthio, or cyclohexylthio groups, and the like can be mentioned.

In the present invention, the "$C_3$-$C_6$ cycloalkylsulfinyl group" refers to a ($C_3$-$C_6$ cycloalkyl)-S(=O)- group in which the cycloalkyl moiety has the above-mentioned meaning, unless otherwise specified. For example, cyclopropylsulfinyl, cyclobutylsulfinyl, cyclopentylsulfinyl, or cyclohexylsulfinyl groups, and the like can be mentioned.

In the present invention, the "$C_3$-$C_6$ cycloalkylsulfonyl group" refers to a ($C_3$-$C_6$ cycloalkyl)-$S(=O)_2$-group in which the cycloalkyl moiety has the above-mentioned meaning, unless otherwise specified. For example, cyclopropylsulfonyl, cyclobutylsulfonyl, cyclopentylsulfonyl, or cyclohexylsulfonyl groups, and the like can be mentioned.

In the present invention, the "$C_3$-$C_6$ halocycloalkylthio group" refers to a ($C_3$-$C_6$ halocycloalkyl)-S- group in which the halocycloalkyl moiety has the above-mentioned meaning, unless otherwise specified. For example, 2,2-difluorocyclopropylthio, 2,2-dichlorocyclopropylthio, 3,3-difluorocyclobutylthio, 3,3-dichlorocyclobutylthio, 3-fluorocyclopentylthio, 3,3-difluorocyclopentylthio, nonafluorocyclopentylthio, 3,3-dichlorocyclopentylthio, 4,4-difluorocyclohexylthio, or 4,4-dichlorocyclohexylthio groups, and the like can be mentioned.

In the present invention, the "$C_3$-$C_6$ halocycloalkylsulfinyl group" refers to a ($C_3$-$C_6$ halocycloalkyl)-S(=O)- group in which the halocycloalkyl moiety has the above-mentioned meaning, unless otherwise specified. For example, 2,2-difluorocyclopropylsulfinyl, 2,2-dichlorocyclopropylsulfinyl, 3,3-difluorocyclobutylsulfinyl, 3,3-dichlorocyclobutylsulfinyl, 3-fluorocyclopentylsulfinyl, 3,3-difluorocyclopentylsulfinyl, nonafluorocyclopentylsulfinyl, 3,3-dichlorocyclopentylsulfinyl, 4,4-difluorocyclohexylsulfinyl, or 4,4-dichlorocyclohexylsulfinyl groups, and the like can be mentioned.

In the present invention, the "$C_3$-$C_6$ halocycloalkylsulfonyl group" refers to a ($C_3$-$C_6$ halocycloalkyl)-$S(=O)_2$- group in which the halocycloalkyl moiety has the above-mentioned meaning, unless otherwise specified. For example, 2,2-difluorocyclopropylsulfonyl, 2,2-dichlorocyclopropylsulfonyl, 3,3-difluorocyclobutylsulfonyl, 3,3-dichlorocyclobutylsulfonyl, 3-fluorocyclopentylsulfonyl, 3,3-difluorocyclopentylsulfonyl, nonafluorocyclopentylsulfonyl, 3,3-dichlorocyclopentylsulfonyl, 4,4-difluorocyclohexylsulfonyl, or 4,4-dichlorocyclohexylsulfonyl groups, and the like can be mentioned.

In the present invention, the "$C_1$-$C_6$ alkylsulfonyloxy group" refers to a ($C_1$-$C_6$ alkylsulfonyl)-O- group in which the alkylsulfonyl moiety has the above-mentioned meaning, unless otherwise specified. For example, methylsulfonyloxy, ethylsulfonyloxy, n-propylsulfonyloxy, isopropylsulfonyloxy, n-butylsulfonyloxy, isobutylsulfonyloxy, sec-butylsulfonyloxy, tert-butylsulfonyloxy, pentylsulfonyloxy, or hexylsulfonyloxy groups, and the like can be mentioned.

In the present invention, the "$C_1$-$C_6$ haloalkylsulfonyloxy group" refers to a ($C_1$-$C_6$ haloalkylsulfonyl)-O- group in which the haloalkylsulfonyl moiety has the above-mentioned meaning, unless otherwise specified. For example, difluoromethylsulfonyloxy, trifluoromethylsulfonyloxy, trichloromethylsulfonyloxy, 2,2,2-trifluoroethylsulfonyloxy, pentafluoroethylsulfonyloxy, 3,3,3-trifluoropropylsulfonyloxy, heptafluoropropylsulfonyloxy, heptafluoro-2-propylsulfonyloxy, or nonafluorobutylsulfonyloxy groups, and the like can be mentioned.

In the present invention, the "$C_6$-$C_{12}$ arylthio group" refers to a ($C_6$-$C_{12}$ aryl)-S- group in which the aryl moiety has the above-mentioned meaning, unless otherwise specified. For example, phenylthio, 1-naphthylthio, 2-naphthylthio groups, and the like can be mentioned.

In the present invention, the "$C_6$-$C_{12}$ arylsulfinyl group" refers to a ($C_6$-$C_{12}$ aryl)-S(=O)- group in which the aryl moiety has the above-mentioned meaning, unless otherwise specified. For example, phenylsulfinyl, 1-naphthylsulfinyl, 2-naphthylsulfinyl groups, and the like can be mentioned.

In the present invention, the "$C_6$-$C_{12}$ arylsulfonyl group" refers to a ($C_6$-$C_{12}$ aryl)-$S(=O)_2$- group in which the aryl moiety has the above-mentioned meaning, unless otherwise specified. For example, phenylsulfonyl, 1-naphthylsulfonyl, 2-naphthylsulfonyl groups, and the like can be mentioned.

In the present invention, the "$C_5$-$C_{12}$ heteroarylthio group" refers to a ($C_5$-$C_{12}$heteroaryl)-S- group in which the aryl moiety has the above-mentioned meaning, unless otherwise specified. For example, pyridin-2-ylthio, pyridin-3-ylthio, pyridin-4-ylthio, pyrazin-2-ylthio, pyridazin-3-ylthio, pyridazin-4-ylthio, pyrimidin-2-ylthio, pyrimidin-4-ylthio, pyrimidin-5-ylthio, thiazol-2-ylthio, thiazol-4-ylthio, or thiazol-5-ylthio groups, and the like can be mentioned.

In the present invention, the "$C_5$-$C_{12}$ heteroarylsulfinyl group" refers to a ($C_5$-$C_{12}$ heteroaryl)-S(=O)- group in which the aryl moiety has the above-mentioned meaning, unless otherwise specified. For example, pyridin-2-ylsulfinyl, pyridin-3-ylsulfinyl, pyridin-4-ylsulfinyl, pyrazin-2-ylsulfinyl, pyridazin-3-ylsulfinyl, pyridazin-4-ylsulfinyl, pyrimidin-2-ylsulfinyl, pyrimidin-4-ylsulfinyl, pyrimidin-5-ylsulfinyl, thiazol-2-ylsulfinyl, thiazol-4-ylsulfinyl, or thiazol-5-ylsulfinyl groups, and the like can be mentioned.

In the present invention, the "$C_5$-$C_{12}$ heteroarylsulfonyl group" refers to a ($C_5$-$C_{12}$ heteroaryl)-S(=O)$_2$- group in which the aryl moiety has the above-mentioned meaning, unless otherwise specified. For example, pyridin-2-ylsulfonyl, pyridin-3-ylsulfonyl, pyridin-4-ylsulfonyl, pyrazin-2-ylsulfonyl, pyridazin-3-ylsulfonyl, pyridazin-4-ylsulfonyl, pyrimidin-2-ylsulfonyl, pyrimidin-4-ylsulfonyl, pyrimidin-5-ylsulfonyl, thiazol-2-ylsulfonyl, thiazol-4-ylsulfonyl, or thiazol-5-ylsulfonyl groups, and the like can be mentioned.

In the present invention, the "$C_6$-$C_{12}$ arylsulfonyloxy group" refers to a ($C_6$-$C_{12}$ arylsulfonyl) -O- group in which the arylsulfonyl moiety has the above-mentioned meaning, unless otherwise specified. For example, phenylsulfonyloxy, 1-naphthylsulfonyloxy, 2-naphthylsulfonyloxy groups and the like can be mentioned.

In the present invention, the "$C_1$-$C_6$ haloalkylthio $C_1$-$C_6$ alkoxy group" means a group in which any position of the $C_1$-$C_6$ alkoxy group is substituted with a $C_1$-$C_6$ haloalkylthio group, wherein the haloalkylthio moiety and the alkoxy moiety have the above-mentioned meanings, unless otherwise specified.

In the present invention, the "$C_1$-$C_6$ haloalkylsulfinyl $C_1$-$C_6$ alkoxy group" means a group in which any position of the $C_1$-$C_6$ alkoxy group is substituted with a $C_1$-$C_6$ haloalkylsulfinyl group, wherein the haloalkylsulfinyl moiety and alkoxy moiety have the above-mentioned meanings, unless otherwise specified.

In the present invention, the "$C_1$-$C_6$ haloalkylsulfonyl $C_1$-$C_6$ alkoxy group" means a group in which any position of the $C_1$-$C_6$ alkoxy group is substituted with a $C_1$-$C_6$ haloalkylsulfonyl group, wherein the haloalkylsulfonyl moiety and alkoxy moiety have the above-mentioned meanings, unless otherwise specified.

In the present invention, the "$C_3$-$C_6$ cycloalkyl $C_1$-$C_6$ alkylthio group" means a group in which any position of the $C_1$-$C_6$ alkylthio group is substituted with a $C_3$-$C_6$ cycloalkyl group, wherein the cycloalkyl moiety and the alkylthio moiety have the above-mentioned meanings, unless otherwise specified.

In the present invention, the "$C_3$-$C_6$ cycloalkyl $C_1$-$C_6$ alkylsulfinyl group" means a group in which any position of the $C_1$-$C_6$ alkylsulfinyl group is substituted with a $C_3$-$C_6$ cycloalkyl group, wherein the cycloalkyl moiety and the alkylsulfinyl moiety have the above-mentioned meanings, unless otherwise specified.

In the present invention, the "$C_3$-$C_6$ cycloalkyl $C_1$-$C_6$ alkylsulfonyl group" means a group in which any position of the $C_1$-$C_6$ alkylsulfonyl group is substituted with a $C_3$-$C_6$ cycloalkyl group, wherein the cycloalkyl moiety and the alkylsulfonyl moiety have the above-mentioned meanings, unless otherwise specified.

In the present invention, the "$C_3$-$C_6$ halocycloalkyl $C_1$-$C_6$ alkylthio group" means a group in which any position of the $C_1$-$C_6$ alkylthio group is substituted with a $C_3$-$C_6$ halocycloalkyl group, wherein the halocycloalkyl moiety and the alkylthio moiety have the above-mentioned meanings, unless otherwise specified.

In the present invention, the "$C_3$-$C_6$ halocycloalkyl $C_1$-$C_6$ alkylsulfinyl group" means a group in which any position of the $C_1$-$C_6$ alkylsulfinyl group is substituted with a $C_3$-$C_6$ halocycloalkyl group, wherein the halocycloalkyl moiety and the alkylsulfinyl moiety have the above-mentioned meanings, unless otherwise specified.

In the present invention, the "$C_3$-$C_6$ halocycloalkyl $C_1$-$C_6$ alkylsulfonyl group" means a group in which any position of the $C_1$-$C_6$ alkylsulfonyl group is substituted with a $C_3$-$C_6$ halocycloalkyl group, wherein the halocycloalkyl moiety and the alkylsulfonyl moiety have the above-mentioned meanings, unless otherwise specified.

In the present invention, the "hydroxy $C_1$-$C_6$ alkoxy group" means a group in which any position of the alkoxy group is substituted with "HO-".

In the present invention, the "hydroxy $C_3$-$C_6$ cycloalkyloxy group" means a group in which any position of the cycloalkyloxy group is substituted with "HO-".

In the present invention, the "hydroxyimino $C_1$-$C_6$ alkyl group" means a group in which any position of the alkyl group is substituted with "HO-N=".

In the present invention, the "$C_1$-$C_6$ alkoxyimino $C_1$-$C_6$ alkyl group" means a group in which any position of the alkyl group is substituted with "($C_1$-$C_6$ alkoxy)-N=".

In the present invention, the "$C_1$-$C_6$ haloalkoxyimino $C_1$-$C_6$ alkyl group" means a group in which any position of the alkyl group is substituted with "($C_1$-$C_6$ haloalkoxy)-N=".

In the present invention, the "cyano $C_1$-$C_6$ alkyl group" means a group in which any position of the $C_1$-$C_6$ alkyl group is substituted with a cyano group, wherein the alkyl moiety has the above-mentioned meaning, unless otherwise specified. For example, cyanomethyl, 1-cyanoethyl, 2-cyanoethyl, 3-cyanopropyl, 2-cyanopropan-2-yl, 1-cyanobutyl, 4-cyanobutyl, 5-cyanopentyl, or 6-cyanohexyl groups, and the like can be mentioned.

In the present invention, the "cyano $C_3$-$C_6$ cycloalkyl group" means a group in which any position of the $C_3$-$C_6$ cycloalkyl group is substituted with a cyano group, wherein the cycloalkyl moiety has the above-mentioned meaning, unless otherwise specified. For example, 1-cyanocyclopropyl, 2-cyanocyclopropyl, 1-cyanocyclobutyl, 2-cyanocyclobutyl, 3-cyanocyclobutyl, 1-cyanocyclopentyl, 3-cyanocyclopentyl, 1-cyanocyclohexyl, or 4-cyanocyclohexyl groups, and the like can be mentioned.

In the present invention, the "hydroxy $C_1$-$C_6$ alkyl group" means a group in which any position of the $C_1$-$C_6$ alkyl group is substituted with a hydroxy group wherein the alkyl moiety has the above-mentioned meanings, unless otherwise specified. For example, hydroxymethyl, 1-hydroxyethyl, 2-hydroxyethyl, 3-hydroxypropyl, 2-hydroxypropane-2-yl, 1-hydroxybutyl, 4-hydroxybutyl, 5-hydroxypentyl or 6-hydroxyhexyl groups and the like can be mentioned.

In the present invention, the "hydroxy $C_3$-$C_6$ cycloalkyl group" means a group in which any position of the cycloalkyl group is substituted with a hydroxy group wherein the cycloalkyl moiety has the above-mentioned meanings, unless otherwise specified. For example, 1-hydroxycyclopropyl, 2-hydroxycyclopropyl, 1-hydroxycyclobutyl, 2-hydroxycyclobutyl, 3-hydroxycyclobutyl, 1-hydroxycyclopentyl, 3-hydroxycyclopentyl, 1-hydroxycyclohexyl or 4-hydroxycyclohexyl groups and the like can be mentioned.

In the present invention, the "cyano $C_3$-$C_6$ cycloalkyloxy group" means a group in which any position of the $C_3$-$C_6$ cycloalkyloxy group is substituted with a cyano group, wherein the cycloalkyloxy moiety has the above-mentioned meaning, unless otherwise specified. For example, 1-cyanocyclopropoxy, 2-cyanocyclopropoxy, 1-cyanocyclobutoxy, 2-cyanocyclobutoxy, 3-cyanocyclobutoxy, 1-cyanocyclopentyloxy, 3-cyanocyclopentyloxy, 1-cyanocyclohexyloxy, or 4-cyanocyclohexyloxy groups, and the like can be mentioned.

In the present invention, the "cyano $C_1$-$C_6$ alkoxy group" means a group in which any position of the $C_1$-$C_6$ alkoxy group is substituted with a cyano group, wherein the alkoxy moiety has the above-mentioned meaning, unless otherwise specified. For example, cyanomethoxy, 1-cyanoethoxy, 2-cyanoethoxy, 1-cyanopropoxy, 3-cyanopropoxy, 2-cyano-2-propoxy, 1-cyanobutoxy, 4-cyanobutoxy, 5-cyanopentyloxy, or 6-cyanohexyloxy groups, and the like can be mentioned.

In the present invention, the "(cyano $C_3$-$C_6$ cycloalkyl) $C_1$-$C_6$ alkoxy group" means a group in which any position of the $C_1$-$C_6$ alkoxy group is substituted with a cyano $C_3$-$C_6$ cycloalkyl group, wherein the cyano $C_3$-$C_6$ cycloalkyl moiety and the alkoxy moiety have the above-mentioned meanings, unless otherwise specified. For example, (1-cyanocyclopropyl) methoxy, (2-cyanocyclopropyl) methoxy, (1-cyanocyclobutyl) methoxy, (2-cyanocyclobutyl) methoxy, (3-cyanocyclobutyl) methoxy, (1-cyanocyclopentyl) methoxy, (3-cyanocyclopentyl) methoxy, (1-cyanocyclohexyl) methoxy, or (4-cyanocyclohexyl) methoxy groups, and the like can be mentioned.

In the present invention, the "mono ($C_1$-$C_6$ alkyl) amino group" refers to a ($C_1$-$C_6$ alkyl)-NH- group in which the alkyl moiety has the above-mentioned meaning, unless otherwise specified. For example, methylamino, ethylamino, isopropylamino, n-propylamino, or tert-butylamino groups, and the like can be mentioned.

In the present invention, the "di($C_1$-$C_6$ alkyl) amino group" refers to a ($C_1$-$C_6$ alkyl)$_2$-N- group in which the alkyl moiety has the above-mentioned meaning, and the two alkyl groups may be different from each other, unless otherwise specified. For example, dimethylamino, methylethylamino, or methyl-n-propylamino groups, and the like can be mentioned.

In the present invention, the "(di($C_1$-$C_6$ alkyl) sulfinylidene) amino group" refers to an O = S ($C_1$-$C_6$ alkyl)$_2$ = N- group, wherein the two alkyl groups may be different from each other. For example, (S,S-dimethylsulfinylidene) amino, (S,S-diethylsulfinylidene) amino, (S-ethyl-S-methylsulfinylidene) amino groups, and the like can be mentioned.

In the present invention, the "di ($C_1$-$C_6$ alkyl) phosphoryl group" refers to an O = P ($C_1$-$C_6$ alkyl)$_2$ group wherein the two alkyl groups may be different from each other. For example, dimethylphosphoryl, diethylphosphoryl, ethylmethylphosphoryl groups, and the like can be mentioned.

In the present invention, the "$C_1$-$C_7$ acyl group" refers to an H-C(=O)- group or a ($C_1$-$C_6$ alkyl)-C(=O)- group, unless otherwise specified. For example, formyl, acetyl, propionyl, isobutyryl, or pivaloyl groups, and the like can be mentioned.

The "$C_1$-$C_7$ acyl group which is unsubstituted or substituted with halogen atoms" may have 1 to 10, preferably 1 to 5 halogen atoms at its substitutable positions. When the number of halogen atoms is two or more, the halogen atoms may be the same or different.

In the present invention, the "$C_1$-$C_7$ acyloxy group" refers to a ($C_1$-$C_7$ acyl) -O- group in which the acyl moiety has the above-mentioned meaning, unless otherwise specified. For example, formyloxy, acetoxy, propionyloxy, isobutyryloxy, or pivaloyloxy groups, and the like can be mentioned.

In the present invention, the "$C_1$-$C_7$ haloacyl group" refers to a halogen-substituted formyl group or ($C_1$-$C_6$ haloalkyl)-C(=O)- group, unless otherwise specified. For example, chloroformyl, chloroacetyl, trifluoroacetyl, trichloroacetyl, 2-chloropropionyl, 2-chloroisobutyryl, 2-bromoisobutyryl, or 3-chloropivaloyl groups, and the like can be mentioned.

In the present invention, the "hydroxyimino (amino) $C_1$-$C_6$ alkyl group" means a $(H_2N)C(=NOH)$- group or a group in which any position of the $C_1$-$C_6$ alkyl group is substituted with "$(H_2N)C(=NOH)$-".

In the present invention, the "carboxyl group" refers to a HO-C(=O)- group.

In the present invention, the "$C_1$-$C_6$ alkoxycarbonyl group" refers to a ($C_1$-$C_6$ alkoxy)-C(=O)-group.

In the present invention, the "N-($C_1$-$C_6$ alkoxy)-N-($C_1$-$C_6$ alkyl) aminocarbonyl group" refers to a ($C_1$-$C_6$ alkoxy)($C_1$-$C_6$ alkyl)N-C(=O)- group.

In the present invention, the "aminocarbonyl group" refers to an $NH_2$-C(=O)- group.

In the present invention, the "aminothiocarbonyl group" refers to an $NH_2$-C(=S)- group.

In the present invention, the "mono ($C_1$-$C_6$ alkyl) aminocarbonyl group" refers to a ($C_1$-$C_6$ alkyl)-NH-C(=O)- group in which the alkyl moiety has the above-mentioned meaning, unless otherwise specified. For example, methylaminocarbonyl, ethylaminocarbonyl, propylaminocarbonyl, isopropylaminocarbonyl, or tert-butylaminocarbonyl groups, and the like can be mentioned.

In the present invention, the "mono (cyano $C_3$-$C_6$ cycloalkyl) aminocarbonyl group" refers to a (cyano $C_3$-$C_6$ cycloalkyl)-NH-C(=O)- group wherein the cyano $C_3$-$C_6$ cycloalkyl moiety has the above meanings, unless otherwise specified. For example, 1-cyanocyclopropylaminocarbonyl, 2-cyanocyclopropylaminocarbonyl, 1-cyanocyclobutylaminocarbonyl, 2-cyanocyclobutylaminocarbonyl, 3-cyanocyclobutylaminocarbonyl, 1-cyanocyclopentylaminocarbonyl, 3-cyano cyclopentylaminocarbonyl, 1-cyanocyclohexylaminocarbonyl or 4-cyanocyclohexylaminocarbonyl groups, and the like can be mentioned.

In the present invention, the "mono ($C_1$-$C_6$ alkoxyimino $C_1$-$C_6$ alkyl) aminocarbonyl group" refers to a ($C_1$-$C_6$ alkoxyimino $C_1$-$C_6$ alkyl)-NH-C(= O)- group wherein the $C_1$-$C_6$ alkoxyimino $C_1$-$C_6$ alkyl moiety has the above meanings, unless otherwise specified. For example, methoxyiminomethylaminocarbonyl, ethoxyiminomethylaminocarbonyl groups, and the like can be mentioned.

In the present invention, the "di ($C_1$-$C_6$ alkyl) aminomethylidene aminocarbonyl group" refers to a ($C_1$-$C_6$ alkyl)$_2$-N-C=N-C (= O)- group wherein the alkyl moiety has the above meanings, unless otherwise specified. For example, dimethylaminomethylidene aminocarbonyl, diethylaminomethylidene aminocarbonyl or diisopropylaminomethylidene aminocarbonyl groups, and the like can be mentioned.

In the present invention, the "mono ($C_1$-$C_6$ alkyl) aminothiocarbonyl group" refers to a ($C_1$-$C_6$ alkyl)-NH-C(=S)- group in which the alkyl moiety has the above-mentioned meaning, unless otherwise specified.

In the present invention, the "di($C_1$-$C_6$ alkyl) aminocarbonyl group" refers to a ($C_1$-$C_6$ alkyl)$_2$-N-C(=O)- group in which the alkyl moiety has the above-mentioned meaning, and the two alkyl groups may be different from each other, unless otherwise specified. For example, dimethylaminocarbonyl, diethylaminocarbonyl, or diisopropylaminocarbonyl groups, and the like can be mentioned.

In the present invention, the "di($C_1$-$C_6$ alkyl) aminothiocarbonyl group" refers to a ($C_1$-$C_6$ alkyl)$_2$-N-C(=S)- group in which the alkyl moiety has the above-mentioned meaning, and the two alkyl groups may be different from each other, unless otherwise specified.

In the present invention, the "mono ($C_1$-$C_6$ haloalkyl) aminocarbonyl group" refers to a ($C_1$-$C_6$ haloalkyl)-NH-C(=O)- group in which the haloalkyl moiety has the above-mentioned meaning, unless otherwise specified. For example, 2-fluoroethylaminocarbonyl, 2,2-difluoroethylaminocarbonyl, 2,2,2-trifluoroethylaminocarbonyl, 2,2,2-trichloroethylaminocarbonyl, or 1,1,1,3,3,3-hexafluoro-2-propylaminocarbonyl groups, and the like can be mentioned.

In the present invention, the "mono ($C_3$-$C_6$ cycloalkyl) aminocarbonyl group" refers to a ($C_3$-$C_6$ cycloalkyl)-NH-C(=O)- group in which the cycloalkyl moiety has the above-mentioned meaning, unless otherwise specified. For example, cyclopropylaminocarbonyl, cyclobutylaminocarbonyl, cyclopentylaminocarbonyl, cyclohexylaminocarbonyl groups, and the like can be mentioned.

In the present invention, the notations such as "$C_6$-$C_{12}$ aryl group which is unsubstituted or optionally substituted with $R^{11}$" represent an aryl group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on an aryl group, the respective $R^{11}$ may be the same as each other or different from each other.

In the present invention, the notations such as "$C_5$-$C_{12}$ heteroaryl group which is unsubstituted or optionally substituted with $R^{11}$" represent a heteroaryl group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on a heteroaryl group, the respective $R^{11}$ may be the same as each other or different from each other.

In the present invention, notations such as "$C_7$-$C_{14}$ aralkyl group which is unsubstituted or optionally substituted with $R^{11}$" represent an aralkyl group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on an aralkyl group, the respective $R^{11}$ may be the same as each other or different from each other.

In the present invention, the notations such as "$C_6$-$C_{14}$ heteroaralkyl group which is unsubstituted or optionally substituted with $R^{11}$" represent a heteroaralkyl group in which a hydrogen atom bonded to a carbon atom is substituted

by any $R^{11}$. The number of $R^{11}$ to be substituted is optionally selected within the range of each specified number of carbon atoms, for example, 1 to 10, preferably 1 to 5. When there are two or more substituents $R^{11}$ on a heteroaralkyl group, the respective $R^{11}$ may be the same as each other or different from each other.

In the present invention, the notations such as "$C_6$-$C_{12}$ aryloxy group which is unsubstituted or optionally substituted with $R^{11}$" represent an aryloxy group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on an aryloxy group, the respective $R^{11}$ may be the same as each other or different from each other.

In the present invention, the notations such as "$C_5$-$C_{12}$ heteroaryloxy group which is unsubstituted or optionally substituted with $R^{11}$" represent a heteroaryloxy group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents R 11 on a heteroaryloxy group, the respective $R^{11}$ may be the same as each other or different from each other.

In the present invention, the notations such as "$C_7$-$C_{14}$ aralkyloxy group which is unsubstituted or optionally substituted with $R^{11}$" represent an aralkyloxy group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on an aralkyloxy group, the respective $R^{11}$ may be the same as each other or different from each other.

In the present invention, the notations such as "$C_6$-$C_{14}$ heteroaralkyloxy group which is unsubstituted or optionally substituted with $R^{11}$" represent a heteroaralkyloxy group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on a heteroaralkyloxy group, the respective $R^{11}$ may be the same as each other or different from each other.

In the present invention, the notation "heterocyclic group which is unsubstituted or optionally substituted with $R^{11}$" represents a heterocyclic group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on a heterocyclic group, the respective $R^{11}$ may be the same as each other or different from each other.

In the present invention, a heterocyclic group means a non-aromatic, monocyclic or polycyclic cyclic group having one or more identical or different heteroatoms (for example, nitrogen atoms, oxygen atoms and / or sulfur atoms) instead of one or more carbon atoms.

In the present invention, examples of heterocyclic groups include, but are not limited to, non-aromatic, monocyclic, bicyclic or tricyclic, and 3- to 14-membered (preferably 4- to 14-membered and more preferably 5- to 14-membered) heterocyclic groups having 1 to 13 carbon atoms and 1 to 4 heteroatoms independently selected from nitrogen, oxygen and sulfur atoms.

In the present invention, examples of heterocyclic groups include, but are not limited to, non-aromatic, monocyclic or bicyclic, and 3- to 10-membered (preferably 4- to 10-membereed, more preferably 5- to 10-membered), and still more preferably 5- to 7-membered) heterocyclic groups having 1 to 9 carbon atoms and 1 to 4 heteroatoms independently selected from nitrogen, oxygen and sulfur atoms.

Further, in the present invention, examples of heterocyclic groups include, but are not limited to, non-aromatic, monocyclic, and 3- to 8-membered (preferably 5- to 6-membered) heterocyclic groups having 1 to 5 carbon atoms and 1 to 4 heteroatoms independently selected from nitrogen, oxygen and sulfur atoms.

Specific examples of monocyclic heterocyclic groups include, but are not limited to, isoxazolinyl, piperidinyl, piperazinyl, azetidinyl, aziridinyl, morpholinyl, dihydropyranyl, tetrahydropyranyl, tetrahydrofuryl, oxetanyl, oxiranyl, dioxanyl (for example, 1,3-dioxanyl, 1,4-dioxanyl, and the like), dioxiranyl (for example, 1,3-dioxolanyl and the like), thianyl, tetrahydrothienyl, thietanyl, thiiranyl, and the like.

Specific examples of bicyclic heterocyclic groups include, but are not limited to, indolinyl, isoindolinyl, chromanyl, isochromanyl, tetrahydroisoquinolinyl, benzotetrahydrofuryl, benzotetrahydrothienyl, tetrahydroisobenzofuryl, tetrahydroisobenzothienyl, benzodioxolyl, dihydrobenzisoxazinyl, dihydrobenzisothiazinyl, 1-oxidotetrahydroquinolinyl, tetrahydroquinolinyl, dihydroquinolinyl, dihydroquinolinone-yl, dihydroisoquinolinone-yl, dihydrocoumarinyl, dihydroisocoumarinyl, isoindolinone-yl, benzodioxanyl, benzoxazolinone-yl, quinolizidinyl, indolizidinyl, tropanyl, nortropanyl, quinuclidinyl, 3-azabicyclo [3.1.0] hexanyl, 6-oxa-3-azabicyclo [3.1.0] hexanyl, and the like.

Here, the substitutional position is not particularly limited. That is, the heterocyclic group includes all its positional isomers. For example, when the heterocyclic group is piperidinyl, the piperidinyl group comprises 1-piperidinyl, 2-piperidinyl, 3-piperidinyl, and 4-piperidinyl groups.

The heterocyclic groups as defined or exemplified above may include non-fused cyclic (for example, monocyclic or spirocyclic) and fused cyclic groups, if possible.

The heterocyclic groups as defined or exemplified above may be unsaturated, partially saturated, or saturated,

if possible.

When a heteroatom of the heterocyclic groups as defined or exemplified above is a nitrogen atom, the nitrogen atom may be N-oxide.

When a heteroatom of the heterocyclic groups as defined or exemplified above is a sulfur atom, the sulfur atom may be sulfinyl (-SO-) or sulfonyl ($-SO_2-$).

In the present invention, the "heterocyclyl $C_1$-$C_6$ alkyl group" means a group in which any position of the $C_1$-$C_6$ alkyl group is substituted with a heterocyclyl group, wherein the heterocyclyl moiety and the alkyl moiety have the above-mentioned meanings, unless otherwise specified. For example, (1,3-dioxolan-2-yl) methyl group and the like can be mentioned.

In the present invention, the notations such as "heterocyclyl $C_1$-$C_6$ alkyl group which is unsubstituted or optionally substituted with $R^{11}$" represent a heterocyclyl $C_1$-$C_6$ alkyl group in which a hydrogen atom bonded to a carbon atom is substituted by any $R^{11}$. The number of $R^{11}$ to be substituted is optionally selected within the range of each specified number of carbon atoms, for example, 1 to 10, preferably 1 to 5. When there are two or more substituents $R^{11}$ on a heterocyclyl $C_1$-$C_6$ alkyl group, the respective $R^{11}$s may be the same as each other or different from each other.

When hydroxy groups, carboxyl groups, or nitrogen atoms of amino groups or pyridine rings are present in the structure of the compounds of the present invention represented by the general formula [I], a salt or agriculturally acceptable salt in the present invention is a salt formed by these groups or atoms with a metal or an organic base, or a salt formed with a mineral acid or an organic acid. Examples of the metal include alkali metals such as sodium and potassium or alkaline earth metals such as magnesium and calcium. Examples of the organic base include triethylamine or diisopropylamine, and the like. Examples of the mineral acid include phosphoric acid, hydrochloric acid, hydrobromic acid, hydrogen iodide acid, boric acid, or sulfuric acid, and the like. In addition, Examples of the organic acid include formic acid, acetic acid, lactic acid, ascorbic acid, succinic acid, fumaric acid, maleic acid, oxalic acid, citric acid, benzoic acid, salicylic acid, tartaric acid, methanesulfonic acid, 4-toluenesulfonic acid, or trifluoromethanesulfonic acid, and the like.

Next, typical examples of the compounds included in the heterocyclic compounds represented by the general formula [I] of the present invention are shown in Tables 1 to 23. However, the compounds included in the heterocyclic compounds of the present invention are not limited thereto. Besides, the compound numbers in the tables will be referred to in the following descriptions.

Although a compound included in the heterocyclic compounds of the present invention may contain a geometric isomer of E-form and Z-form depending on the types of substituents, the E-form, Z-form, and a mixture of E-form and Z-form in any proportion are included in the present invention. In addition, although compounds included in the present invention may contain optical isomers due to the presence of one or more asymmetric carbon atoms and asymmetric sulfur atoms, all optically active substances, racemic compounds, or diastereomers are included in the present invention.

The following notations in the tables herein represent, for example, the corresponding groups, respectively, as below:

Me: methyl,
Et: ethyl,
nPr: normal propyl,
iPr: isopropyl,
cPr: cyclopropyl,
nBu: normal butyl,
sBu: secondary butyl,
iBu: isobutyl,
tBu: tertiary butyl,
cBu: cyclobutyl,
nPen: normal pentyl,
cPen: cyclopentyl,
nHex: normal hexyl,
cHex: cyclohexyl,
(1-CN)cPr: 1-cyanocyclopropyl
(2,2-$F_2$)cPr: 2,2-difluorocyclopropyl
Ph: phenyl,
(4-$CF_3$) Ph: 4-trifluoromethylphenyl

Table 1

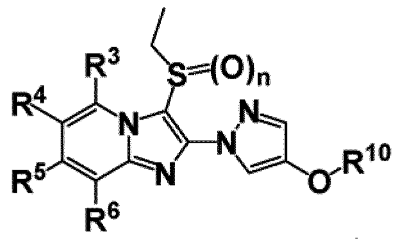

| Compd. No. | n | $R^3$ | $R^4$ | $R^5$ | $R^6$ | $R^{10}$ |
|---|---|---|---|---|---|---|
| A-0001 | 0 | H | H | H | H | $CH_2CF_2CF_2CF_3$ |
| A-0002 | 1 | H | H | H | H | $CH_2CF_2CF_2CF_3$ |
| A-0003 | 2 | H | H | H | H | $CH_2CF_2CF_2CF_3$ |
| A-0004 | 0 | H | H | H | H | $CH_2CF_2CHFCF_3$ |
| A-0005 | 1 | H | H | H | H | $CH_2CF_2CHFCF_3$ |
| A-0006 | 2 | H | H | H | H | $CH_2CF_2CHFCF_3$ |
| A-0007 | 2 | F | H | H | H | $CH_2CF_2CF_2CF_3$ |
| A-0008 | 2 | Cl | H | H | H | $CH_2CF_2CF_2CF_3$ |
| A-0009 | 2 | Br | H | H | H | $CH_2CF_2CF_2CF_3$ |
| A-0010 | 2 |  | H | H | H | $CH_2CF_2CF_2CF_3$ |
| A-0011 | 2 | Me | H | H | H | $CH_2CF_2CF_2CF_3$ |
| A-0012 | 2 | $CF_3$ | H | H | H | $CH_2CF_2CF_2CF_3$ |
| A-0013 | 2 | cPr | H | H | H | $CH_2CF_2CF_2CF_3$ |
| A-0014 | 2 | $(2,2\text{-}F_2)$cPr | H | H | H | $CH_2CF_2CF_2CF_3$ |
| A-0015 | 2 | (1-CN)cPr | H | H | H | $CH_2CF_2CF_2CF_3$ |
| A-0016 | 2 | $OCF_3$ | H | H | H | $CH_2CF_2CF_2CF_3$ |
| A-0017 | 2 | $OCHF_2$ | H | H | H | $CH_2CF_2CF_2CF_3$ |
| A-0018 | 2 | $OC(Me)_2CN$ | H | H | H | $CH_2CF_2CF_2CF_3$ |
| A-0019 | 2 | $SCF_3$ | H | H | H | $CH_2CF_2CF_2CF_3$ |
| A-0020 | 2 | $S(=O)CF_3$ | H | H | H | $CH_2CF_2CF_2CF_3$ |
| A-0021 | 2 | $S(=O)_2CF_3$ | H | H | H | $CH_2CF_2CF_2CF_3$ |
| A-0022 | 2 | F | H | H | H | $CH_2CF_2CHFCF_3$ |
| A-0023 | 2 | Cl | H | H | H | $CH_2CF_2CHFCF_3$ |
| A-0024 | 2 | Br | H | H | H | $CH_2CF_2CHFCF_3$ |
| A-0025 | 2 |  | H | H | H | $CH_2CF_2CHFCF_3$ |
| A-0026 | 2 | Me | H | H | H | $CH_2CF_2CHFCF_3$ |
| A-0027 | 2 | $CF_3$ | H | H | H | $CH_2CF_2CHFCF_3$ |
| A-0028 | 2 | cPr | H | H | H | $CH_2CF_2CHFCF_3$ |
| A-0029 | 2 | $(2,2\text{-}F_2)$cPr | H | H | H | $CH_2CF_2CHFCF_3$ |

(continued)

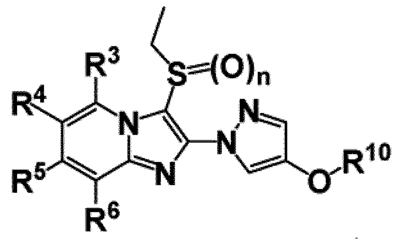

| Compd. No. | n | $R^3$ | $R^4$ | $R^5$ | $R^6$ | $R^{10}$ |
|---|---|---|---|---|---|---|
| A-0030 | 2 | (1-CN)cPr | H | H | H | $CH_2CF_2CHFCF_3$ |
| A-0031 | 2 | $OCF_3$ | H | H | H | $CH_2CF_2CHFCF_3$ |
| A-0032 | 2 | $OCHF_2$ | H | H | H | $CH_2CF_2CHFCF_3$ |
| A-0033 | 2 | $OC(Me)_2CN$ | H | H | H | $CH_2CF_2CHFCF_3$ |
| A-0034 | 2 | $SCF_3$ | H | H | H | $CH_2CF_2CHFCF_3$ |
| A-0035 | 2 | $S(=O)CF_3$ | H | H | H | $CH_2CF_2CHFCF_3$ |
| A-0036 | 2 | $S(=O)_2CF_3$ | H | H | H | $CH_2CF_2CHFCF_3$ |
| A-0037 | 2 | H | F | H | H | $CH_2CF_2CF_2CF_3$ |
| A-0038 | 2 | H | Cl | H | H | $CH_2CF_2CF_2CF_3$ |

Table 2

| Compd. No. | n | $R^3$ | $R^4$ | $R^5$ | $R^6$ | $R^{10}$ |
|---|---|---|---|---|---|---|
| A-0039 | 2 | H | Br | H | H | $CH_2CF_2CF_2CF_3$ |
| A-0040 | 2 | H | I | H | H | $CH_2CF_2CF_2CF_3$ |
| A-0041 | 2 | H | N3 | H | H | $CH_2CF_2CF_2CF_3$ |
| A-0042 | 2 | H | CN | H | H | $CH_2CF_2CF_2CF_3$ |
| A-0043 | 2 | H | $NO_2$ | H | H | $CH_2CF_2CF_2CF_3$ |
| A-0044 | 2 | H | Me | H | H | $CH_2CF_2CF_2CF_3$ |
| A-0045 | 2 | H | $CH_2CN$ | H | H | $CH_2CF_2CF_2CF_3$ |
| Ä-0046 | 2 | H | $CF_3$ | H | H | $CH_2CF_2CF_2CF_3$ |
| A-0047 | 2 | H | $CHF_2$ | H | H | $CH_2CF_2CF_2CF_3$ |
| A-0048 | 2 | H | $CF(CF_3)_2$ | H | H | $CH_2CF_2CF_2CF_3$ |
| A-0049 | 2 | H | cPr | H | H | $CH_2CF_2CF_2CF_3$ |
| A-0050 | 2 | H | cBu | H | H | $CH_2CF_2CF_2CF_3$ |
| A-0051 | 2 | H | cPen | H | H | $CH_2CF_2CF_2CF_3$ |
| A-0052 | 2 | H | $CH_2$(cPr) | H | H | $CH_2CF_2CF_2CF_3$ |
| A-0053 | 2 | H | $CH_2$(cBu) | H | H | $CH_2CF_2CF_2CF_3$ |
| A-0054 | 2 | H | $CH_2$(cPen) | H | H | $CH_2CF_2CF_2CF_3$ |
| A-0055 | 2 | H | $CH_2CH_2$(cPr) | H | H | $CH_2CF_2CF_2CF_3$ |

(continued)

| Compd. No. | n | $R^3$ | $R^4$ | $R^5$ | $R^6$ | $R^{10}$ |
|---|---|---|---|---|---|---|
| A-0056 | 2 | H | $(2,2\text{-}F_2)$cPr | H | H | $CH_2CF_2CF_2CF_3$ |
| A-0057 | 2 | H | $(2,2\text{-}Cl_2)$cPr | H | H | $CH_2CF_2CF_2CF_3$ |
| A-0058 | 2 | H | (1-CN)cPr | H | H | $CH_2CF_2CF_2CF_3$ |
| A-0059 | 2 | H | Ph | H | H | $CH_2CF_2CF_2CF_3$ |
| A-0060 | 2 | H | (4-F)Ph | H | H | $CH_2CF_2CF_2CF_3$ |
| A-0061 | 2 | H | (4-Cl)Ph | H | H | $CH_2CF_2CF_2CF_3$ |
| A-0062 | 2 | H | (4-Me)Ph | H | H | $CH_2CF_2CF_2CF_3$ |
| A-0063 | 2 | H | $(4\text{-}CF_3)$Ph | H | H | $CH_2CF_2CF_2CF_3$ |
| A-0064 | 2 | H | (4-OMe)Ph | H | H | $CH_2CF_2CF_2CF_3$ |
| A-0065 | 2 | H | $(4\text{-}OCF_3)$Ph | H | H | $CH_2CF_2CF_2CF_3$ |
| A-0066 | 2 | H | (4-CN)Ph | H | H | $CH_2CF_2CF_2CF_3$ |
| A-0067 | 2 | H | pyridin-2-yl | H | H | $CH_2CF_2CF_2CF_3$ |
| \|A-0068 | 2 | H | pyridin-3-yl | H | H | $CH_2CF_2CF_2CF_3$ |
| A-0069 | 2 | H | pyridin-4-yl | H | H | $CH_2CF_2CF_2CF_3$ |
| A-0070 | 2 | H | (3-Cl)pyridin-2-yl | H | H | $CH_2CF_2CF_2CF_3$ |
| A-0071 | 2 | H | $(3\text{-}CF_3)$pyridin-2-yl | H | H | $CH_2CF_2CF_2CF_3$ |
| A-0072 | 2 | H | (5-Cl)pyridin-2-yl | H | H | $CH_2CF_2CF_2CF_3$ |
| A-0073 | 2 | H | $(5\text{-}CF_3)$pyridin-2-yl | H | H | $CH_2CF_2CF_2CF_3$ |
| A-0074 | 2 | H | (2-Cl)pyridin-5-yl | H | H | $CH_2CF_2CF_2CF_3$ |
| A-0075 | 2 | H | $(2\text{-}CF_3)$pyridin-5-yl | H | H | $CH_2CF_2CF_2CF_3$ |
| A-0076 | 2 | H | pyrrol-1-yl | H | H | $CH_2CF_2CF_2CF_3$ |
| A-0077 | 2 | H | pyrazol-1-yl | H | H | $CH_2CF_2CF_2CF_3$ |
| A-0078 | 2 | H | imidazol-1-yl | H | H | $CH_2CF_2CF_2CF_3$ |
| A-0079 | 2 | H | 1,2,3-triazol-1-yl | H | H | $CH_2CF_2CF_2CF_3$ |
| A-0080 | 2 | H | 1,2,4-triazol-1-yl | H | H | $CH_2CF_2CF_2CF_3$ |
| A-0081 | 2 | H | 1H-tetrazol-1-yl | H | H | $CH_2CF_2CF_2CF_3$ |
| A-0082 | 2 | H | 2H-tetrazol-2-yl | H | H | $CH_2CF_2CF_2CF_3$ |

Table 3

| Compd. No. | n | $R^3$ | $R^4$ | $R^5$ | $R^6$ | $R^{10}$ |
|---|---|---|---|---|---|---|
| A-0083 | 2 | H | furan-2-yl | H | H | $CH_2CF_2CF_2CF_3$ |
| A-0084 | 2 | H | furan-3-yl | H | H | $CH_2CF_2CF_2CF_3$ |
| A-0085 | 2 | H | oxazol-2-yl | H | H | $CH_2CF_2CF_2CF_3$ |
| A-0086 | 2 | H | oxazol-4-yl | H | H | $CH_2CF_2CF_2CF_3$ |

(continued)

| Compd. No. | n | $R^3$ | $R^4$ | $R^5$ | $R^6$ | $R^{10}$ |
|---|---|---|---|---|---|---|
| A-0087 | 2 | H | isoxazol-3-yl | H | H | $CH_2CF_2CF_2CF_3$ |
| A-0088 | 2 | H | isoxazol-4-yl | H | H | $CH_2CF_2CF_2CF_3$ |
| A-0089 | 2 | H | isoxazol-5-yl | H | H | $CH_2CF_2CF_2CF_3$ |
| A-0090 | 2 | H | 1,2,4-oxadiazol-3-yl | H | H | $CH_2CF_2CF_2CF_3$ |
| A-0091 | 2 | H | 1,2,4-oxadiazol-5-yl | H | H | $CH_2CF_2CF_2CF_3$ |
| A-0092 | 2 | H | 1,2,5-oxadiazol-3-yl | H | H | $CH_2CF_2CF_2CF_3$ |
| A-0093 | 2 | H | 1,3,4-oxadiazol-2-yl | H | H | $CH_2CF_2CF_2CF_3$ |
| A-0094 | 2 | H | OH | H | IH | $CH_2CF_2CF_2CF_3$ |
| A-0095 | 2 | H | OMe | H | H | $CH_2CF_2CF_2CF_3$ |
| A-0096 | 2 | H | $OCF_3$ | H | H | $CH_2CF_2CF_2CF_3$ |
| A-0097 | 2 | H | $OCHF_2$ | H | H | $CH_2CF_2CF_2CF_3$ |
| A-0098 | 2 | H | $OCH_2CF_3$ | H | H | $CH_2CF_2CF_2CF_3$ |
| A-0099 | 2 | H | $OCF_2CF_3$ | H | H | $CH_2CF_2CF_2CF_3$ |
| A-0100 | 2 | H | $OCF_2CHF_2$ | H | H | $CH_2CF_2CF_2CF_3$ |
| A-0101 | 2 | H | $OCF_2CHFCl$ | H | H | $CH_2CF_2CF_2CF_3$ |
| A-0102 | 2 | H | $OCH_2CF_2CHF_2$ | H | H | $CH_2CF_2CF_2CF_3$ |
| A-0103 | 2 | H | $OCF_2CHFCF_3$ | H | H | $CH_2CF_2CF_2CF_3$ |
| A-0104 | 2 | H | $OC(Me)_2CN$ | H | H | $CH_2CF_2CF_2CF_3$ |
| A-0105 | 2 | H | $OCH_2CH_2OH$ | H | H | $CH_2CF_2CF_2CF_3$ |
| A-0106 | 2 | H | $OCH_2CH_2OCH_2CF_3$ | H | H | $CH_2CF_2CF_2CF_3$ |
| A-0107 | 2 | H | $OCF_2CHFOMe$ | H | H | $CH_2CF_2CF_2CF_3$ |
| A-0108 | 2 | H | $OCF_2CHFOCF_3$ | H | H | $CH_2CF_2CF_2CF_3$ |
| A-0109 | 2 | H | $OCF_2CHFOCF_2CF_3$ | H | H | $CH_2CF_2CF_2CF_3$ |
| A-0110 | 2 | H | $OCF_2CHFOCF_2CF_2CF_3$ | H | H | $CH_2CF_2CF_2CF_3$ |
| A-0111 | 2 H | H | O(cPr) | H | H | $CH_2CF_2CF_2CF_3$ |
| A-0112 | 2 | H | O(cBu) | H | H | $CN_2CF_2CF_2CF_3$ |
| A-0113 | 2 | H | O(cPen) | H | H | $CH_2CF_2CF_2CF_3$ |
| A-0114 | 2 | H | $OCH_2$(cPr) | H | H | $CH_2CF_2CF_2CF_3$ |
| A-0115 | 2 | H | O((2,2-$F_2$)cPr) | H | H | $CH_2CF_2CF_2CF_3$ |
| A-0116 | 2 | H | O((2,2-$Cl_2$)cPr) | H | H | $CH_2CF_2CF_2CF_3$ |
| A-0117 | 2 | H | O((1-CN)cPr) | H | H | $CH_2CF_2CF_2CF_3$ |
| A-0118 | 2 | H | $OCH_2$((2,2-$F_2$)cPr) | H | H | $CH_2CF_2CF_2CF_3$ |
| A-0119 | 2 | H | $OCH_2$((2,2-$Cl_2$)cPr) | H | H | $CH_2CF_2CF_2CF_3$ |
| A-0120 | 2 | H | $OCH_2$((1-CN)cPr) | H | H | $CH_2CF_2CF_2CF_3$ |

(continued)

| Compd. No. | n | $R^3$ | $R^4$ | $R^5$ | $R^6$ | $R^{10}$ |
|---|---|---|---|---|---|---|
| A-0121 | 2 | H | $OCH_2SCF_3$ | H | H | $CH_2CF_2CF_2CF_3$ |
| A-0122 | 2 | H | $O(CH2)_2SCF_3$ | H | H | $CH_2CF_2CF_2CF_3$ |
| A-0123 | 2 | H | $O(CH_2)_3SCF_3$ | H | H | $CH_2CF_2CF_2CF_3$ |
| A-0124 | 2 | H | $O(CH_2)_4SCF_3$ | H | H | $CH_2CF_2CF_2CF_3$ |
| A-0125 | 2 | H | $O(CH_2)_5SCF_3$ | H | H | $CH_2CF_2CF_2CF_3$ |
| A-0126 | 2 | H | $OCH_2S(=O)CF_3$ | H | H | $CH_2CF_2CF_2CF_3$ |

Table 4

| Compd. No. | n | $R^3$ | $R^4$ | $R^5$ | $R^6$ | $R^{10}$ |
|---|---|---|---|---|---|---|
| A-0127 | 2 | H | $O(CH_2)_2S(=O)CF_3$ | H | H | $CH_2CF_2CF_2CF_3$ |
| A-0128 | 2 | H | $O(CH_2)_3S(=O)CF_3$ | H | H | $CH_2CF_2CF_2CF_3$ |
| A-0129 | 2 | H | $O(CH_2)_4S(=O)CF_3$ | H | H | $CH_2CF_2CF_2CF_3$ |
| A-01 30 | 2 | H | $O(CH_2)_5S(=O)CF_3$ | H | H | $CH_2CF_2CF_2CF_3$ |
| A-0131 | 2 | H | $OCH_2S(=O)_2CF_3$ | H | H | $CH_2CF_2CF_2CF_3$ |
| A-0132 | 2 | H | $O(CH_2)_2S(=O)_2CF_3$ | H | H | $CH_2CF_2CF_2CF_3$ |
| A-0133 | 2 | H | $O(CH_2)_3S(=O)_2CF_3$ | H | H | $CH_2CF_2CF_2CF_3$ |
| A-0134 | 2 | H | $O(CH_2)_4S(=O)_2CF_3$ | H | H | $CH_2CF_2CF_2CF_3$ |
| A-0135 | 2 | H | $O(CH_2)_5S(=O)_2CF_3$ | H | H | $CH_2CF_2CF_2CF_3$ |
| A-0136 | 2 | H | $OCH_2Ph$ | H | H | $CH_2CF_2CF_2CF_3$ |
| A-0137 | 2 | H | $OCH_2$((4-OMe)Ph) | H | H | $CH_2CF_2CF_2CF_3$ |
| A-0138 | 2 | H | $OCH_2$(pyridin-2-yl) | H | H | $CH_2CF_2CF_2CF_3$ |
| A-0139 | 2 | H | $OCH_2$(pyridin-3-yl) | H | H | $CH_2CF_2CF_2CF_3$ |
| A-0140 | 2 | H | $OCH_2$(pyridin-4-yl) | H | H | $CH_2CF_2CF_2CF_3$ |
| A-0141 | 2 | H | OPh | H | H | $CH_2CF_2CF_2CF_3$ |
| A-0142 | 2 | H | O((2-F)Ph) | H | H | $CH_2CF_2CF_2CF_3$ |
| A-0143 | 2 | H | O((3-F)Ph) | H | H | $CH_2CF_2CF_2CF_3$ |
| A-0144 | 2 | H | O((4-F)Ph) | H | H | $CH_2CF_2CF_2CF_3$ |
| A-0145 | 2 | H | O((2-Cl)Ph) | H | H | $CH_2CF_2CF_2CF_3$ |
| A-0146 | 2 | H | O((3-Cl)Ph) | H | H | $CH_2CF_2CF_2CF_3$ |
| A-0147 | 2 | H | O((4-Cl)Ph) | H | H | $CH_2CF_2CF_2CF_3$ |
| A-01 48 | 2 | H | O((2-$CF_3$)Ph) | H | H | $CH_2CF_2CF_2CF_3$ |
| A-0149 | 2 | H | O((3-$CF_3$)Ph) | H | H | $CH_2CF_2CF_2CF_3$ |
| A-0150 | 2 | H | O((4-$CF_3$)Ph) | H | H | $CH_2CF_2CF_2CF_3$ |
| A-0151 | 2 | H | O((2-CN)Ph) | H | H | $CH_2CF_2CF_2CF_3$ |

(continued)

| Compd. No. | n | $R^3$ | $R^4$ | $R^5$ | $R^6$ | $R^{10}$ |
|---|---|---|---|---|---|---|
| A-0152 | 2 | H | O((3-CN)Ph) | H | H | $CH_2CF_2CF_2CF_3$ |
| A-0153 | 2 | H | O((4-CN)Ph) | H | H | $CH_2CF_2CF_2CF_3$ |
| A-0154 | 2 | H | O(pyridin-2-yl) | H | H | $CH_2CF_2CF_2CF_3$ |
| A-0155 | 2 | H | O(pyridin-3-yl) | H | H | $CH_2CF_2CF_2CF_3$ |
| A-0156 | 2 | H | O(pyridin-4-yl) | H | H | $CH_2CF_2CF_2CF_3$ |
| A-0157 | 2 | H | O((3-$CF_3$)pyridin-2-yl) | H | H | $CH_2CF_2CF_2CF_3$ |
| A-0158 | 2 | H | O((5-$CF_3$)pyridin-2-yl) | H | H | $CH_2CF_2CF_2CF_3$ |
| A-0159 | 2 | H | SH | H | H | $CH_2CF_2CF_2CF_3$ |
| A-0160 | 2 | H | $SF_4Cl$ | H | H | $CH_2CF_2CF_2CF_3$ |
| A-0161 | 2 | H | $SF_5$ | H | H | $CH_2CF_2CF_2CF_3$ |
| A-0 162 | 2 | H | SMe | H | H | $CH_2CF_2CF_2CF_3$ |
| A-0163 | 2 | H | S(=O)Me | H | H | $CH_2CF_2CF_2CF_3$ |
| A-01 fi4 | 2 | H | $S(=O)_2Me$ | H | H | $CH_2CF_2CF_2CF_3$ |
| A-0165 | 2 | H | $SCF_3$ | H | H | $CH_2CF_2CF_2CF_3$ |
| A-0166 | 2 | H | $S(=O)CF_3$ | H | H | $CH_2CF_2CF_2CF_3$ |
| A-0167 | 2 | H | $S(=O)_2CF_3$ | H | H | $CH_2CF_2CF_2CF_3$ |
| A-0168 | 2 | H | S(cPr) | H | H | $CH_2CF_2CF_2CF_3$ |
| A-0 169 | 2 | H | S(=O)(cPr) | H | H | $CH_2CF_2CF_2CF_3$ |
| A-0170 | 2 | H | $S(=O)_2$(cPr) | H | H | $CH_2CF_2CF_2CF_3$ |

Table 5

| Compd. No. | n | $R^3$ | $R^4$ | $R^5$ | $R^6$ | $R^{10}$ |
|---|---|---|---|---|---|---|
| A-0171 | 2 | H | S((2,2-$F_2$)cPr) | H | H | $CH_2CF_2CF_2CF_3$ |
| A-0172 | 2 | H | S(=O)((2,2-$F_2$)cPr) | H | H | $CH_2CF_2CF_2CF_3$ |
| A-0173 | 2 | H | $S(=O)_2$((2,2-$F_2$)cPr) | H | H | $CH_2CF_2CF_2CF_3$ |
| A-0174 | 2 | H | $SCH_2$(cPr) | H | H | $CH_2CF_2CF_2CF_3$ |
| A-0175 | 2 | H | $S(=O)CH_2$(cPr) | H | H | $CH_2CF_2CF_2CF_3$ |
| A-0176 | 2 | H | $S(=O)_2CH_2$(cPr) | H | H | $CH_2CF_2CF_2CF_3$ |
| A-0177 | 2 | H | $SCH_2$((2,2-$F_2$)cPr) | H | H | $CH_2CF_2CF_2CF_3$ |
| A-0178 | 2 | H | $S(=O)CH_2$((2,2-$F_2$)cPr) | H | H | $CH_2CF_2CF_2CF_3$ |
| A-0179 | 2 | H | $S(=O)_2CH_2$((2,2-$F_2$)cPr) | H | H | $CH_2CF_2CF_2CF_3$ |
| A-0180 | 2 | H | $NH_2$ | H | H | $CH_2CF_2CF_2CF_3$ |
| A-0181 | 2 | H | INHMe | H | H | $CH_2CF_2CF_2CF_3$ |
| A-0182 | 2 | IH | $N(Me)_2$ | H | H | $CH_2CF_2CF_2CF_3$ |

(continued)

| Compd. No. | n | $R^3$ | $R^4$ | $R^5$ | $R^6$ | $R^{10}$ |
|---|---|---|---|---|---|---|
| A-0183 | 2 | H | $N{=}S({=}O)Me_2$ | H | H | $CH_2CF_2CF_2CF_3$ |
| A-0184 | 2 | H | C(=O)H | H | H | $CH_2CF_2CF_2CF_3$ |
| A-0185 | 2 | H | C(=O)Me | H | H | $CH_2CF_2CF_2CF_3$ |
| A-0186 | 2 | H | $C({=}O)CF_3$ | H | H | $CH_2CF_2CF_2CF_3$ |
| A-0187 | 2 | H | CH(=N-OH) | H | H | $CH_2CF_2CF_2CF_3$ |
| A-0188 | 2 | H | $C({=}N{-}OH)NH_2$ | H | H | $CH_2CF_2CF_2CF_3$ |
| A-0189 | 2 | H | CH(=N-OMe) | H | H | $CH_2CF_2CF_2CF_3$ |
| A-0190 | 2 | H | CH(=N-OEt) | H | H | $CH_2CF_2CF_2CF_3$ |
| A-0191 | 2 | H | $CH({=}N{-}OCH_2CF_3)$ | H | H | $CH_2CF_2CF_2CF_3$ |
| A-0192 | 2 | H | C(=O)OH | H | H | $CH_2CF_2CF_2CF_3$ |
| A-0193 | 2 | H | C(=O)OMe | H | H | $CH_2CF_2CF_2CF_3$ |
| A-0 194 | 2 | H | C(=O)OEt | H | H | $CH_2CF_2CF_2CF_3$ |
| A-0195 | 2 | H | $C({=}O)NH_2$ | H | H | $CH_2CF_2CF_2CF_3$ |
| A-0196 | 2 | H | C(=O)NHMe | H | H | $CH_2CF_2CF_2CF_3$ |
| A-0197 | 2 | H | $C({=}O)NMe_2$ | H | H | $CH_2CF_2CF_2CF_3$ |
| A-0198 | 2 | H | $C({=}O)NHCH_2CF_3$ | H | H | $CH_2CF_2CF_2CF_3$ |
| A-0199 | 2 | H | C(=O)NHcPr | H | H | $CH_2CF_2CF_2CF_3$ |
| A-0200 | 2 | H | C(=O)N(OMe)Me | H | H | $CH_2CF_2CF_2CF_3$ |
| A-0201 | 2 | H | F | H | H | $CH_2CF_2CHFCF_3$ |
| A-0202 | 2 | H | Cl | H | H | $CH_2CF_2CHFCF_3$ |
| A-0203 | 2 | H | Br | H | H | $CH_2CF_2CHFCF_3$ |
| A-0204 | 2 | H | I | H | H | $CH_2CF_2CHFCF_3$ |
| A-0205 | 2 | H | $N_3$ | H | H | $CH_2CF_2CHFCF_3$ |
| A-0206 | 2 | H | CN | H | H | $CH_2CF_2CHFCF_3$ |
| A-0207 | 2 | H | Me | H | H | $CH_2CF_2CHFCF_3$ |
| A-0208 | 2 | H | $CH_2CN$ | H | H | $CH_2CF_2CHFCF_3$ |
| A-0209 | 0 | H | $CF_3$ | H | H | $CH_2CF_2CHFCF_3$ |
| A-0210 | 2 | H | $CF_3$ | H | H | $CH_2CF_2CHFCF_3$ |
| A-0211 | 2 | H | $CHF_2$ | H | H | $CH_2CF_2CHFCF_3$ |
| A-0212 | 2 | H | $CF(CF_3)_2$ | H | H | $CH_2CF_2CHFCF_3$ |
| A-0213 | 2 | H | cPr | H | H | $CH_2CF_2CHFCF_3$ |
| A-0214 | 2 | H | cBu | H | H | $CH_2CF_2CHFCF_3$ |

Table 6

| Compd. No. | n | $R^3$ | $R^4$ | $R^5$ | $R^6$ | $R^{10}$ |
|---|---|---|---|---|---|---|
| A-0215 | 2 | H | cPen | H | H | $CH_2CF_2CHFCF_3$ |
| A-0216 | 2 | H | (2,2-$F_2$)cPr | H | H | $CH_2CF_2CHFCF_3$ |
| A-0217 | 2 | H | (2,2-$Cl_2$)cPr | H | H | $CH_2CF_2CHFCF_3$ |
| A-0218 | 2 | H | (1-CN)cPr | H | H | $CH_2CF_2CHFCF_3$ |
| A-0219 | 2 | H | (4-$CF_3$)Ph | H | H | $CH_2CF_2CHFCF_3$ |
| A-0220 | 2 | H | (4-CN)Ph | H | H | $CH_2CF_2CHFCF_3$ |
| A-0221 | 2 | H | pyridin-2-yl | H | H | $CH_2CF_2CHFCF_3$ |
| A-0222 | 2 | H | pyridin-3-yl | H | H | $CH_2CF_2CHFCF_3$ |
| A-0223 | 2 | H | pyridin-4-yl | H | H | $CH_2CF_2CHFCF_3$ |
| A-0224 | 2 | H | 1,2,3-triazol-1-yl | H | H | $CH_2CF_2CHFCF_3$ |
| A-0225 | 2 | H | 1,2,4-triazol-1-yl | H | H | $CH_2CF_2CHFCF_3$ |
| A-0226 | 2 | H | 1,2,4-oxadiazol-3-yl | H | H | $CH_2CF_2CHFCF_3$ |
| A-0227 | 2 | H | 1,2,4-oxadiazol-5-yl | H | H | $CH_2CF_2CHFCF_3$ |
| A-0228 | 2 | H | 1,2,5-oxadiazol-3-yl | H | H | $CH_2CF_2CHFCF_3$ |
| A-0229 | 2 | H | 1,3,4-oxadiazol-2-yl | H | H | $CH_2CF_2CHFCF_3$ |
| A-0230 | 2 | H | $OCF_3$ | H | H | $CH_2CF_2CHFCF_3$ |
| A-0231 | 2 | H | $OCHF_2$ | H | H | $CH_2CF_2CHFCF_3$ |
| A-0232 | 2 | H | $OCF_2CF_3$ | H | H | $CH_2CF_2CHFCF_3$ |
| A-0233 | 2 | H | $OCF_2CHF_2$ | H | H | $CH_2CF_2CHFCF_3$ |
| A-0234 | 2 | H | $OCF_2CHFCl$ | H | H | $CH_2CF_2CHFCF_3$ |
| A-0235 | 2 | H | $OCH_2CF_2CHF_2$ | H | H | $CH_2CF_2CHFCF_3$ |
| A-0236 | 2 | H | $OCF_2CHFCF_3$ | H | H | $CH_2CF_2CHFCF_3$ |
| A-0237 | 2 | H | $OC(Me)_2CN$ | H | H | $CH_2CF_2CHFCF_3$ |
| A-0238 | 2 | H | $OCF_2CHFOCF_3$ | H | H | $CH_2CF_2CHFCF_3$ |
| A-0239 | 2 | H | O(cPr) | H | H | $CH_2CF_2CHFCF_3$ |
| A-0240 | 2 | H | $OCH_2$(cPr) | H | H | $CH_2CF_2CHFCF_3$ |
| A-0241 | 2 | H | O((2,2-$F_2$)cPr) | H | H | $CH_2CF_2CHFCF_3$ |
| A-0242 | 2 | H | O((2,2-$Cl_2$)cPr) | H | H | $CH_2CF_2CHFCF_3$ |
| A-0243 | 2 | H | O((1-CN)cPr) | H | H | $CH_2CF_2CHFCF_3$ |
| A-0244 | 2 | H | $OCH_2$((2,2-$F_2$)cPr) | H | H | $CH_2CF_2CHFCF_3$ |
| A-0245 | 2 | H | $OCH_2$((2,2-$Cl_2$)cPr) | H | H | $CH_2CF_2CHFCF_3$ |
| A-0246 | 2 | H | $OCH_2$((1-CN)cPr) | H | H | $CH_2CF_2CHFCF_3$ |
| A-0247 | 2 | H | O((4-$CF_3$)Ph) | H | H | $CH_2CF_2CHFCF_3$ |
| A-0248 | 2 | H | O((4-CN)Ph) | H | H | $CH_2CF_2CHFCF_3$ |

(continued)

| Compd. No. | n | $R^3$ | $R^4$ | $R^5$ | $R^6$ | $R^{10}$ |
|---|---|---|---|---|---|---|
| A-0249 | 2 | H | $SF_5$ | H | H | $CH_2CF_2CHFCF_3$ |
| A-0250 | 2 | H | $N=S(=O)Me_2$ | H | H | $CH_2CF_2CHFCF_3$ |
| A-0251 | 2 | H | CH(=N-OH) | H | H | $CH_2CF_2CHFCF_3$ |
| A-0252 | 2 | H | $CH(=N-OCH_2Me)$ | H | H | $CH_2CF_2CHFCF_3$ |
| A-0253 | 2 | H | $CH(=N-OCH_2CF_3)$ | H | H | $CH_2CF_2CHFCF_3$ |
| A-0254 | 2 | H | H | F | H | $CH_2CF_2CF_2CF_3$ |
| A-0255 | 2 | H | H | Cl | H | $CH_2CF_2CF_2CF_3$ |
| A-0256 | 2 | H | H | Br | H | $CH_2CF_2CF_2CF_3$ |
| A-0257 | 2 | H | H | I | H | $CH_2CF_2CF_2CF_3$ |
| A-0258 | 2 | H | H | Me | H | $CH_2CF_2CF_2CF_3$ |

Table 7

| Compd. No. | n | $R^3$ | $R^4$ | $R^5$ | $R^6$ | $R^{10}$ |
|---|---|---|---|---|---|---|
| A-0259 | 2 | H | H | $CF_3$ | H | $CH_2CF_2CF_2CF_3$ |
| A-0260 | 2 | H | H | cPr | H | $CH_2CF_2CF_2CF_3$ |
| A-0261 | 2 | H | H | $(2,2-F_2)cPr$ | H | $CH_2CF_2CF_2CF_3$ |
| A-0262 | 2 | H | H | (1-CN)cPr | H | $CH_2CF_2CF_2CF_3$ |
| A-0263 | 2 | H | H | $OCF_3$ | H | $CH_2CF_2CF_2CF_3$ |
| A-0264 | 2 | H | H | $OCHF_2$ | H | $CH_2CF_2CF_2CF_3$ |
| A-0265 | 2 | H | H | $OC(Me)_2CN$ | H | $CH_2CF_2CF_2CF_3$ |
| A-0266 | 2 | H | H | $SCF_3$ | H | $CH_2CF_2CF_2CF_3$ |
| A-0267 | 2 | H | H | $S(=O)CF_3$ | H | $CH_2CF_2CF_2CF_3$ |
| A-0268 | 2 | H | H | $S(=O)_2CF_3$ | H | $CH_2CF_2CF_2CF_3$ |
| A-0269 | 2 | H | H | F | H | $CH_2CF_2CHFCF_3$ |
| A-0270 | 2 | H | H | Cl | H | $CH_2CF_2CHFCF_3$ |
| A-0271 | 2 | IH | H | Br | H | $CH_2CF_2CHFCF_3$ |
| A-0272 | 2 | H | H | I | H | $CH_2CF_2CHFCF_3$ |
| A-0273 | 2 | H | H | Me | H | $CH_2CF_2CHFCF_3$ |
| A-0274 | 2 | H | H | $CF_3$ | H | $CH_2CF_2CHFCF_3$ |
| A-0275 | 2 | H | H | cPr | H | $CH_2CF_2CHFCF_3$ |
| A-0276 | 2 | H | H | $(2,2-F_2)cPr$ | H | $CH_2CF_2CHFCF_3$ |
| A-0277 | 2 | H | H | (1-CN)cPr | H | $CH_2CF_2CHFCF_3$ |
| A-0278 | 2 | H | H | $OCF_3$ | H | $CH_2CF_2CHFCF_3$ |
| A-0279 | 2 | H | H | $OCHF_2$ | H | $CH_2CF_2CHFCF_3$ |

(continued)

| Compd. No. | n | $R^3$ | $R^4$ | $R^5$ | $R^6$ | $R^{10}$ |
|---|---|---|---|---|---|---|
| A-0280 | 2 | H | H | $OC(Me)_2CN$ | H | $CH_2CF_2CHFCF_3$ |
| A-0281 | 2 | H | H | $SCF_3$ | H | $CH_2CF_2CHFCF_3$ |
| A-0282 | 2 | H | H | $S(=O)CF_3$ | H | $CH_2CF_2CHFCF_3$ |
| A-0283 | 2 | H | H | $S(=O)_2CF_3$ | H | $CH_2CF_2CHFCF_3$ |
| A-0284 | 2 | H | H | H | F | $CH_2CF_2CF_2CF_3$ |
| A-0285 | 2 | H | H | H | Cl | $CH_2CF_2CF_2CF_3$ |
| A-0286 | 2 | H | H | H | Br | $CH_2CF_2CF_2CF_3$ |
| A-0287 | 2 | H | H | H | I | $CH_2CF_2CF_2CF_3$ |
| A-0288 | 2 | H | H | H | Me | $CH_2CF_2CF_2CF_3$ |
| A-0289 | 2 | H | H | H | $CF_3$ | $CH_2CF_2CF_2CF_3$ |
| A-0290 | 2 | H | H | H | cPr | $CH_2CF_2CF_2CF_3$ |
| A-0291 | 2 | H | H | H | $(2,2\text{-}F_2)$cPr | $CH_2CF_2CF_2CF_3$ |
| A-0292 | 2 |  | H | H | (1-CN)cPr | $CH_2CF_2CF_2CF_3$ |
| A-0293 | 2 | H | H | H | $OCF_3$ | $CH_2CF_2CF_2CF_3$ |
| A-0294 | 2 | H | H | H | $OCHF_2$ | $CH_2CF_2CF_2CF_3$ |
| A-0295 | 2 | H | H | H | $OC(Me)_2CN$ | $CH_2CF_2CF_2CF_3$ |
| A-0296 | 2 | H | H | H | $SCF_3$ | $CH_2CF_2CF_2CF_3$ |
| A-0297 | 2 | H | H | H | $S(=O)CF_3$ | $CH_2CF_2CF_2CF_3$ |
| A-0298 | 2 | H | H | H | $S(=O)_2CF_3$ | $CH_2CF_2CF_2CF_3$ |
| A-0299 | 2 | H | H | H | F | $CH_2CF_2CHFCF_3$ |
| A-0300 | 2 | H | H | H | Cl | $CH_2CF_2CHFCF_3$ |
| A-0301 | 2 | H | H | H | Br | $CH_2CF_2CHFCF_3$ |
| A-0302 | 2 | H | H | H | I | $CH_2CF_2CHFCF_3$ |

Table 8

| Compd. No. | n | $R^3$ | $R^4$ | $R^5$ | $R^6$ | $R^{10}$ |
|---|---|---|---|---|---|---|
| A-0303 | 2 | H | H | H | Me | $CH_2CF_2CHFCF_3$ |
| A-0304 | 2 | H | H | H | $CF_3$ | $CH_2CF_2CHFCF_3$ |
| A-0305 | 2 | H | H | H | cPr | $CH_2CF_2CHFCF_3$ |
| A-0306 | 2 | H | H | H | $(2,2\text{-}F_2)$cPr | $CH_2CF_2CHFCF_3$ |
| A-0307 | 2 | H | H | H | (1-CN)cPr | $CH_2CF_2CHFCF_3$ |
| A-0308 | 2 | H | H | H | $OCF_3$ | $CH_2CF_2CHFCF_3$ |
| A-0309 | 2 | H | H | H | $OCHF_2$ | $CH_2CF_2CHFCF_3$ |
| A-0310 | 2 | H | H | H | $OC(Me)_2CN$ | $CH_2CF_2CHFCF_3$ |

(continued)

| Compd. No. | n | $R^3$ | $R^4$ | $R^5$ | $R^6$ | $R^{10}$ |
|---|---|---|---|---|---|---|
| A-0311 | 2 | H | H | H | $SCF_3$ | $CH_2CF_2CHFCF_3$ |
| A-0312 | 2 | H | H | H | $S(=O)CF_3$ | $CH_2CF_2CHFCF_3$ |
| A-0313 | 2 | H | H | H | $S(=O)_2CF_3$ | $CH_2CF_2CHFCF_3$ |
| A-0314 | 2 | H | H | H | H | Me |
| A-0315 | 2 | H | H | H | H | Et |
| A-0316 | 2 | H | H | H | H | iPr |
| A-0317 | 2 | H | H | H | H | tBu |
| A-0318 | 2 | H | H | H | H | nPr |
| A-0319 | 2 | H | H | H | H | $CH_2$(iPr) |
| A-0320 | 2 | H | H | H | H | CH(Me)$CH_2$Me |
| A-0321 | 2 | H | H | H | H | $CH_2$(tBu) |
| A-0322 | 2 | H | H | H | H | $CH_2(CH_2)_2$Me |
| A-0323 | 2 | H | H | H | H | $CH_2CH_2$(iPr) |
| A-0324 | 2 | H | H | H | H | $CH_2$CH(Me)$CH_2$Me |
| A-0325 | 2 | H | H | H | H | CH(Me)$CH_2CH_2$Me |
| A-0326 | 2 | H | H | H | H | $CH_2CH_2$(tBu) |
| A-0327 | 2 | H | H | H | H | nPen |
| A-0328 | 2 | H | H | H | H | $CH_2CH_2CH_2$(iPr) |
| A-0329 | 2 | H | H | H | H | nHex |
| A-0330 | 2 | H | H | H | H | $CH_2CH_2CH_2CH_2$(iPr) |
| A-0331 | 2 | H | H | H | H | $CH_2(CH_2)_5$Me |
| A-0332 | 2 | H | H | H | H | $CH_2(CH_2)_6$Me |
| A-0333 | 2 | H | H | H | H | $CH_2$OMe |
| A-0334 | 2 | H | H | H | H | $CH_2$OEt |
| A-0335 | 2 | H | H | H | H | $CH_2CH_2$OMe |
| A-0336 | 2 | H | H | H | H | $CH_2CH_2$OEt |
| A-0337 | 2 | H | H | H | H | $CH_2CH_2CH_2$OMe |
| A-0338 | 2 | H | H | H | H | $CF_3$ |
| A-0339 | 2 | H | H | H | H | $CHF_2$ |
| A-0340 | 2 | H | H | H | H | $CH_2CF_3$ |
| A-0341 | 2 | H | H | H | H | $CH_2CHF_2$ |
| A-0342 | 2 | H | H | H | H | $CH_2CClF_2$ |
| A-0343 | 2 | H | H | H | H | $CF_2CHCl_2$ |
| A-0344 | 2 | H | H | H | H | $CF_2$CHFCl |
| A-0345 | 2 | H | H | H | H | $CH_2CH_2$Cl |

(continued)

| Compd. No. | n | $R^3$ | $R^4$ | $R^5$ | $R^6$ | $R^{10}$ |
|---|---|---|---|---|---|---|
| A-0346 | 2 | H | H | H | H | $CH_2CCl_3$ |

Table 9

| Compd. No. | n | $R^3$ | $R^4$ | $R^5$ | $R^6$ | $R^{10}$ |
|---|---|---|---|---|---|---|
| A-0347 | 2 | H | H | H | H | $CH_2CBrF_2$ |
| A-0348 | 2 | H | H | H | H | $CF_2CF_3$ |
| A-0349 | 2 | H | H | H | H | $CF_2CHF_2$ |
| A-0350 | 2 | H | H | H | H | $CH_2CF_2CF_3$ |
| A-0351 | 2 | H | H | H | H | $CH_2CF_2CHF_2$ |
| A-0352 | 2 | H | H | H | H | $CF_2CHFCF_3$ |
| A-0353 | 2 | H | H | H | H | $CH_2CF_2CF_2CF_2CF_3$ |
| A-0354 | 2 | H | H | H | H | $CH_2CF_2CF_2CF_2CHF_2$ |
| A-0355 | 2 | H | H | H | H | $CF_2CHFOMe$ |
| A-0356 | 2 | H | H | H | H | $CF_2CHFOCH_2Me$ |
| A-0357 | 2 | H | H | H | H | $CH_2CH_2OCH_2CF_3$ |
| A-0358 | 2 | H | H | H | H | $CF_2CHFOCF_3$ |
| A-0359 | 2 | H | H | H | H | $CF_2CHFOCF_2CF_3$ |
| A-0360 | 2 | H | H | H | H | $CF_2CHFOCF_2CF_2CF_3$ |
| A-0361 | 2 | H | H | H | H | $CH_2CH=CH_2$ |
| A-0362 | 2 | H | H | H | H | $CH_2CH=CHCl$ |
| A-0363 | 2 | H | H | H | H | $CH_2CH=CCl_2$ |
| A-0364 | 2 | H | H | H | H | $CH_2CH_2CF=CF_2$ |
| A-0365 | 2 | H | H | H | H | $CH_2CH_2CH=CF_2$ |
| A-0366 | 2 | H | H | H | H | $CH_2C\equiv CH$ |
| A-0367 | 2 | H | H | H | H | $CH_2C\equiv CMe$ |
| A-0368 | 2 | H | H | H | H | $CH_2C\equiv CC(Me)_3$ |
| A-0369 | 2 | H | H | H | H | $CH_2C\equiv C(cPr)$ |
| A-0370 | 2 | H | H | H | H | $CH_2C\equiv CCl$ |
| A-0371 | 2 | H | H | H | H | $CH_2C\equiv CCF_3$ |
| A-0372 | 2 | H | H | H | H | cPr |
| A-0373 | 2 | H | H | H | H | cBu |
| A-0374 | 2 | H | H | H | H | cPen |
| A-0375 | 2 | H | H | \|H | H | cHex |
| A-0376 | 2 | H | H | H | H | $(2,2-F_2)cPr$ |

(continued)

| Compd. No. | n | $R^3$ | $R^4$ | $R^5$ | $R^6$ | $R^{10}$ |
|---|---|---|---|---|---|---|
| A-0377 | 2 | H | H | H | H | $(4,4\text{-}F_2)cHex$ |
| A-0378 | 2 | H | H | H | H | $(4\text{-}CF_3)cHex$ |
| A-0379 | 2 | H | H | H | H | $CH_2(cPr)$ |
| A-0380 | 2 | H | H | H | H | $CH_2(cBu)$ |
| A-0381 | 2 | H | H | H | H | $CH_2(cPen)$ |
| A-0382 | 2 | H | H | H | H | $CH_2(cHex)$ |
| A-0383 | 2 | H | H | H | H | $CH_2(2,2\text{-}F_2)cPr$ |
| A-0384 | 2 | H | H | H | H | $CH_2(2,2\text{-}Cl_2)cPr$ |
| A-0385 | 2 | H | H | H | H | $CH_2((4,4\text{-}F_2)cHex)$ |
| A-0386 | 2 | H | $CF_3$ | H | H | Me |
| A-0387 | 2 | H | $CF_3$ | H | H | Et |
| A-0388 | 2 | H | $CF_3$ | H | H | iPr |
| A-0389 | 2 | H | $CF_3$ | H | H | tBu |
| A-0390 | 2 | H | $CF_3$ | H | H | nPr |

Table 10

| Compd. No. | n | $R^3$ | $R^4$ | $R^5$ | $R^6$ | $R^{10}$ |
|---|---|---|---|---|---|---|
| A-0391 | 2 | H | $CF_3$ | H | H | $CH_2(iPr)$ |
| A-0392 | 2 | H | $CF_3$ | H | H | $CH(Me)CH_2Me$ |
| A-0393 | 2 | H | $CF_3$ | H | H | $CH_2(tBu)$ |
| A-0394 | 2 | H | $CF_3$ | H | H | nBu |
| A-0395 | 2 | H | $CF_3$ | H | H | $CH_2CH_2(iPr)$ |
| A-0396 | 2 | H | $CF_3$ | H | H | $CH_2CH(Me)CH_2Me$ |
| A-0397 | 2 | H | $CF_3$ | H | H | $CH(Me)CH_2CH_2Me$ |
| A-0398 | 2 | H | $CF_3$ | H | H | $CH_2CH_2(tBu)$ |
| A-0399 | 2 | H | $CF_3$ | H | H | nPen |
| A-0400 | 2 | H | $CF_3$ | H | H | $CH_2CH_2CH_2(iPr)$ |
| A-0401 | 2 | H | $CF_3$ | H | H | nHex |
| A-0402 | 2 | H | $CF_3$ | H | H | $CH_2CH_2CH_2CH_2(iPr)$ |
| A-0403 | 2 | H | $CF_3$ | H | H | $CH_2(CH_2)_5Me$ |
| A-0404 | 2 | H | $CF_3$ | H | H | $CH_2(CH_2)_6Me$ |
| A-0405 | 2 | H | $CF_3$ | H | H | $CH_2OMe$ |
| A-0406 | 2 | H | $CF_3$ | H | H | $CH_2OEt$ |
| A-0407 | 2 | H | $CF_3$ | H | H | $CH_2CH_2OMe$ |

(continued)

| Compd. No. | n | $R^3$ | $R^4$ | $R^5$ | $R^6$ | $R^{10}$ |
|---|---|---|---|---|---|---|
| A-0408 | 2 | H | $CF_3$ | H | H | $CH_2CH_2OCH_2Me$ |
| A-0409 | 2 | H | $CF_3$ | H | H | $CH_2CH_2CH_2OMe$ |
| A-041 0 | 2 | H | $CF_3$ | H | H | $CF_3$ |
| A-0411 | 2 | H | $CF_3$ | H | H | $CHF_2$ |
| A-0412 | 2 | H | $CF_3$ | H | H | $CH_2CF_3$ |
| A-0413 | 2 | H | $CF_3$ | H | H | $CH_2CHF_2$ |
| A-0414 | 2 | H | $CF_3$ | H | H | $CH_2CClF_2$ |
| A-0415 | 2 | H | $CF_3$ | H | H | $CF_2CHCl_2$ |
| A-0416 | 2 | H | $CF_3$ | H | H | $CF_2CHFCl$ |
| A-0417 | 2 | H | $CF_3$ | H | H | $CH_2CH_2Cl$ |
| A-0418 | 2 | H | $CF_3$ | H | H | $CH_2CCl_3$ |
| A-0419 | 2 | H | $CF_3$ | H | H | $CH_2CBrF_2$ |
| A-0420 | 2 | H | $CF_3$ | H | H | $CF_2CF_3$ |
| A-0421 | 2 | H | $CF_3$ | H | H | $CF_2CHF_2$ |
| A-0422 | 2 | H | $CF_3$ | H | H | $CH_2CF_2CF_3$ |
| A-0423 | 2 | H | $CF_3$ | H | H | $CH_2CF_2CHF_2$ |
| A-0424 | 2 | H | $CF_3$ | H | H | $CF_2CHFCF_3$ |
| A-0425 | 2 | H | $CF_3$ | H | H | $CH_2CF_2CF_2CF_2CF_3$ |
| A-0426 | 2 | H | $CF_3$ | H | H | $CH_2CF_2CF_2CF_2CHF_2$ |
| A-0427 | 2 | H | $CF_3$ | H | H | $CF_2CHFOMe$ |
| A-0428 | 2 | H | $CF_3$ | H | H | $CF_2CHFOCH_2Me$ |
| A-0429 | 2 | H | $CF_3$ | H | H | $CH_2CH_2OCH_2CF_3$ |
| A-0430 | 2 | H | $CF_3$ | H | H | $CF_2CHFOCF_3$ |
| A-0431 | 2 | H | $CF_3$ | H | H | $CF_2CHFOCF_2CF_3$ |
| A-0432 | 2 | H | $CF_3$ | H | H | $CF_2CHFOCF_2CF_2CF_3$ |
| A-0433 | 2 | H | $CF_3$ | H | H | $CH_2CH=CH_2$ |
| A-0434 | 2 | H | $CF_3$ | H | H | $CH_2CH=CHCl$ |

Table 11

| Compd. No. | n | $R^3$ | $R^4$ | $R^5$ | $R^6$ | $R^{10}$ |
|---|---|---|---|---|---|---|
| A-0435 | 2 | H | $CF_3$ | H | H | $CH_2CH=CCl_2$ |
| A-0436 | 2 | H | $CF_3$ | H | H | $CH_2CH_2CF=CF_2$ |
| A-0437 | 2 | H | $CF_3$ | H | H | $CH_2CH_2CH=CF_2$ |
| A-0438 | 2 | H | $CF_3$ | H | H | $CH_2C\equiv CH$ |

(continued)

| Compd. No. | n | $R^3$ | $R^4$ | $R^5$ | $R^6$ | $R^{10}$ |
|---|---|---|---|---|---|---|
| A-0439 | 2 | H | $CF_3$ | H | H | $CH_2C{\equiv}CMe$ |
| A-0440 | 2 | H | $CF_3$ | H | H | $CH_2C{\equiv}C(tBu)$ |
| A-0441 | 2 | H | $CF_3$ | H | H | $CH_2C{\equiv}C(cPr)$ |
| A-0442 | 2 | H | $CF_3$ | H | H | $CH_2C{\equiv}Cl$ |
| A-0443 | 2 | H | $CF_3$ | H | H | $CH_2C{\equiv}CCF_3$ |
| A-0444 | 2 | H | $CF_3$ | H | H | cPr |
| A-0445 | 2 | H | $CF_3$ | H | H | cBu |
| A-0446 | 2 | H | $CF_3$ | H | H | cPen |
| A-0447 | 2 | H | $CF_3$ | H | H | cHex |
| A-0448 | 2 | H | $CF_3$ | H | H | $(2,2\text{-}Cl_2)cPr$ |
| A-0449 | 2 | H | $CF_3$ | H | H | $(2,2\text{-}F_2)cHex$ |
| A-0450 | 2 | H | $CF_3$ | H | H | $(4\text{-}CF_3)cHex$ |
| A-0451 | 2 | H | $CF_3$ | H | H | $CH_2(cPr)$ |
| A-0452 | 2 | H | $CF_3$ | H | H | $CH_2(cBu)$ |
| A-0453 | 2 | H | $CF_3$ | H | H | $CH_2(cPen)$ |
| A-0454 | 2 | H | $CF_3$ | H | H | $CH_2(cHex)$ |
| A-0455 | 2 | H | $CF_3$ | H | H | $CH_2((2,2\text{-}F_2)cPr)$ |
| A-0456 | 2 | H | $CF_3$ | H | H | $CH_2((2,2\text{-}Cl_2)cPr)$ |
| A-0457 | 2 | H | $CF_3$ | H | H | $CH_2((2,2\text{-}F_2)cHex)$ |
| A-0458 | 2 | H | $CF_3$ | H | H | $CH_2(2\text{-}CF_3\text{-}1,3\text{-dioxalan-}2\text{-yl})$ |
| A-0459 | 0 | F | H | H | H | H |
| A-0460 | 0 | Cl | H | H | H | H |
| A-0461 | 0 | Br | H | H | H | H |
| A-0462 | 0 | I | H | H | H | H |
| A-0463 | 0 | Me | H | H | H | H |
| A-0464 | 0 | $CF_3$ | H | H | H | H |
| A-0465 | 0 | cPr | H | H | H | H |
| A-0466 | 0 | $(2,2\text{-}F_2)cPr$ | H | H | H | H |
| A-0467 | 0 | (1-CN)cPr | H | H | H | H |
| A-04E8 | 0 | $OCF_3$ | H | H | H | H |
| A-0469 | 0 | $OCHF_2$ | H | H | H | H |
| A-0470 | 0 | $OC(Me)_2CN$ | H | H | H | H |
| A-0471 | 0 | $SCF_3$ | H | H | H | H |
| A-0472 | 0 | $S(=O)CF_3$ | H | H | H | H |
| A-0473 | 0 | $S(=O)_2CF_3$ | H | H | H | H |

(continued)

| Compd. No. | n | $R^3$ | $R^4$ | $R^5$ | $R^6$ | $R^{10}$ |
|---|---|---|---|---|---|---|
| A-0474 | 0 | H | F | H | H | H |
| A-0475 | 0 | H | Cl | H | H | H |
| A-0476 | 0 | H | Br | H | H | H |
| A-0477 | 0 | H | I | H | H | H |
| A-0478 | 0 | H | Me | H | H | H |

Table 12

| Compd. No. | n | $R^3$ | $R^4$ | $R^5$ | $R^6$ | $R^{10}$ |
|---|---|---|---|---|---|---|
| A-0479 | 0 | H | $CF_3$ | H | H | H |
| A-0480 | 0 | H | cPr | H | H | H |
| A-0481 | 0 | H | (2,2-$F_2$)cPr | H | H | H |
| A-0482 | 0 | H | (1-CN)cPr | H | H | H |
| A-0483 | 0 | H | $OCF_3$ | H | H | H |
| A-0484 | 0 | H | $OCHF_2$ | H | H | H |
| A-0485 | 0 | H | $OC(Me)_2CN$ | H | H | H |
| A-0486 | 0 | H | $SCF_3$ | H | H | H |
| A-0487 | 0 | H | $S(=O)CF_3$ | H | H | H |
| A-0488 | 0 | H | $S(=O)_2CF_3$ | H | H | H |
| A-0489 | 0 | H | H | F | H | H |
| A-0490 | 0 | H | H | Cl | H | H |
| A-0491 | 0 | H | H | Br | H | H |
| A-0492 | 0 | H | H | I | H | H |
| A-0493 | 0 | H | H | Me | H | H |
| A-0494 | 0 | H | H | $CF_3$ | H | H |
| A-0495 | 0 | H | H | cPr | H | H |
| A-0496 | 0 | H | H | (2,2-$F_2$)cPr | H | H |
| A-0497 | 0 | H | H | (1-CN)cPr | H | H |
| A-0498 | 0 | H | H | $OCF_3$ | H | H |
| A-0499 | 0 | H | H | $OCHF_2$ | H | H |
| A-0500 | 0 | H | H | $OC(Me)_2CN$ | H | H |
| A-0501 | 0 | H | H | $SCF_3$ | H | H |
| A-0502 | 0 | H | H | $S(=O)CF_3$ | H | H |
| A-0503 | 0 | H | H | $S(=O)_2CF_3$ | H | H |
| A-0504 | 0 | H | H | H | F | H |
| A-0505 | 0 | H | H | H | Cl | H |
| A-0506 | 0 | H | H | H | Br | H |

(continued)

| Compd. No. | n | $R^3$ | $R^4$ | $R^5$ | $R^6$ | $R^{10}$ |
|---|---|---|---|---|---|---|
| A-0507 | 0 | H | H | H | I | H |
| A-0508 | 0 | H | H | H | Me | H |
| A-0509 | 0 | H | H | H | $CF_3$ | H |
| A-0510 | 0 | H | H | H | cPr | H |
| A-0511 | 0 | H | H | H | (2,2-$F_2$)cPr | H |
| A-0512 | 0 | H | H | H | (1-CN)cPr | H |
| A-0513 | 0 | H | H | H | $OCF_3$ | H |
| A-0514 | 0 | H | H | H | $OCHF_2$ | H |
| A-0515 | 0 | H | H | H | $OC(Me)_2CN$ | H |
| A-0516 | 0 | H | H | H | $SCF_3$ | H |
| A-0517 | 0 | H | H | H | $S(=O)CF_3$ | H |
| A-0518 | 0 | H | H | H | $S(=O)_2CF_3$ | H |
| A-0519 | 2 | F | H | H | H | H |
| A-0520 | 2 | Cl | H | H | H | H |
| A-0521 | 2 | Br | H | H | H | H |
| A-0522 | 2 | I | H | H | H | H |

Table 13

| Compd. No. | n | $R^3$ | $R^4$ | $R^5$ | $R^6$ | $R^{10}$ |
|---|---|---|---|---|---|---|
| A-0523 | 2 | Me | H | H | H | H |
| A-0524 | 2 | $CF_3$ | H | H | H | H |
| A-0525 | 2 | cPr | H | H | H | H |
| A-0526 | 2 | (2,2-$F_2$)cPr | H | H | H | H |
| A-0527 | 2 | (1-CN)cPr | H | H | H | H |
| A-0528 | 2 | $OCF_3$ | H | H | H | H |
| A-0529 | 2 | $OCHF_2$ | H | H | H | H |
| A-0530 | 2 | $OC(Me)_2CN$ | H | H | H | H |
| A-0531 | 2 | $SCF_3$ | H | H | H | H |
| A-0532 | 2 | $S(=O)CF_3$ | H | H | H | H |
| A-0533 | 2 | $S(=O)_2CF_3$ | H | H | H | H |
| A-0534 | 2 | H | F | H | H | H |
| A-0535 | 2 | H | Cl | H | H | H |
| A-0536 | 2 | H | Br | H | H | H |
| A-0537 | 2 | H | I | H | H | H |
| A-0538 | 2 | H | Me | H | H | H |

(continued)

| Compd. No. | n | $R^3$ | $R^4$ | $R^5$ | $R^6$ | $R^{10}$ |
|---|---|---|---|---|---|---|
| A-0539 | 2 | H | $CF_3$ | H | H | H |
| A-0540 | 2 | H | cPr | H | H | H |
| A-0541 | 2 | H | $(2,2\text{-}F_2)$cPr | H | H | H |
| A-0542 | 2 | H | (1-CN)cPr | H | H | H |
| A-0543 | 2 | H | $OCF_3$ | H | H | H |
| A-0544 | 2 | H | $OCHF_2$ | H | H | H |
| A-0545 | 2 | H | $OC(Me)_2CN$ | H | H | H |
| A-0546 | 2 | H | $SCF_3$ | H | H | H |
| A-0547 | 2 | H | $S(=O)CF_3$ | H | H | H |
| A-0548 | 2 | H | $S(=O)_2CF_3$ | H | H | H |
| A-0549 | 2 | H | H | F | H | H |
| A-0550 | 2 | H | H | Cl | H | H |
| A-0551 | 2 | H | H | Br | H | H |
| A-0552 | 2 | H | H | I | H | H |
| A-0553 | 2 | H | H | Me | H | H |
| A-0554 | 2 | H | H | $CF_3$ | H | H |
| A-0555 | 2 | H | H | cPr | H | H |
| A-0556 | 2 | H | H | $(2,2\text{-}F_2)$cPr | H | H |
| A-0557 | 2 | H | H | (1-CN)cPr | H | H |
| A-0558 | 2 | H | H | $OCF_3$ | H | H |
| A-0559 | 2 | H | H | $OCHF_2$ | H | H |
| A-0560 | 2 | H | H | $OC(Me)_2CN$ | H | H |
| A-0561 | 2 | H | H | $SCF_3$ | H | H |
| A-0562 | 2 | H | H | $S(=O)CF_3$ | H | H |
| A-0563 | 2 | H | H | $S(=O)_2CF_3$ | H | H |
| A-0564 | 2 | H | H | H | F | H |
| A-0565 | 2 | H | H | H | Cl | H |
| A-0566 | 2 | H | H | H | Br | H |

Table 14

| Compd. No. | n | $R^3$ | $R^4$ | $R^5$ | $R^6$ | $R^{10}$ |
|---|---|---|---|---|---|---|
| A-0567 | 2 | H | H | H |  | H |
| A-0568 | 2 | H | H | H | Me | H |
| A-0569 | 2 | H | H | H | $CF_3$ | H |
| A-0570 | 2 | H | H | H | cPr | H |

(continued)

| Compd. No. | n | $R^3$ | $R^4$ | $R^5$ | $R^6$ | $R^{10}$ |
|---|---|---|---|---|---|---|
| A-0571 | 2 | H | H | H | (2,2-$F_2$)cPr | H |
| A-0572 | 2 | H | H | H | (1-CN)cPr | H |
| A-0573 | 2 | H | H | H | $OCF_3$ | H |
| A-0574 | 2 | H | H | H | $OCHF_2$ | H |
| A-0575 | 2 | H | H | H | $OC(Me)_2CN$ | H |
| A-0576 | 2 | H | H | H | $SCF_3$ | H |
| A-0577 | 2 | H | H | H | $S(=O)CF_3$ | H |
| A-0578 | 2 | H | H | H | $S(=O)_2CF_3$ | H |
| A-0579 | 2 | H | Et | H | H | $CH_2CF_2CF_2CF_3$ |
| A-0580 | 2 | H | $CHMe_2$ | H | H | $CH_2CF_2CF_2CF_3$ |
| A-0581 | 2 | H | $CH=CH_2$ | H | H | $CH_2CF_2CF_2CF_3$ |
| A-0582 | 2 | H | $C(Me)=CH_2$ | H | H | $CH_2CF_2CF_2CF_3$ |
| A-0583 | 2 | H | CH=CH(cPr) | H | H | $CH_2CF_2CF_2CF_3$ |
| A-0584 | 2 | H | C≡C(cPr) | H | H | $CH_2CF_2CF_2CF_3$ |
| A-0585 | 2 | H | (1-OH)cPr | H | H | $CH_2CF_2CF_2CF_3$ |
| A-0586 | 2 | H | (2-$CF_3$)Ph | H | H | $CH_2CF_2CF_2CF_3$ |
| A-0587 | 2 | H | (2-CN)Ph | H | H | $CH_2CF_2CF_2CF_3$ |
| A-0588 | 2 | H | pyrimidin-2-yl | H | H | $CH_2CF_2CF_2CF_3$ |
| A-0589 | 2 | H | pyrimidin-5-yl | H | H | $CH_2CF_2CF_2CF_3$ |
| A-0590 | 2 | H | thiazol-2-yl | H | H | $CH_2CF_2CF_2CF_3$ |
| A-0591 | 2 | H | 3,6-dihydro-2H-pyran-4-yl | H | H | $CH_2CF_2CF_2CF_3$ |
| A-0592 | 2 | H | S(=N-CN)Me | H | H | $CH_2CF_2CF_2CF_3$ |
| A-0593 | 2 | H | $S(=N-C(=O)CF_3)Me$ | H | H | $CH_2CF_2CF_2CF_3$ |
| A-0594 | 2 | H | S(=O)(=NH)Me | H | H | $CH_2CF_2CF_2CF_3$ |
| A-0595 | 2 | H | $P(=O)Me_2$ | H | H | $CH_2CF_2CF_2CF_3$ |
| A-0596 | 2 | H | C(=N-OMe)Me | H | H | $CH_2CF_2CF_2CF_3$ |
| A-0597 | 2 | H | C(=O)NH((1-CN)cPr) | H | H | $CH_2CF_2CF_2CF_3$ |
| A-0598 | 2 | H | C(=O)NHCH(=NOMe) | H | H | $CH_2CF_2CF_2CF_3$ |
| A-0599 | 2 | H | $C(=O)N(=CHNMe_2)$ | H | H | $CH_2CF_2CF_2CF_3$ |
| A-0600 | 2 | H | $C(=S)NH_2$ | H | H | $CH_2CF_2CF_2CF_3$ |
| A-0601 | 2 | H | $CF_3$ | H | H | (5-$CF_3$)pyridin-2-yl |
| A-0602 | 2 | H | $CF_3$ | H | H | (6-$CF_3$)pyridin-2-yl |
| A-0603 | 2 | H | cPr | H | H | (3-$CF_3$)pyridin-2-yl |
| A-0604 | 2 | H | cPr | H | H | (5-$CF_3$)pyridin-2-yl |

(continued)

| Compd. No. | n | $R^3$ | $R^4$ | $R^5$ | $R^6$ | $R^{10}$ |
|---|---|---|---|---|---|---|
| A-0605 | 2 | H | cPr | H | H | (6-$CF_3$)pyridin-2-yl |
| A-0606 | 2 | H | cPr | H | H | (5-$CF_3$)pyrimidin-2-yl |
| A-0607 | 2 | H | cPr | H | H | $CH_2CH_2CF=CF_2$ |
| A-0608 | 2 | H | IcHex | H | H | $CH_2CF_2CF_2CF_3$ |
| A-0609 | 2 | H | cyclohex-1-en-1-yl | H | H | $CH_2CF_2CF_2CF_3$ |
| A-0610 | 2 | H | $CH_2OH$ | H | H | $CH_2CF_2CF_2CF_3$ |
| A-0611 | 2 | H | tetrahydro-2H-pyran-4-yl | H | H | $CH_2CF_2CF_2CF_3$ |
| A-0612 | 2 | H | Br | H | H | $CH_2Ph$ |

Table 15

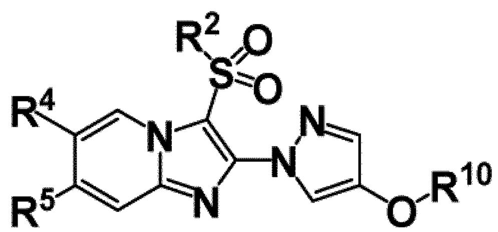

| Compd. No. | $R^2$ | $R^4$ | $R^5$ | $R^{10}$ |
|---|---|---|---|---|
| B-0001 | Me | H | H | $CH_2CF_2CF_2CF_3$ |
| B-0002 | nPr | H | H | $CH_2CF_2CF_2CF_3$ |
| B-0003 | iPr | H | H | $CH_2CF_2CF_2CF_3$ |
| B-0004 | $CH_2OMe$ | H | H | $CH_2CF_2CF_2CF_3$ |
| B-0005 | $CH_2Cl$ | H | H | $CH_2CF_2CF_2CF_3$ |
| B-0006 | $CH_2CF_3$ | H | H | $CH_2CF_2CF_2CF_3$ |
| B-0007 | $CF_2CF_3$ | H | H | $CH_2CF_2CF_2CF_3$ |
| B-0008 | cPr | H | H | $CH_2CF_2CF_2CF_3$ |
| B-0009 | (1-CN)cPr | H | H | $CH_2CF_2CF_2CF_3$ |
| B-0010 | $CH_2cPr$ | H | H | $CH_2CF_2CF_2CF_3$ |
| B-0011 | $CH_2CN$ | H | H | $CH_2CF_2CF_2CF_3$ |
| B-0012 | Me | H | H | $CH_2CF_2CHFCF_3$ |
| B-0013 | nPr | H | H | $CH_2CF_2CHFCF_3$ |
| B-0014 | iPr | H | H | $CH_2CF_2CHFCF_3$ |
| B-0015 | $CH_2OMe$ | H | H | $CH_2CF_2CHFCF_3$ |
| B-0016 | $CH_2Cl$ | H | H | $CH_2CF_2CHFCF_3$ |
| B-0017 | $CH_2CF_3$ | H | H | $CH_2CF_2CHFCF_3$ |
| B-0018 | $CF_2CF_3$ | H | H | $CH_2CF_2CHFCF_3$ |

(continued)

| 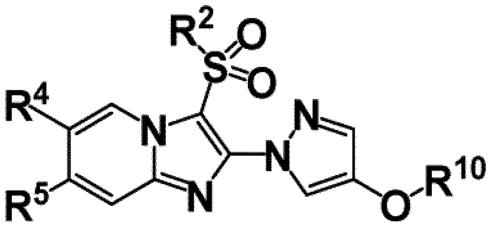 | | | | |
|---|---|---|---|---|
| Compd. No. | $R^2$ | $R^4$ | $R^5$ | $R^{10}$ |
| B-0019 | cPr | H | H | $CH_2CF_2CHFCF_3$ |
| B-0020 | (1-CN)cPr | H | H | $CH_2CF_2CHFCF_3$ |
| B-0021 | $CH_2cPr$ | H | H | $CH_2CF_2CHFCF_3$ |
| B-0022 | $CH_2CN$ | H | H | $CH_2CF_2CHFCF_3$ |
| B-0023 | Me | $CF_3$ | H | $CH_2CF_2CF_2CF_3$ |
| B-0024 | nPr | $CF_3$ | H | $CH_2CF_2CF_2CF_3$ |
| B-0025 | iPr | $CF_3$ | H | $CH_2CF_2CF_2CF_3$ |
| B-0026 | $CH_2OMe$ | $CF_3$ | H | $CH_2CF_2CF_2CF_3$ |
| B-0027 | $CH_2Cl$ | $CF_3$ | H | $CH_2CF_2CF_2CF_3$ |
| B-0028 | $CH_2CF_3$ | $CF_3$ | H | $CH_2CF_2CF_2CF_3$ |
| B-0029 | $CF_2CF_3$ | $CF_3$ | H | $CH_2CF_2CF_2CF_3$ |
| B-0030 | cPr | $CF_3$ | H | $CH_2CF_2CF_2CF_3$ |
| B-0031 | (1-CN)cPr | $CF_3$ | H | $CH_2CF_2CF_2CF_3$ |
| B-0032 | $CH_2cPr$ | $CF_3$ | H | $CH_2CF_2CF_2CF_3$ |
| B-0033 | $CH_2CN$ | $CF_3$ | H | $CH_2CF_2CF_2CF_3$ |
| B-0034 | Me | $CF_3$ | H | $CH_2CF_2CHFCF_3$ |
| B-0035 | nPr | $CF_3$ | H | $CH_2CF_2CHFCF_3$ |
| B-0036 | iPr | $CF_3$ | H | $CH_2CF_2CHFCF_3$ |
| B-0037 | $CH_2OMe$ | $CF_3$ | H | $CH_2CF_2CHFCF_3$ |
| B-0038 | $CH_2Cl$ | $CF_3$ | H | $CH_2CF_2CHFCF_3$ |

Table 16

| Compd. No. | $R^2$ | $R^4$ | $R^5$ | $R^{10}$ |
|---|---|---|---|---|
| B-0039 | $CH_2CF_3$ | $CF_3$ | H | $CH_2CF_2CHFCF_3$ |
| B-0040 | $CF_2CF_3$ | $CF_3$ | H | $CH_2CF_2CHFCF_3$ |
| B-0041 | cPr | $CF_3$ | H | $CH_2CF_2CHFCF_3$ |
| B-0042 | (1-CN)cPr | $CF_3$ | H | $CH_2CF_2CHFCF_3$ |
| B-0043 | $CH_2cPr$ | $CF_3$ | H | $CH_2CF_2CHFCF_3$ |
| B-0044 | $CH_2CN$ | $CF_3$ | H | $CH_2CF_2CHFCF_3$ |

(continued)

| Compd. No. | $R^2$ | $R^4$ | $R^5$ | $R^{10}$ |
|---|---|---|---|---|
| B-0045 | Me | H | $CF_3$ | $CH_2CF_2CF_2CF_3$ |
| B-0046 | nPr | H | $CF_3$ | $CH_2CF_2CF_2CF_3$ |
| B-0047 | iPr | H | $CF_3$ | $CH_2CF_2CF_2CF_3$ |
| B-0048 | $CH_2OMe$ | H | $CF_3$ | $CH_2CF_2CF_2CF_3$ |
| B-0049 | $CH_2Cl$ | H | $CF_3$ | $CH_2CF_2CF_2CF_3$ |
| B-0050 | $CH_2CF_3$ | H | $CF_3$ | $CH_2CF_2CF_2CF_3$ |
| B-0051 | $CF_2CF_3$ | H | $CF_3$ | $CH_2CF_2CF_2CF_3$ |
| B-0052 | cPr | H | $CF_3$ | $CH_2CF_2CF_2CF_3$ |
| B-0053 | (1-CN)cPr | H | $CF_3$ | $CH_2CF_2CF_2CF_3$ |
| B-0054 | $CH_2cPr$ | H | $CF_3$ | $CH_2CF_2CF_2CF_3$ |
| B-0055 | $CH_2CN$ | H | $CF_3$ | $CH_2CF_2CF_2CF_3$ |
| B-0056 | Me | H | $CF_3$ | $CH_2CF_2CHFCF_3$ |
| B-0057 | nPr | H | $CF_3$ | $CH_2CF_2CHFCF_3$ |
| B-0058 | iPr | H | $CF_3$ | $CH_2CF_2CHFCF_3$ |
| B-0059 | $CH_2OMe$ | H | $CF_3$ | $ICH_2CF_2CHFCF_3$ |
| B-0060 | $CH_2Cl$ | H | $CF_3$ | $CH_2CF_2CHFCF_3$ |
| B-0061 | $CH_2CF_3$ | H | $CF_3$ | $CH_2CF_2CHFCF_3$ |
| B-0062 | $CF_2CF_3$ | H | $CF_3$ | $CH_2CF_2CHFCF_3$ |
| B-0063 | cPr | H | $CF_3$ | $CH_2CF_2CHFCF_3$ |
| B-0064 | (1-CN)cPr | H | $CF_3$ | $CH_2CF_2CHFCF_3$ |
| B-0065 | $CH_2cPr$ | H | $CF_3$ | $CH_2CF_2CHFCF_3$ |
| B-0066 | $CH_2CN$ | H | $CF_3$ | $CH_2CF_2CHFCF_3$ |

Table 17

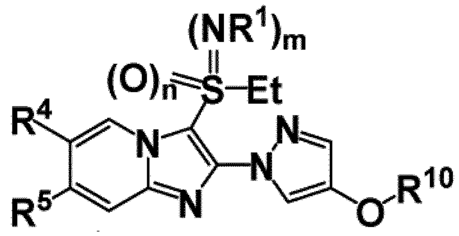

| Compd. No. | m | n | $R^1$ | $R^4$ | $R^5$ | $R^{10}$ |
|---|---|---|---|---|---|---|
| C-0001 | 1 | 0 | H | H | H | $CH_2CF_2CF_2CF_3$ |
| C-0002 | 1 | 0 | CN | H | H | $CH_2CF_2CF_2CF_3$ |
| C-0003 | 1 | 0 | C(=O)H | H | H | $CH_2CF_2CF_2CF_3$ |
| C-0004 | 1 | 0 | C(=O)Me | H | H | $CH_2CF_2CF_2CF_3$ |

(continued)

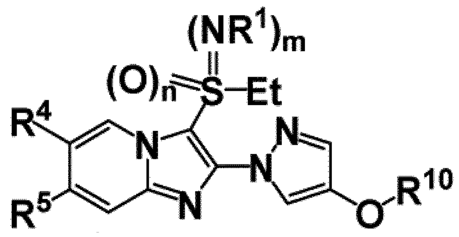

| Compd. No. | m | n | $R^1$ | $R^4$ | $R^5$ | $R^{10}$ |
|---|---|---|---|---|---|---|
| C-0005 | 1 | 0 | $C(=O)CF_3$ | H | H | $CH_2CF_2CF_2CF_3$ |
| C-0006 | 1 | 1 | H | H | H | $CH_2CF_2CF_2CF_3$ |
| C-0007 | 1 | 1 | CN | H | H | $CH_2CF_2CF_2CF_3$ |
| C-0008 | 1 | 111 | C(=O)H | H | H | $CH_2CF_2CF_2CF_3$ |
| C-0009 | 1 | 1 | C(=O)Me | H | H | $CH_2CF_2CF_2CF_3$ |
| C-0010 | 1 | 1 | $C(=O)CF_3$ | H | H | $CH_2CF_2CF_2CF_3$ |
| C-0011 | 1 | 0 | H | H | H | $CH_2CF_2CHFCF_3$ |
| C-0012 | 1 | 0 | CN | H | H | $CH_2CF_2CHFCF_3$ |
| C-0013 | 1 | 0 | C(=O)H | H | H | $CH_2CF_2CHFCF_3$ |
| C-0014 | 1 | 0 | C(=O)Me | H | H | $CH_2CF_2CHFCF_3$ |
| C-001 5 | 1 | 0 | $C(=O)CF_3$ | H | H | $CH_2CF_2CHFCF_3$ |
| C-0016 | 1 | 1 | H | H | H | $CH_2CF_2CHFCF_3$ |
| C-0017 | 1 | 1 | CN | H | H | $CH_2CF_2CHFCF_3$ |
| C-0018 | 1 | 1 | C(=O)H | H | H | $CH_2CF_2CHFCF_3$ |
| C-0019 | 1 | 1 | C(=O)Me | H | H | $CH_2CF_2CHFCF_3$ |
| C-0020 | 1 | 1 | $C(=O)CF_3$ | H | H | $CH_2CF_2CHFCF_3$ |
| C-0021 | 1 | 0 | H | $CF_3$ | H | $CH_2CF_2CF_2CF_3$ |
| C-0022 | 1 | 0 | CN | $CF_3$ | H | $CH_2CF_2CF_2CF_3$ |
| C-0023 | 1 | 0 | C(=O)H | $CF_3$ | H | $CH_2CF_2CF_2CF_3$ |
| C-0024 | 1 | 0 | C(=O)Me | $CF_3$ | H | $CH_2CF_2CF_2CF_3$ |
| C-0025 | 1 | 0 | $C(=O)CF_3$ | $CF_3$ | H | $CH_2CF_2CF_2CF_3$ |
| C-0026 | 1 | 1 | H | $CF_3$ | H | $CH_2CF_2CF_2CF_3$ |
| C-0027 | 1 | 1 | CN | $CF_3$ | H | $CH_2CF_2CF_2CF_3$ |
| C-0028 | 1 | 1 | C(=O)H | $CF_3$ | H | $CH_2CF_2CF_2CF_3$ |
| C-0029 | 1 | 1 | C(=O)Me | $CF_3$ | H | $CH_2CF_2CF_2CF_3$ |
| C-0030 | 1 | 1 | $C(=O)CF_3$ | $CF_3$ | H | $CH_2CF_2CF_2CF_3$ |
| C-0031 | 1 | 0 | H | $CF_3$ | H | $CH_2CF_2CHFCF_3$ |
| C-0032 | 1 | 0 | CN | $CF_3$ | H | $CH_2CF_2CHFCF_3$ |
| C-0033 | 1 | 0 | C(=O)H | $CF_3$ | H | $CH_2CF_2CHFCF_3$ |

(continued)

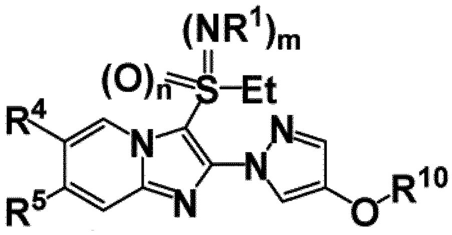

| Compd. No. | m | n | $R^1$ | $R^4$ | $R^5$ | $R^{10}$ |
|---|---|---|---|---|---|---|
| C-0034 | 1 | 0 | C(=O)Me | $CF_3$ | H | $CH_2CF_2CHFCF_3$ |
| C-0035 | 1 | 0 | $C(=O)CF_3$ | $CF_3$ | H | $CH_2CF_2CHFCF_3$ |
| C-0036 | 1 | 1 | H | $CF_3$ | H | $CH_2CF_2CHFCF_3$ |
| C-0037 | 1 | 1 | CN | $CF_3$ | H | $CH_2CF_2CHFCF_3$ |
| C-0038 | 1 | 1 | C(=O)H | $CF_3$ | H | $CH_2CF_2CHFCF_3$ |

Table 18

| Compd. No. | m | n | $R^1$ | $R^4$ | $R^5$ | $R^{10}$ |
|---|---|---|---|---|---|---|
| C-0039 | 1 | 1 | C(=O)Me | $CF_3$ | H | $CH_2CF_2CHFCF_3$ |
| C-0040 | 1 | 1 | $C(=O)CF_3$ | $CF_3$ | H | $CH_2CF_2CHFCF_3$ |
| C-0041 | 1 | 0 | H | H | $CF_3$ | $CH_2CF_2CF_2CF_3$ |
| C-0042 | 1 | 0 | CN | H | $CF_3$ | $CH_2CF_2CF_2CF_3$ |
| C-0043 | 1 | 0 | C(=O)H | H | $CF_3$ | $CH_2CF_2CF_2CF_3$ |
| C-0044 | 1 | 0 | C(=O)Me | H | $CF_3$ | $CH_2CF_2CF_2CF_3$ |
| C-0045 | 1 | 0 | $C(=O)CF_3$ | H | $CF_3$ | $CH_2CF_2CF_2CF_3$ |
| C-0046 | 1 | 1 | H | H | $CF_3$ | $CH_2CF_2CF_2CF_3$ |
| C-0047 | 1 | 1 | CN | H | $CF_3$ | $CH_2CF_2CF_2CF_3$ |
| C-0048 | 1 | 1 | C(=O)H | H | $CF_3$ | $CH_2CF_2CF_2CF_3$ |
| C-0049 | 1 | 1 | C(=O)Me | H | $CF_3$ | $CH_2CF_2CF_2CF_3$ |
| C-0050 | 1 | 1 | $C(=O)CF_3$ | H | $CF_3$ | $CH_2CF_2CF_2CF_3$ |
| C-0051 | 1 | 0 | H | H | $CF_3$ | $CH_2CF_2CHFCF_3$ |
| C-0052 | 1 | 0 | CN | H | $CF_3$ | $CH_2CF_2CHFCF_3$ |
| C-0053 | 1 | 0 | C(=O)H | H | $CF_3$ | $CH_2CF_2CHFCF_3$ |
| C-0054 | 1 | 0 | C(=O)Me | H | $CF_3$ | $CH_2CF_2CHFCF_3$ |
| C-0055 | 1 | 0 | $C(=O)CF_3$ | H | $CF_3$ | $CH_2CF_2CHFCF_3$ |
| C-0056 | 1 | 1 | H | H | $CF_3$ | $CH_2CF_2CHFCF_3$ |
| C-0057 | 1 | 1 | CN | H | $CF_3$ | $CH_2CF_2CHFCF_3$ |
| C-0058 | 1 | 1 | C(=O)H | H | $CF_3$ | $CH_2CF_2CHFCF_3$ |
| C-0059 | 1 | 1 | C(=O)Me | H | $CF_3$ | $CH_2CF_2CHFCF_3$ |

(continued)

| Compd. No. | m | n | $R^1$ | $R^4$ | $R^5$ | $R^{10}$ |
|---|---|---|---|---|---|---|
| C-0060 | 1 | 1 | $C(=O)CF_3$ | H | $CF_3$ | $CH_2CF_2CHFCF_3$ |

Table 19

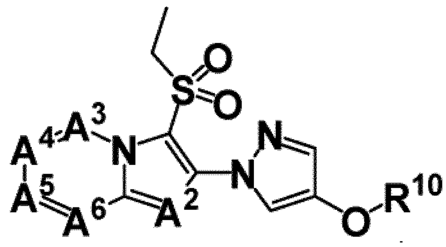

| Compd. No. | $A^2$ | $A^3$ | $A^4$ | $A^5$ | $A^6$ | $R^{10}$ |
|---|---|---|---|---|---|---|
| D-0001 | N | N | C-F | CH | CH | $CH_2CF_2CF_2CF_3$ |
| D-0002 | N | N | C-Cl | CH | CH | $CH_2CF_2CF_2CF_3$ |
| D-0003 | N | N | C-Br | CH | CH | $CH_2CF_2CF_2CF_3$ |
| D-0004 | N | N | C-I | CH | CH | $CH_2CF_2CF_2CF_3$ |
| D-0005 | N | N | C-Me | CH | CH | $CH_2CF_2CF_2CF_3$ |
| D-0006 | N | N | C-CF3 | CH | CH | $CH_2CF_2CF_2CF_3$ |
| D-0007 | N | N | C-cPr | CH | CH | $CH_2CF_2CF_2CF_3$ |
| D-0008 | N | N | C-(2,2-$F_2$)cPr | CH | CH | $CH_2CF_2CF_2CF_3$ |
| D-0009 | N | N | C-(1-CN)cPr | CH | CH | $CH_2CF_2CF_2CF_3$ |
| D-0010 | N | N | C-$OCF_3$ | CH | CH | $CH_2CF_2CF_2CF_3$ |
| D-0011 | N | N | C-$OCHF_2$ | CH | CH | $CH_2CF_2CF_2CF_3$ |
| D-0012 | N | N | C-OC(Me)$_2$CN | CH | CH | $CH_2CF_2CF_2CF_3$ |
| D-0013 | N | N | C-$SCF_3$ | CH | CH | $CH_2CF_2CF_2CF_3$ |
| D-0014 | N | N | C-S(=O)$CF_3$ | CH | CH | $CH_2CF_2CF_2CF_3$ |
| 0.0015 | N | N | C-S(=O)$_2CF_3$ | CH | CH | $CH_2CF_2CF_2CF_3$ |
| D-0016 | N | N | C-F | CH | CH | $CH_2CF_2CHFCF_3$ |
| D-0017 | N | N | C-Cl | CH | CH | $CH_2CF_2CHFCF_3$ |
| D-D018 | N | N | C-Br | CH | CH | $CH_2CF_2CHFCF_3$ |
| D-0019 | N | N | C-I | CH | CH | $CH_2CF_2CHFCF_3$ |
| D-0020 | N | N | C-Me | CH | CH | $CH_2CF_2CHFCF_3$ |
| D-0021 | N | N | C-$CF_3$ | CH | CH | $CH_2CF_2CHFCF_3$ |
| D-0022 | N | N | C-cPr | CH | CH | $CH_2CF_2CHFCF_3$ |
| D-0023 | N | N | C-(2,2-$F_2$)cPr | CH | CH | $CH_2CF_2CHFCF_3$ |
| D-0024 | N | N | C-(1-CN)cPr | CH | CH | $CH_2CF_2CHFCF_3$ |
| D-0025 | N | N | C-$OCF_3$ | CH | CH | $CH_2CF_2CHFCF_3$ |

(continued)

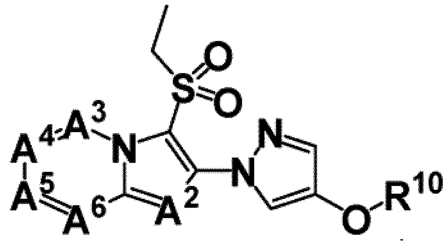

| Compd. No. | $A^2$ | $A^3$ | $A^4$ | $A^5$ | $A^6$ | $R^{10}$ |
|---|---|---|---|---|---|---|
| D-0026 | N | N | C-$OCHF_2$ | CH | CH | $CH_2CF_2CHFCF_3$ |
| D-0027 | N | N | C-$OC(Me)_2CN$ | CH | CH | $CH_2CF_2CHFCF_3$ |
| D-0028 | N | N | C-$SCF_3$ | CH | CH | $CH_2CF_2CHFCF_3$ |
| D-0029 | N | N | C-S(=O)$CF_3$ | CH | CH | $CH_2CF_2CHFCF_3$ |
| D-0030 | N | N | C-S(=O)$_2CF_3$ | CH | CH | $CH_2CF_2CHFCF_3$ |
| D-0031 | N | CH | C-F | N | CH | $CH_2CF_2CF_2CF_3$ |
| D-0032 | N | CH | C-Cl | N | CH | $CH_2CF_2CF_2CF_3$ |
| D-0033 | N | CH | C-Br | N | CH | $CH_2CF_2CF_2CF_3$ |
| D-0034 | N | CH | C-I | N | CH | $CH_2CF_2CF_2CF_3$ |
| D-0035 | N | CH | C-Me | N | CH | $CH_2CF_2CF_2CF_3$ |
| D-0036 | N | CH | C-$CF_3$ | N | CH | $CH_2CF_2CF_2CF_3$ |
| D-0037 | N | CH | C-cPr | N | CH | $CH_2CF_2CF_2CF_3$ |
| D-0038 | N | CH | C-(2,2-$F_2$)cPr | N | CH | $CH_2CF_2CF_2CF_3$ |

Table 20

| Compd. No. | $A^2$ | A3 | $A^4$ | $A^5$ | $A^6$ | $R^{10}$ |
|---|---|---|---|---|---|---|
| D-0039 | N | CH | C-(1-CN)cPr | N | CH | $CH_2CF_2CF_2CF_3$ |
| D-0040 | N | CH | C-$OCF_3$ | N | CH | $CH_2CF_2CF_2CF_3$ |
| D-0041 | N | CH | C-$OCHF_2$ | N | CH | $CH_2CF_2CF_2CF_3$ |
| 0-0042 | N | CH | C-$OC(Me)_2CN$ | N | CH | $CH_2CF_2CF_2CF_3$ |
| 0-0043 | N | CH | C-$SCF_3$ | N | CH | $CH_2CF_2CF_2CF_3$ |
| D-0044 | N | CH | C-S(=O)$CF_3$ | N | CH | $CH_2CF_2CF_2CF_3$ |
| D-0045 | N | CH | C-S(=O)$_2CF_3$ | N | CH | $CH_2CF_2CF_2CF_3$ |
| D-0046 | N | CH | C-F | N | CH | $CH_2CF_2CHFCF_3$ |
| D-0047 | N | CH | C-Cl | N | CH | $CH_2CF_2CHFCF_3$ |
| D-0048 | N | CH | C-Br | N | CH | $CH_2CF_2CHFCF_3$ |
| D-0049 | N | CH | C-I | N | CH | $CH_2CF_2CHFCF_3$ |
| D-0050 | N | CH | C-Me | N | CH | $CH_2CF_2CHFCF_3$ |
| D-0051 | N | CH | C-$CF_3$ | N | CH | $CH_2CF_2CHFCF_3$ |

(continued)

| Compd. No. | $A^2$ | A3 | $A^4$ | $A^5$ | $A^6$ | $R^{10}$ |
|---|---|---|---|---|---|---|
| D-0052 | N | CH | C-cPr | N | CH | $CH_2CF_2CHFCF_3$ |
| D-0053 | N | CH | C-(2,2-$F_2$)cPr | N | CH | $CH_2CF_2CHFCF_3$ |
| D-0054 | N | CH | C-(1-CN)cPr | N | CH | $CH_2CF_2CHFCF_3$ |
| D-0055 | N | CH | C-$OCF_3$ | N | CH | $CH_2CF_2CHFCF_3$ |
| D-0056 | N | CH | C-$OCHF_2$ | N | CH | $CH_2CF_2CHFCF_3$ |
| D-0057 | N | CH | C-OC(Me)$_2$CN | N | CH | $CH_2CF_2CHFCF_3$ |
| D-0058 | N | CH | C-$SCF_3$ | N | CH | $CH_2CF_2CHFCF_3$ |
| D-0059 | N | CH | C-S(=O)$CF_3$ | N | CH | $CH_2CF_2CHFCF_3$ |
| D-0060 | N | CH | C-S(=O)$_2CF_3$ | N | CH | $CH_2CF_2CHFCF_3$ |
| D-0061 | N | CH | C-F | CH | N | $CH_2CF_2CF_2CF_3$ |
| D-0062 | N | CH | C-Cl | CH | N | $CH_2CF_2CF_2CF_3$ |
| D-0063 | N | CH | C-Br | CH | N | $CH_2CF_2CF_2CF_3$ |
| 0-0064 | N | CH | C-I | CH | N | $CH_2CF_2CF_2CF_3$ |
| D-0065 | N | CH | C-Me | CH | N | $CH_2CF_2CF_2CF_3$ |
| D-0066 | N | CH | C-$CF_3$ | CH | N | $CH_2CF_2CF_2CF_3$ |
| D-0067 | N | CH | C-cPr | CH | N | $CH_2CF_2CF_2CF_3$ |
| D-0068 | N | CH | C-(2,2-$F_2$)cPr | CH | N | $CH_2CF_2CF_2CF_3$ |
| D-0069 | N | CH | C-(1-CN)cPr | CH | N | $CH_2CF_2CF_2CF_3$ |
| D-0070 | N | CH | C-$OCF_3$ | CH | N | $CH_2CF_2CF_2CF_3$ |
| D-0071 | N | CH | C-$OCHF_2$ | CH | N | $CH_2CF_2CF_2CF_3$ |
| D-0072 | N | CH | C-OC(Me)$_2$CN | CH | N | $CH_2CF_2CF_2CF_3$ |
| D-0073 | N | CH | C-$SCF_3$ | CH | N | $CH_2CF_2CF_2CF_3$ |
| D-0074 | N | CH | C-S(=O)$CF_3$ | CH | N | $CH_2CF_2CF_2CF_3$ |
| D-0075 | N | CH | C-S(=O)$_2CF_3$ | CH | N | $CH_2CF_2CF_2CF_3$ |
| D-0076 | N | CH | C-F | CH | N | $CH_2CF_2CHFCF_3$ |
| D-0077 | N | CH | C-Cl | CH | N | $CH_2CF_2CHFCF_3$ |
| D-0078 | N | CH | C-Br | CH | N | $CH_2CF_2CHFCF_3$ |
| D-0079 | N | CH | C-I | CH | N | $CH_2CF_2CHFCF_3$ |
| D-0080 | N | CH | C-Me | CH | N | $CH_2CF_2CHFCF_3$ |
| D-0081 | N | CH | C-$CF_3$ | CH | N | $CH_2CF_2CHFCF_3$ |
| D-0082 | N | CH | C-cPr | CH | N | $CH_2CF_2CHFCF_3$ |

Table 21

| Compd. No. | $A^2$ | $A^3$ | $A^4$ | $A^5$ | $A^6$ | $R^{10}$ |
|---|---|---|---|---|---|---|
| D-0083 | N | CH | C-(2,2-$F_2$)cPr | CH | N | $CH_2CF_2CHFCF_3$ |
| D-0084 | N | CH | C-(1-CN)cPr | CH | N | $CH_2CF_2CHFCF_3$ |
| D-0085 | N | CH | C-$OCF_3$ | CH | N | $CH_2CF_2CHFCF_3$ |
| D-0086 | N | CH | C-$OCHF_2$ | CH | N | $CH_2CF_2CHFCF_3$ |
| D-0087 | N | CH | C-OC$(Me)_2$CN | CH | N | $CH_2CF_2CHFCF_3$ |
| D-0088 | N | CH | C-$SCF_3$ | CH | N | $CH_2CF_2CHFCF_3$ |
| D-0089 | N | CH | C-S(=O)$CF_3$ | CH | N | $CH_2CF_2CHFCF_3$ |
| D-0090 | N | CH | C-S$(=O)_2CF_3$ | CH | N | $CH_2CF_2CHFCF_3$ |
| D-0091 | CH | CH | C-F | CH | CH | $CH_2CF_2CF_2CF_3$ |
| D-0092 | CH | CH | C-Cl | CH | CH | $CH_2CF_2CF_2CF_3$ |
| D-0093 | CH | CH | C-Br | CH | CH | $CH_2CF_2CF_2CF_3$ |
| D-0094 | CH | CH | C-I | CH | CH | $CH_2CF_2CF_2CF_3$ |
| D-0095 | CH | CH | C-Me | CH | CH | $CH_2CF_2CF_2CF_3$ |
| D-0096 | CH | CH | C-$CF_3$ | CH | CH | $CH_2CF_2CF_2CF_3$ |
| D-0097 | CH | CH | C-cPr | CH | CH | $CH_2CF_2CF_2CF_3$ |
| D-0098 | CH | CH | C-(2,2-$F_2$)cPr | CH | CH | $CH_2CF_2CF_2CF_3$ |
| D-0099 | CH | CH | C-(1-CN)cPr | CH | CH | $CH_2CF_2CF_2CF_3$ |
| D-0100 | CH | CH | C-$OCF_3$ | CH | CH | $CH_2CF_2CF_2CF_3$ |
| D-0101 | CH | CH | C-$OCHF_2$ | CH | CH | $CH_2CF_2CF_2CF_3$ |
| D-0102 | CH | CH | C-OC$(Me)_2$CN | CH | CH | $CH_2CF_2CF_2CF_3$ |
| D-0103 | CH | CH | C-$SCF_3$ | CH | CH | $CH_2CF_2CF_2CF_3$ |
| D-0104 | CH | CH | C-S(=O)$CF_3$ | CH | CH | $CH_2CF_2CF_2CF_3$ |
| D-0105 | CH | CH | C-S$(=O)_2CF_3$ | CH | CH | $CH_2CF_2CF_2CF_3$ |
| D-0106 | CH | CH | C-F | CH | CH | $CH_2CF_2CHFCF_3$ |
| D-0107 | CH | CH | C-Cl | CH | CH | $CH_2CF_2CHFCF_3$ |
| D-0108 | CH | CH | C-Br | CH | CH | $CH_2CF_2CHFCF_3$ |
| D-0109 | CH | CH | C-I | CH | CH | $CH_2CF_2CHFCF_3$ |
| D-0110 | CH | CH | C-Me | CH | CH | $CH_2CF_2CHFCF_3$ |
| D-0111 | CH | CH | C-$CF_3$ | CH | CH | $CH_2CF_2CHFCF_3$ |
| D-0112 | CH | CH | C-cPr | CH | CH | $CH_2CF_2CHFCF_3$ |
| D-0113 | CH | CH | C-(2,2-$F_2$)cPr | CH | CH | $CH_2CF_2CHFCF_3$ |
| D-0114 | CH | CH | C-(1-CN)cPr | CH | CH | $CH_2CF_2CHFCF_3$ |
| D-0115 | CH | CH | C-$OCF_3$ | CH | CH | $CH_2CF_2CHFCF_3$ |
| D-0116 | CH | CH | C-$OCHF_2$ | CH | CH | $CH_2CF_2CHFCF_3$ |

(continued)

| Compd. No. | $A^2$ | $A^3$ | $A^4$ | $A^5$ | $A^6$ | $R^{10}$ |
|---|---|---|---|---|---|---|
| D-0117 | CH | CH | C-OC(Me)$_2$CN | CH | CH | $CH_2CF_2CHFCF_3$ |
| D-0118 | CH | CH | C-SCF$_3$ | CH | CH | $CH_2CF_2CHFCF_3$ |
| D-0119 | CH | CH | C-S(=O)CF$_3$ | CH | CH | $CH_2CF_2CHFCF_3$ |
| D-0120 | CH | CH | C-S(=O)$_2$CF$_3$ | CH | CH | $CH_2CF_2CHFCF_3$ |
| D-0121 | CH | CH | CH | C-F | CH | $CH_2CF_2CF_2CF_3$ |
| D-0122 | CH | CH | CH | C-Cl | CH | $CH_2CF_2CF_2CF_3$ |
| D-0123 | CH | CH | CH | C-Br | CH | $CH_2CF_2CF_2CF_3$ |
| D-0124 | CH | CH | CH | C-I | CH | $CH_2CF_2CF_2CF_3$ |
| D-0125 | CH | CH | CH | C-Me | CH | $CH_2CF_2CF_2CF_3$ |
| D-0126 | CH | CH | CH | C-CF$_3$ | CH | $CH_2CF_2CF_2CF_3$ |

Table 22

| Compd. No. | $A^2$ | $A^3$ | $A^4$ | $A^5$ | $A^6$ | $R^{10}$ |
|---|---|---|---|---|---|---|
| D-0127 | CH | CH | CH | C-cPr | CH | $CH_2CF_2CF_2CF_3$ |
| 0-0128 | CH | CH | CH | C-(2,2-F$_2$)cPr | CH | $CH_2CF_2CF_2CF_3$ |
| D-0129 | CH | CH | CH | C-(1-CN)cPr | CH | $CH_2CF_2CF_2CF_3$ |
| D-01 30 | CH | CH | CH | C-OCF$_3$ | CH | $CH_2CF_2CF_2CF_3$ |
| D-0131 | CH | CH | CH | C-OCHF$_2$ | CH | $CH_2CF_2CF_2CF_3$ |
| D-0132 | CH | CH | CH | C-OC(Me)$_2$CN | CH | $CH_2CF_2CF_2CF_3$ |
| D-0133 | CH | CH | CH | C-SCF$_3$ | CH | $CH_2CF_2CF_2CF_3$ |
| D-0134 | CH | CH | CH | C-S(=O)CF$_3$ | CH | $CH_2CF_2CF_2CF_3$ |
| D-0135 | CH | CH | CH | C-S(=O)$_2$CF$_3$ | CH | $CH_2CF_2CF_2CF_3$ |
| D-0136 | CH | CH | CH | C-F | CH | $CH_2CF_2CHFCF_3$ |
| D-0137 | CH | CH | CH | C-Cl | CH | $CH_2CF_2CHFCF_3$ |
| D-0138 | CH | CH | CH | C-Br | CH | $CH_2CF_2CHFCF_3$ |
| D-0139 | CH | CH | CH | C-I | CH | $CH_2CF_2CHFCF_3$ |
| D-0140 | CH | CH | CH | C-Me | CH | $CH_2CF_2CHFCF_3$ |
| D-0141 | CH | CH | CH | C-CF$_3$ | CH | $CH_2CF_2CHFCF_3$ |
| D-0142 | CH | CH | CH | C-cPr | CH | $CH_2CF_2CHFCF_3$ |
| D-0143 | CH | CH | CH | C-(2,2-F$_2$)cPr | CH | $CH_2CF_2CHFCF_3$ |
| D-0144 | CH | CH | CH | C-(1-CN)cPr | CH | $CH_2CF_2CHFCF_3$ |
| D-0145 | CH | CH | CH | C-OCF$_3$ | CH | $CH_2CF_2CHFCF_3$ |
| D-0146 | CH | CH | CH | C-OCHF$_2$ | CH | $CH_2CF_2CHFCF_3$ |
| D-0147 | CH | CH | CH | C-OC(Me)$_2$CN | CH | $CH_2CF_2CHFCF_3$ |

(continued)

| Compd. No. | $A^2$ | $A^3$ | $A^4$ | $A^5$ | $A^6$ | $R^{10}$ |
|---|---|---|---|---|---|---|
| D-0148 | CH | CH | CH | C-$SCF_3$ | CH | $CH_2CF_2CHFCF_3$ |
| D-0149 | CH | CH | CH | C-S(=O)$CF_3$ | CH | $CH_2CF_2CHFCF_3$ |
| D-0 150 | CH | CH | CH | C-S(=O)$_2$$CF_3$ | CH | $CH_2CF_2CHFCF_3$ |
| D-0151 | CH | N | C-F | CH | CH | $CH_2CF_2CF_2CF_3$ |
| D-0152 | CH | N | C-Cl | CH | CH | $CH_2CF_2CF_2CF_3$ |
| D-0153 | CH | N | C-Br | CH | CH | $CH_2CF_2CF_2CF_3$ |
| D-0154 | CH | N | C-I | CH | CH | $CH_2CF_2CF_2CF_3$ |
| D-0155 | CH | N | C-Me | CH | CH | $CH_2CF_2CF_2CF_3$ |
| D-0156 | CH | N | C-$CF_3$ | CH | CH | $CH_2CF_2CF_2CF_3$ |
| D-0157 | CH | N | C-cPr | CH | CH | $CH_2CF_2CF_2CF_3$ |
| D-0158 | CH | N | C-(2,2-$F_2$)cPr | CH | CH | $CH_2CF_2CF_2CF_3$ |
| D-0159 | CH | N | C-(1-CN)cPr | CH | CH | $CH_2CF_2CF_2CF_3$ |
| D-0160 | CH | N | C-$OCF_3$ | CH | CH | $CH_2CF_2CF_2CF_3$ |
| D-0161 | CH | N | C-$OCHF_2$ | CH | CH | $CH_2CF_2CF_2CF_3$ |
| D-0162 | CH | N | C-OC(Me)$_2$CN | CH | CH | $CH_2CF_2CF_2CF_3$ |
| D-0163 | CH | N | C-$SCF_3$ | CH | CH | $CH_2CF_2CF_2CF_3$ |
| D-0164 | CH | N | C-S(=O)$CF_3$ | CH | CH | $CH_2CF_2CF_2CF_3$ |
| D-0165 | CH | N | C-S(=O)$_2$$CF_3$ | CH | CH | $CH_2CF_2CF_2CF_3$ |
| D-0166 | CH | N | C-F | CH | CH | $CH_2CF_2CHFCF_3$ |
| D-0167 | CH | N | C-Cl | CH | CH | $CH_2CF_2CHFCF_3$ |
| D-0168 | CH | N | C-Br | CH | CH | $CH_2CF_2CHFCF_3$ |
| D-0169 | CH | N | C-I | CH | CH | $CH_2CF_2CHFCF_3$ |
| D-0170 | CH | N | C-Me | CH | CH | $CH_2CF_2CHFCF_3$ |

Table 23

| Compd. No. | $A^2$ | $A^3$ | $A^4$ | $A^5$ | $A^6$ | $R^{10}$ |
|---|---|---|---|---|---|---|
| D-0171 | CH | N | C-$CF_3$ | CH | CH | $CH_2CF_2CHFCF_3$ |
| D-0172 | CH | N | C-cPr | CH | CH | $CH_2CF_2CHFCF_3$ |
| D-0173 | CH | N | C-(2,2-$F_2$)cPr | CH | CH | $CH_2CF_2CHFCF_3$ |
| D-0174 | CH | N | C-(1-CN)cPr | CH | CH | $CH_2CF_2CHFCF_3$ |
| D-0175 | CH | N | C-$OCF_3$ | CH | CH | $CH_2CF_2CHFCF_3$ |
| D-0176 | CH | N | C-$OCHF_2$ | CH | CH | $CH_2CF_2CHFCF_3$ |
| 0.0177 | CH | N | C-OC(Me)$_2$CN | CH | CH | $CH_2CF_2CHFCF_3$ |
| D-0178 | CH | N | C-$SCF_3$ | CH | CH | $CH_2CF_2CHFCF_3$ |

(continued)

| Compd. No. | $A^2$ | $A^3$ | $A^4$ | $A^5$ | $A^6$ | $R^{10}$ |
|---|---|---|---|---|---|---|
| D-0179 | CH | N | $C-S(=O)CF_3$ | CH | CH | $CH_2CF_2CHFCF_3$ |
| D-0180 | CH | N | $C-S(=O)_2CF_3$ | CH | CH | $CH_2CF_2CHFCF_3$ |

**Manufacturing methods**

On the other hand, although the compounds of the present invention represented by the general formula [I] can be produced according to the manufacturing methods shown below, their manufacturing methods are not limited thereto. Hereinafter, the expressions of, for example, "compound represented by the general formula [I]", "compound represented by the formula [I]" and "compound [I]" are taken as the same.

Manufacturing method 1

Among the compounds of the present invention, the compounds represented by the general formula [I] can be produced, for example, according to a method consisting of the reaction formula exemplified below.

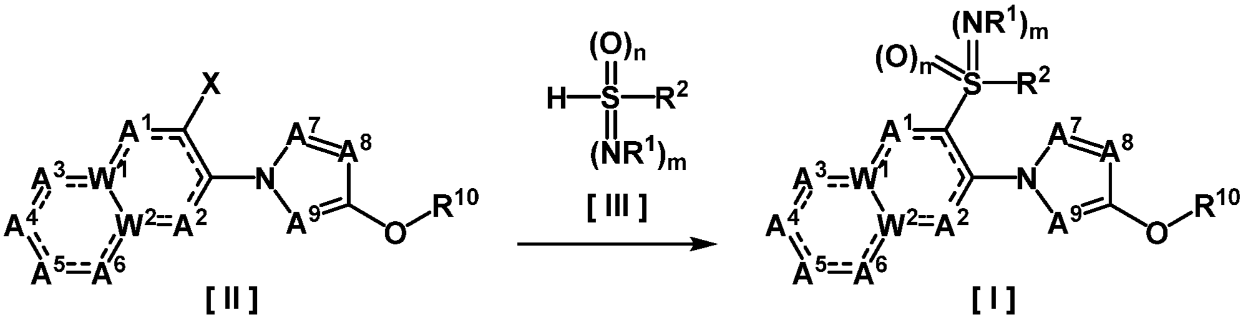

(wherein, X represents a halogen atom, or $C_1$-$C_6$ alkylsulfonyl, $C_1$-$C_6$ haloalkylsulfonyl, optionally substituted $C_6$-$C_{12}$ arylsulfonyl, $C_1$-$C_6$ alkylsulfonyloxy, $C_1$-$C_6$ haloalkylsulfonyloxy, optionally substituted $C_6$-$C_{12}$ arylsulfonyloxy, or $C_1$-$C_7$ acyloxy groups, $W^1$, $W^2$, m, n, $R^1$, $R^2$, $A^1$, $A^2$, $A^3$, $A^4$, $A^5$, $A^6$, $A^7$, $A^8$, $A^9$, and $R^{10}$ have the same meanings as described above.)

That is, the compounds represented by the general formula [I] can be produced by reacting a compound represented by the general formula [II] with a compound represented by the general formula [III] in a suitable solvent in the presence or absence of a suitable base. Furthermore, this reaction can also be carried out in the presence of a suitable catalyst.

The amount of compound [III] used in this reaction can be appropriately selected from the range of usually 1 to 100 mol, and is preferably 1 to 5 mol, with respect to 1 mol of compound [II].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate; metal hydrides such as lithium hydride, sodium hydride, potassium hydride; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 5 mol, and is preferably 0.1 to 2 mol, with respect to 1 mol of compound [II].

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines

such as triethylamine and tributylamine; water, or a mixed solvent thereof and the like. The amount of the solvent used is 0.1 to 1000 liters, preferably 0.2 to 100 liters, with respect to 1 mol of compound [II].

When a catalyst is used in this reaction, the catalyst that can be used includes a metal or metal salt and a compound as ligand. Such catalyst may be prepared in the reaction system (in situ) from a metal or metal salt and a compound as ligand described later or may be prepared in advance outside the reaction system and then added into the reaction system. The catalyst may contain other ingredients except the metal or metal salt and the compound ligand. Examples of the metal or metal salt include copper compounds such as metallic copper, copper acetate (monovalent), copper acetate (divalent), copper oxide (monovalent), copper oxide (divalent), copper chloride (monovalent), and copper iodide (monovalent); palladium compounds such as palladium-carbon, palladium chloride, palladium nitrate, palladium acetate, bis(triphenylphosphine)palladium dichloride, tetrakis(triphenylphosphine)palladium, tris(dibenzylideneacetone)dipalladium, and the like. Examples of the ligand include ethylenediamine, N,N-dimethylethylenediamine, N,N'-dimethylethylenediamine, tetramethylethylenediamine, 2,2'-bipyridine, 1,10-phenanthroline, neocuproine, 3,4,7,8-tetramethyl-1,10-phenanthroline, 2,9-diphenyl-1,10-phenanthroline, 4,7-diphenyl-1,10-phenanthroline, 4,5-bis(diphenylphosphino)-9,9-dimethylxanthene, triphenylphosphine, 1,1'-bis(diphenylphosphino)ferrocene, and the like, as well as salts thereof. The combination of the metal or the metal salt and the compound as ligand may be appropriately selected from the range of 0.1 to 10 mol, and is preferably 1 to 4 mol of the compound as ligand, with respect to 1 mol of the metal or the metal salt. The amount of the catalyst used may be appropriately selected from the range of 0.001 to 10 mol, and is preferably 0.01 to 5 mol as the metal or the metal salt, with respect to 1 mol of compound [II].

The reaction temperature of this reaction may be an arbitrary temperature selected from the range of -78°C to the reflux temperature in the reaction system. The reaction is preferably carried out at a temperature in the range of 0°C to 180°C.

Although the reaction time of this reaction varies depending on the reaction temperature, reaction substrate, reaction amount, etc., it is usually 10 minutes to 48 hours.

After the completion of reaction, compound [I] can be isolated by procedures such as adding the reaction mixture to water and filtering out the precipitated solid, or extracting the reaction mixture with an organic solvent and then concentrating. The isolated compound [I] can be further purified by column chromatography, recrystallization, distillation, or the like, if necessary.

Manufacturing method 2

Among the compounds of the present invention, the compounds represented by the general formula [I] can be produced, for example, according to the following method.

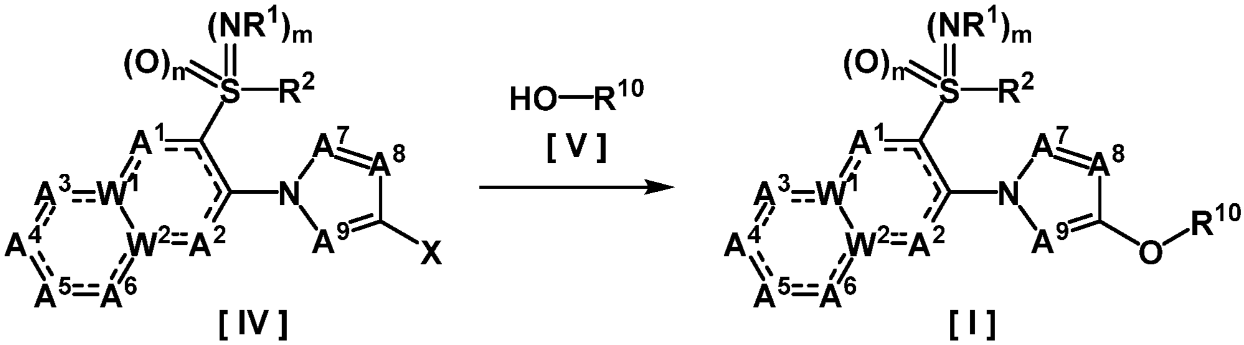


[ IV ] [ I ]

(wherein, X, $W^1$, $W^2$, m, n, $R^1$, $R^2$, $A^1$, $A^2$, $A^3$, $A^4$, $A^5$, $A^6$, $A^7$, $A^8$, $A^9$, and $R^{10}$ have the same meanings as described above.)

That is, the compounds represented by the general formula [I] can be produced by reacting a compound represented by the general formula [IV] with a compound represented by the general formula [V] in a suitable solvent in the presence or absence of a suitable base. Furthermore, this reaction can also be carried out in the presence of a suitable catalyst.

The amount of compound [V] used in this reaction can be appropriately selected from the range of usually 1 to 100 mol, and is preferably 1 to 5 mol, with respect to 1 mol of compound [IV].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such

as tripotassium phosphate; metal hydrides such as lithium hydride, sodium hydride, potassium hydride; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 5 mol, and is preferably 0.1 to 2 mol, with respect to 1 mol of compound [IV].

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [IV].

When a catalyst is used in this reaction, the catalyst that can be used includes a metal or metal salt and a compound as ligand. Such catalyst may be prepared in the reaction system (in situ) from a metal or metal salt and a compound as ligand described later or may be prepared in advance outside the reaction system and then added into the reaction system. The catalyst may contain other ingredients except the metal or metal salt and the compound ligand. Examples of the metal or metal salt include copper compounds such as metallic copper, copper acetate (monovalent), copper acetate (divalent), copper oxide (monovalent), copper oxide (divalent), copper chloride (monovalent), and copper iodide (monovalent); palladium compounds such as palladium-carbon, palladium chloride, palladium nitrate, palladium acetate, bis(triphenylphosphine)palladium dichloride, tetrakis(triphenylphosphine)palladium, tris(dibenzylideneacetone)dipalladium, and the like. Examples of the ligand include ethylenediamine, N,N-dimethylethylenediamine, N,N'-dimethylethylenediamine, tetramethylethylenediamine, 2,2'-bipyridine, 1,10-phenanthroline, neocuproine, 3,4,7,8-tetramethyl-1,10-phenanthroline, 2,9-diphenyl-1,10-phenanthroline, 4,7-diphenyl-1,10-phenanthroline, 4,5-bis(diphenylphosphino)-9,9-dimethylxanthene, triphenylphosphine, 1,1'-bis(diphenylphosphino)ferrocene, and the like, as well as salts thereof. The combination of the metal or the metal salt and the compound as ligand may be appropriately selected from the range of 0.1 to 10 mol, and is preferably 1 to 4 mol of the compound as ligand, with respect to 1 mol of the metal or the metal salt. The amount of the catalyst used may be appropriately selected from the range of 0.001 to 10 mol, and is preferably 0.01 to 5 mol as the metal or the metal salt, with respect to 1 mol of compound [IV].

The reaction temperature of this reaction may be an arbitrary temperature selected from the range of -78°C to the reflux temperature in the reaction system. The reaction is preferably carried out at a temperature in the range of 0°C to 180°C.

Although the reaction time of this reaction varies depending on the reaction temperature, reaction substrate, reaction amount, etc., it is usually 10 minutes to 48 hours.

After the completion of reaction, compound [I] can be isolated by procedures such as adding the reaction mixture to water and filtering out the precipitated solid, or extracting the reaction mixture with an organic solvent and then concentrating. The isolated compound [I] can be further purified by column chromatography, recrystallization, distillation, or the like, if necessary.

Manufacturing method 3

Among the compounds of the present invention, the compounds represented by the general formula [IX] can also be produced, for example, according to the following method.

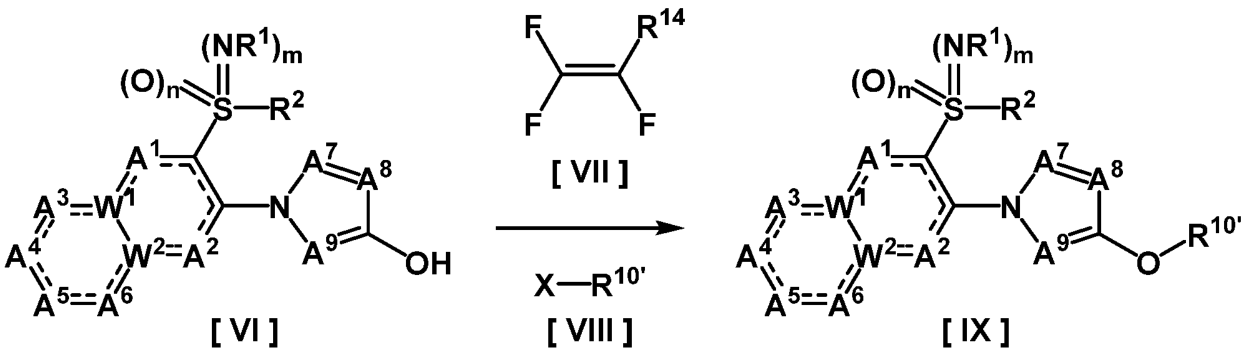

(wherein, $R^{14}$ represents a hydrogen atom, a halogen atom, a $C_1$-$C_6$ haloalkyl group, and a $C_1$-$C_6$ haloalkoxy group,

$R^{10'}$ represents a substituent other than hydrogen atom in $R^{10}$, and X, $W^1$, $W^2$, m, n, $R^1$, $R^2$, $A^1$, $A^2$, $A^3$, $A^4$, $A^5$, $A^6$, $A^7$, $A^8$, and $A^9$ have the same meanings as described above.)

That is, the compounds represented by the general formula [IX] can be produced by reacting a compound represented by the general formula [VI] with compound [VII] or compound [VIII] in a suitable solvent in the presence or absence of a suitable base.

The amount of compound [VII] or compound [VIII] used in this reaction can be appropriately selected from the range of usually 1 to 10 mol, and is preferably 1 to 2 mol, with respect to 1 mol of compound [VI].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate; metal hydrides such as lithium hydride, sodium hydride, potassium hydride; metal salts of alcohols such as sodium methoxide, sodium ethoxide, potassium tert-butoxide; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 10 mol, and is preferably 0 to 5 mol, with respect to 1 mol of compound [VI].

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [VI].

The reaction temperature of this reaction may be an arbitrary temperature selected from the range of -78°C to the reflux temperature in the reaction system. The reaction is preferably carried out at a temperature in the range of 0°C to 100°C.

Alghough the reaction time of this reaction varies depending on the reaction temperature, reaction substrate, reaction amount, etc., it is usually 10 minutes to 24 hours.

After the completion of reaction, compound [IX] can be isolated by procedures such as adding the reaction mixture to water and filtering out the precipitated solid, or extracting the reaction mixture with an organic solvent and then concentrating. The isolated compound [IX] can be further purified by column chromatography, recrystallization, distillation, or the like, if necessary.

Manufacturing method 4

Among the compounds of the present invention, the compounds represented by the general formula [I] can also be produced, for example, according to the following method.

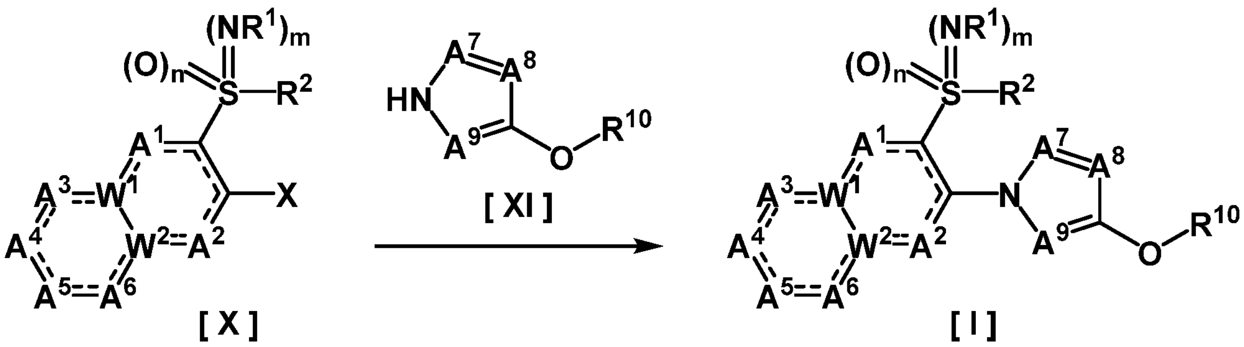

(wherein, X, $W^1$, $W^2$, m, n, $R^1$, $R^2$, $A^1$, $A^2$, $A^3$, $A^4$, $A^5$, $A^6$, $A^7$, $A^8$, $A^9$, and $R^{10}$ have the same meanings as described above.)

That is, the compounds represented by the general formula [I] can be produced by reacting a compound represented by the general formula [X] with a compound represented by the general formula [XI] in a suitable solvent in the presence or absence of a suitable base. Furthermore, this reaction can also be carried out in the presence of a suitable catalyst.

The amount of compound [XI] used in this reaction can be appropriately selected from the range of usually 1 to 5 mol, and is preferably 1 to 1.5 mol, with respect to 1 mol of compound [X].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate; metal hydrides such as lithium hydride, sodium hydride, potassium hydride; metal salts of alcohols such as sodium methoxide, sodium ethoxide, potassium tert-butoxide; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 5 mol, and is preferably 0.1 to 2 mol, with respect to 1 mol of compound [X] .

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [X].

When a catalyst is used in this reaction, the catalyst that can be used includes a metal or metal salt and a compound as ligand. Such catalyst may be prepared in the reaction system (*in situ*) from a metal or metal salt and a compound as ligand described later or may be prepared in advance outside the reaction system and then added into the reaction system. The catalyst may contain other ingredients except the metal or metal salt and the compound ligand. Examples of the metal or metal salt include copper compounds such as metallic copper, copper acetate (monovalent), copper acetate (divalent), copper oxide (monovalent), copper oxide (divalent), copper chloride (monovalent), and copper iodide (monovalent); palladium compounds such as palladium-carbon, palladium chloride, palladium nitrate, palladium acetate, bis(triphenylphosphine)palladium dichloride, tetrakis(triphenylphosphine)palladium, tris(dibenzylideneacetone)dipalladium, and the like. Examples of the ligand include ethylenediamine, N,N-dimethylethylenediamine, N,N'-dimethylethylenediamine, tetramethylethylenediamine, 2,2'-bipyridine, 1,10-phenanthroline, neocuproine, 3,4,7,8-tetramethyl-1,10-phenanthroline, 2,9-diphenyl-1,10-phenanthroline, 4,7-diphenyl-1,10-phenanthroline, 4,5-bis(diphenylphosphino)-9,9-dimethylxanthene, triphenylphosphine, 1,1'-bis(diphenylphosphino)ferrocene, and the like, as well as salts thereof. The combination of the metal or the metal salt and the compound as ligand may be appropriately selected from the range of 0.1 to 10 mol, and is preferably 1 to 4 mol of the compound as ligand, with respect to 1 mol of the metal or the metal salt. The amount of the catalyst used may be appropriately selected from the range of 0.001 to 10 mol, and is preferably 0.01 to 5 mol as the metal or the metal salt, with respect to 1 mol of compound [X].

The reaction temperature of this reaction may be an arbitrary temperature selected from the range of 0°C to the reflux temperature in the reaction system. The reaction is preferably carried out at a temperature in the range of 20°C to 180°C

Although the reaction time of this reaction varies depending on the reaction temperature, reaction substrate, reaction amount, etc., it is usually 10 minutes to 24 hours.

After the completion of reaction, compound [I] can be isolated by procedures such as adding the reaction mixture to water and filtering out the precipitated solid, or extracting the reaction mixture with an organic solvent and then concentrating. The isolated compound [I] can be further purified by column chromatography, recrystallization, distillation, or the like, if necessary.

Manufacturing method 5

Among the compounds of the present invention, the compounds represented by the general formula [XIV] can be produced, for example, according to the following method.

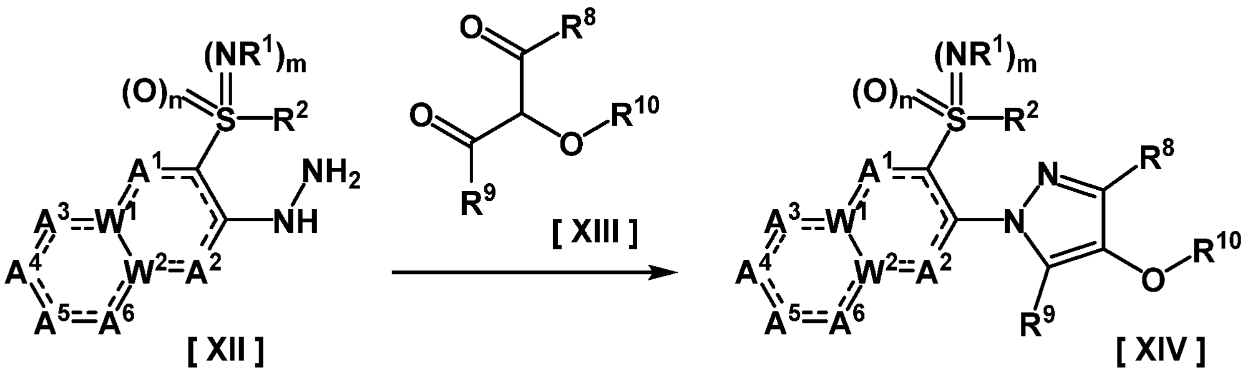

(wherein, $W^1$, $W^2$, m, n, $R^1$, $R^2$, $A^1$, $A^2$, $A^3$, $A^4$, $A^5$, $A^6$, $R^8$, $R^9$, and $R^{10}$ have the same meanings as described above.)

That is, the compounds represented by the general formula [XIV] can be produced by reacting a compound represented by the general formula [XII] with a compound represented by the general formula [XIII] in a suitable solvent in the presence or absence of a suitable base or a suitable acid.

The amount of compound [XIII] used in this reaction can be appropriately selected from the range of usually 1 to 5 mol, and is preferably 1 to 2 mol, with respect to 1 mol of compound [XII].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate; metal hydrides such as lithium hydride, sodium hydride, potassium hydride; metal salts of alcohols such as sodium methoxide, sodium ethoxide, potassium tert-butoxide; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 5 mol, and is preferably 0 to 2 mol, with respect to 1 mol of compound [XII].

When an acid is used in this reaction, examples of the acid that can be used include inorganic acids such as hydrochloric acid, hydrobromic acid, sulfuric acid, nitric acid, and phosphoric acid, and carboxylic acids such as acetic acid or trifluoroacetic acid. The amount of the acid used may be appropriately selected from the range of 0 to 5 mol, and is preferably 0 to 2 mol, with respect to 1 mol of compound [XII].

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [XII].

The reaction temperature of this reaction may be an arbitrary temperature selected from the range of 0°C to the reflux temperature in the reaction system. The reaction is preferably carried out at a temperature in the range of 20°C to 100°C.

Although the reaction time of this reaction varies depending on the reaction temperature, reaction substrate, reaction amount, etc., it is usually 10 minutes to 24 hours.

After the completion of reaction, compound [XIV] can be isolated by procedures such as adding the reaction mixture to water and filtering out the precipitated solid, or extracting the reaction mixture with an organic solvent and then concentrating. The isolated compound [XIV] can be further purified by column chromatography, recrystallization, distillation, or the like, if necessary.

Manufacturing method 6

Among the compounds of the present invention, the compounds represented by the general formula [XVI] can be produced, for example, according to the following method.

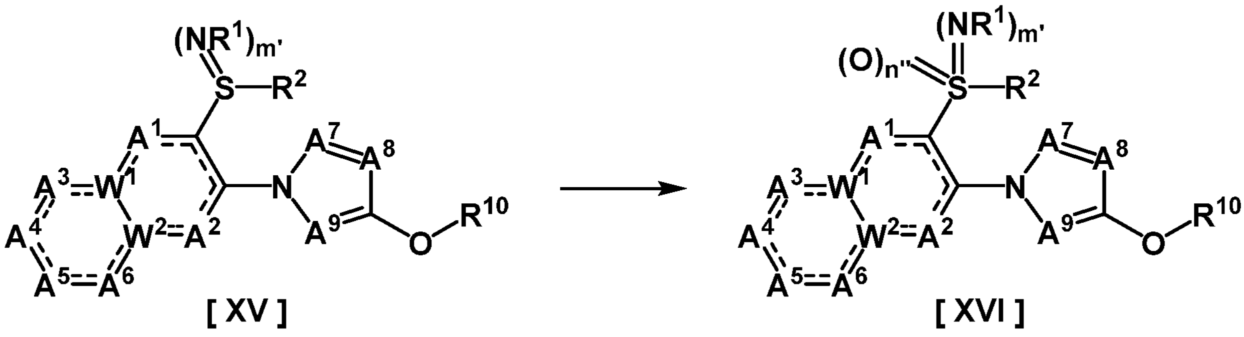

(wherein, m' represents 0 or 1, n" represents 1 or 2, the sum of m' and n" is 1 or 2, and $W^1$, $W^2$, $R^1$, $R^2$, $A^1$, $A^2$, $A^3$, $A^4$, $A^5$, $A^6$, $A^7$, $A^8$, $A^9$, and $R^{10}$ have the same meanings as described above. )

That is, the compounds represented by the general formula [XVI] can be produced by reacting a compound represented by the general formula [XV] with a suitable oxidizing agent in the presence or absence of a suitable catalyst in a suitable solvent.

Examples of the solvent that can be used in this reaction include aromatic hydrocarbons such as benzene, toluene, xylene, mesitylene, and chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane, water, or a mixed solvent thereof. The amount of the solvent used is 0.1 to 1000 liters, preferably 0.2 to 100 liters, with respect to 1 mol of compound [XV].

Examples of the oxidizing agent that can be used in this reaction include organic peroxides such as m-chloroperbenzoic acid, performic acid, peracetic acid, or trifluoroperacetic acid; inorganic peroxides such as hydrogen peroxide, potassium permanganate, Oxone (registered trademark) (trade name of E.I. DuPont, containing potassium peroxymonosulfate); salts of halogen oxoacids such as sodium periodate or sodium hypochlorite. The amount of the oxidizing agent to be used may be appropriately selected from the range of 0.5 to 5 mol, and is preferably 0.9 to 3 mol, with respect to 1 mol of compound [XV].

Examples of the catalyst that can be used in this reaction include ruthenium (III) chloride and its hydrate, vanadium pentoxide, sodium metavanadate, sodium tungstate, and the like. The amount of the catalyst used may be appropriately selected from the range of 0.001 to 10 mol, and is preferably 0.01 to 1 mol, with respect to 1 mol of compound [XV].

The reaction temperature of this reaction may be an arbitrary temperature selected from the range of -78°C to the reflux temperature in the reaction system. The reaction is preferably carried out at a temperature in the range of -10°C to 100°C.

Although the reaction time of this reaction varies depending on the reaction temperature, reaction substrate, reaction amount, etc., it is usually 10 minutes to 24 hours.

After the completion of reaction, compound [XVI] can be isolated by procedures such as adding the reaction mixture to water and filtering out the precipitated solid, or extracting the reaction mixture with an organic solvent and then concentrating. The isolated compound [XVI] can be further purified by column chromatography, recrystallization, distillation, or the like, if necessary.

Manufacturing method 7

Among the compounds of the present invention, the compounds represented by the general formula [XIX] can be produced, for example, according to the following method.

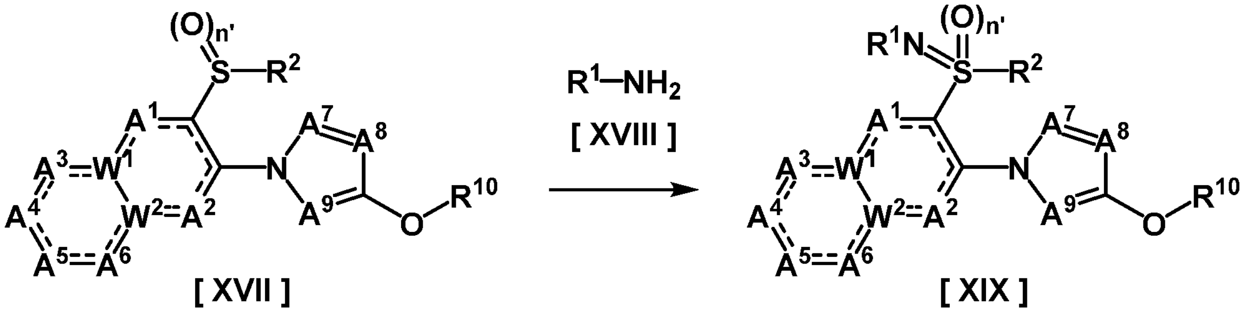


[ XVII ] [ XIX ]

(wherein, n' represents 0 or 1, and $W^1$, $W^2$, $R^1$, $R^2$, $A^1$, $A^2$, $A^3$, $A^4$, $A^5$, $A^6$, $A^7$, $A^8$, $A^9$, and $R^{10}$ have the same meanings as described above.)

That is, the compounds represented by the general formula [XIX] can be produced by reacting compound [XVIII] or a salt thereof, compound [XVII], and a suitable oxidizing agent in a suitable solvent in the presence or absence of a suitable base. Furthermore, this reaction can also be carried out in the presence of a suitable catalyst.

The amount of compound [XVIII] used in this reaction can be appropriately selected from the range of usually 1 to 100 mol, and is preferably 1 to 5 mol, with respect to 1 mol of compound [XVII].

Examples of the oxidizing agent that can be used in this reaction include halogenating agents such as chlorine, bromine, iodine, chloramine, N-chlorosuccinimide (NCS), N-bromosuccinimide (NBS), N-iodosuccinimide (NIS), 1,3-dichloro-5,5-dimethylhydantoin (DCH), 1,3-dibromo-5,5-dimethylhydantoin (DBH), 1-bromo-3-chloro-5,5-dimethylhydantoin (BCDMH); andhypervalent iodine compounds such as (dichloroiodo)benzene, (diacetoxyiodo)benzene, (bis(trifluoroacetoxy)iodo)benzene, iodosylbenzene, iodoxybenzene, 2-iodoxybenzoic acid (IBX), Dess-Martin periodinane (DMP), 2-iodoxybenzenesulfonic acid (IBS). The amount of the oxidizing agent to be used may be appropriately selected from the range of 0.5 to 5 mol, and is preferably 0.9 to 3 mol, with respect to 1 mol of compound [XVII].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate, magnesium oxide, aluminum oxide; metal hydrides such as lithium hydride, sodium hydride, potassium hydride; metal salts of alcohols such as sodium methoxide, sodium ethoxide, potassium tert-butoxide; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used may be appropriately selected from the range of 0 to 20 mol with respect to 1 mol of the compound [XVII], and is preferably 0 to 10 mol.

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [XVII].

Examples of the catalyst that can be used in this reaction include rhodium acetate (II), rhodium trifluoroacetate (II), rhodium triphenylacetate (II), rhodium pivalate (II) , rhodium octanoate (II), bis[rhodium($\alpha$, $\alpha$, $\alpha'$, $\alpha'$-tetramethyl-1,3-benzenedipropionic acid)], rhodium (II) acetimidate, and the like. The amount of the catalyst used may be appropriately selected from the range of 0.001 to 10 mol, and is preferably 0.005 to 1 mol, with respect to 1 mol of compound [XVII].

The reaction temperature of this reaction may be an arbitrary temperature selected from the range of -78°C to the reflux temperature in the reaction system. The reaction is preferably carried out at a temperature in the range of 0°C to 100°C.

Although the reaction time of this reaction varies depending on the reaction temperature, reaction substrate, reaction amount, etc., it is usually 10 minutes to 48 hours.

After the completion of reaction, compound [XIX] can be isolated by procedures such as adding the reaction mixture to water and filtering out the precipitated solid, or extracting the reaction mixture with an organic solvent and then concentrating. The isolated compound [XIX] can be further purified by column chromatography, recrystallization, distillation, or the like, if necessary.

Manufacturing method 8

Among the compounds of the present invention, the compounds represented by the general formula [XXI] can be produced, for example, according to the following method.

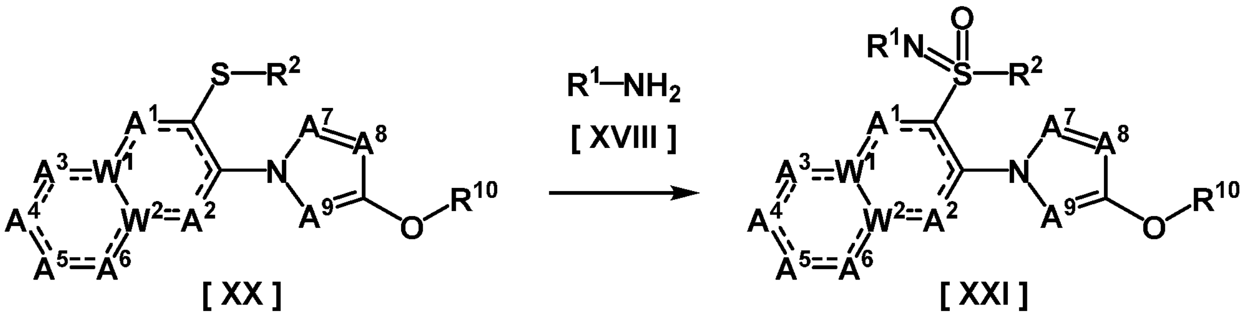


(wherein, $W^1$, $W^2$, $R^1$, $R^2$, $A^1$, $A^2$, $A^3$, $A^4$, $A^5$, $A^6$, $A^7$, $A^8$, $A^9$, and $R^{10}$ have the same meanings as described above.)

That is, the compounds represented by the general formula [XXI] can be produced by reacting compound [XVIII] or a salt thereof, compound [XX], and a suitable oxidizing agent in a suitable solvent in the presence or absence of a suitable base. Furthermore, this reaction can also be carried out in the presence of a suitable catalyst.

The amount of compound [XVIII] used in this reaction can be appropriately selected from the range of usually 1 to 100 mol, and is preferably 1 to 5 mol, with respect to 1 mol of compound [XX].

Examples of the oxidizing agent that can be used in this reaction include halogenating agents such as chlorine, bromine, iodine, chloramine, N-chlorosuccinimide (NCS), N-bromosuccinimide (NBS), N-iodosuccinimide (NIS), 1,3-dichloro-5,5-dimethylhydantoin (DCH), 1,3-dibromo-5,5-dimethylhydantoin (DBH), 1-bromo-3-chloro-5,5-dimethylhydantoin (BCDMH); andhypervalent iodine compounds such as (dichloroiodo)benzene, (diacetoxyiodo)benzene, (bis(trifluoroacetoxy)iodo)benzene, iodosylbenzene, iodoxybenzene, 2-iodoxybenzoic acid (IBX), Dess-Martin periodinane (DMP), 2-iodoxybenzenesulfonic acid (IBS). The amount of the oxidizing agent to be used may be appropriately selected from the range of 0.5 to 5 mol, and is preferably 0.9 to 3 mol, with respect to 1 mol of compound [XX].

When a base is used in this reaction, examples of the base that can be used include hydroxides of alkali metals such as lithium hydroxide, sodium hydroxide and potassium hydroxide; hydroxides of alkaline earth metals such as calcium hydroxide and magnesium hydroxide; alkali metal carbonates such as lithium carbonate, sodium carbonate, potassium carbonate, and cesium carbonate; alkali metal bicarbonates such as sodium hydrogen carbonate and potassium hydrogen carbonate; fluoride of alkali metals such as sodium fluoride and potassium fluoride; inorganic bases such as tripotassium phosphate, magnesium oxide, aluminum oxide; metal hydrides such as lithium hydride, sodium hydride, potassium hydride; metal salts of alcohols such as sodium methoxide, sodium ethoxide, potassium tert-butoxide; or organic bases such as triethylamine, tributylamine, N,N-diisopropylethylamine, N,N-dimethylaniline, pyridine, 2,6-lutidine, 4-N,N-dimethylaminopyridine, 1,8-diazabicyclo[5.4.0]-7-undecene. The amount of the base used can be appropriately selected from the range of 0 to 20 mol, and is preferably 0 to 10 mol, with respect to 1 mol of compound [XX].

Examples of the solvent that can be used in this reaction include ethers such as diethyl ether, methyl tert-butyl ether, cyclopentyl methyl ether, tetrahydrofuran, 2-methyltetrahydrofuran, 1,4-dioxane, monoglym; aromatic hydrocarbons such as benzene, toluene, xylene, mesitylene, chlorobenzene; halogenated hydrocarbons such as dichloromethane, chloroform, 1,2-dichloroethane; aprotic polar solvents such as N,N-dimethylformamide, N,N-dimethylacetamide, N-methyl-2-pyrrolidone, dimethyl sulfoxide, sulfolane, 1,3-dimethyl-2-imidazolidinone; alcohols such as methanol, ethanol, 2-propanol, tert-butyl alcohol, methyl cellosolve; nitriles such as acetonitrile and propionitrile; aliphatic hydrocarbons such as pentane, hexane, cyclohexane and heptane; pyridines such as pyridine, picoline and lutidine; tertiary amines such as triethylamine and tributylamine; water, or a mixed solvent thereof and the like. The amount of the solvent used is 0.1 to 1000 liters, preferably 0.2 to 100 liters, with respect to 1 mol of compound [XX].

Examples of the catalyst that can be used in this reaction include rhodium acetate (II), rhodium trifluoroacetate (II), rhodium triphenylacetate (II), rhodium pivalate (II) , rhodium octanoate (II), bis[rhodium($\alpha$, $\alpha$, $\alpha'$, $\alpha'$-tetramethyl-1,3-benzenedipropionic acid)], rhodium (II) acetimidate, and the like. The amount of the catalyst used may be appropriately selected from the range of 0.001 to 10 mol, and is preferably 0.01 to 5 mol, with respect to 1 mol of compound [XX].

The reaction temperature of this reaction may be an arbitrary temperature selected from the range of -78°C to the reflux temperature in the reaction system. The reaction is preferably carried out at a temperature in the range of 0°C to 100°C.

Although the reaction time of this reaction varies depending on the reaction temperature, reaction substrate, reaction amount, etc., it is usually 10 minutes to 48 hours.

After the completion of reaction, compound [XXI] can be isolated by procedures such as adding the reaction mixture to water and filtering out the precipitated solid, or extracting the reaction mixture with an organic solvent and then concentrating. The isolated compound [XXI] can be further purified by column chromatography, recrystallization, distillation, or the like, if necessary.

The heterocyclic compounds of the present invention represented by the general formulas [II] and [IV] are useful compounds as intermediates in producing the heterocyclic compounds represented by the general formula [I] or agriculturally acceptable salts thereof of the present invention.

The pesticide compositions of the present invention contain the heterocyclic compounds represented by the general formula [I] or agriculturally acceptable salts thereof of the present invention as an active ingredient.

The pesticide compositions of the present invention can contain additive components (carriers) usually used for pesticide formulations, if necessary.

The pest control agents of the present invention contain heterocyclic compounds represented by the general formula [I] or agriculturally acceptable salts thereof of the present invention as active ingredients. The pest control agents of the present invention are typically insecticides, acaricides, and nematicides.

The pest control agents of the present invention can contain additive components (carriers) usually used for pesticide formulations, if necessary.

Examples of the additive component include carriers such as solid or liquid carriers, surfactants, binders, tackifiers, thickeners, colorants, spreading agents, adhesive spreading agents, antifreezing agents, anticaking agents, disintegrants, and decomposition inhibitors, etc. In addition, preservatives, plant pieces, and the like may also be used as additive components, if necessary. Further, these additive components may be used alone or in combination of two or more.

Examples of solid carriers include mineral-based carriers such as pyrophyllite clay, kaolin clay, silica stone clay, talc, diatomaceous earth, zeolite, bentonite, acidic clay, activated clay, attapargas clay, vermiculite, pearlite, pumice, white carbon (synthetic silicic acid, synthetic silicate, etc.), and titanium dioxide; vegetable carriers such as wood flour, corn stalks, walnut shells, fruit nuclei, rice husk, sawdust, wheat bran, soybean flour, powdered cellulose, starch, dextrin, and sugars; inorganic salt carriers such as calcium carbonate, ammonium sulfate, sodium sulfate, and potassium chloride; polymer carriers such as polyethylene, polypropylene, polyvinyl chloride, polyvinyl acetate, ethylene-vinyl acetate copolymer, and urea-aldehyde resin.

Examples of liquid carriers include monohydric alcohols such as methanol, ethanol, propanol, 2-propanol, butanol, and cyclohexanol; polyhydric alcohols such as ethylene glycol, diethylene glycol, propylene glycol, hexylene glycol, polyethylene glycol, polypropylene glycol, and glycerin; polyhydric alcohol derivatives such as propylene glycol ether; ketones such as acetone, methyl ethyl ketone, methyl isobutyl ketone, diisobutyl ketone, cyclohexanone, and isophorone; ethers such as diethyl ether, 1,4-dioxane, cellosolve, dipropyl ether, and tetrahydrofuran; aliphatic hydrocarbons such as normal paraffin, naphthen, isoparaffin, kerosine, and mineral oil; aromatic hydrocarbons such as toluene, $C_9$-$C_{10}$ alkylbenzene, xylene, solventnaphtha, alkylnaphthalene, and aromatic hydrocarbons with high boiling point; halogenated hydrocarbons such as 1,2-dichloroethane, chloroform, and carbon tetrachloride; esters such as ethyl acetate, diisopropylphthalate, dibutylphthalate, dioctylphthalate, and dimethyl adipate; lactones such as $\gamma$-butyrolactone; amides such as N,N-dimethylformamide, N,N-diethylformamide, N,N-dimethylacetamide, and N-methyl-2-pyrrolidone; nitriles such as acetonitrile; sulfur compounds such as dimethylsulfoxide; vegetable oils such as soybean oil, rapeseed oil, cottonseed oil, palm oil, and castor oil; and lower alkyl esters of fatty acids derived from the vegetable oils; water; and the like.

Examples of surfactants include nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl phenyl ether, polyoxyethylene dialkyl phenyl ether, polyoxyethylene alkyl phenyl ether formalin condensate, polyoxyethylene polyoxypropylene block polymer, alkyl polyoxyethylene polypropylene block polymer ether, polyoxyethylene alkylamine, polyoxyethylene fatty acid amide, polyoxyethylene fatty acid bisphenyl ether, polyalkylene benzyl phenyl ether, polyoxyalkylene styryl phenyl ether, acetylene diol, polyoxyalkylene-added acetylene diol, polyoxyethylene ether type silicone, ester type silicone, fluorosurfactant, polyoxyethylene castor oil, and polyoxyethylene hydrogenated castor oil; anionic surfactants such as alkyl sulfate, polyoxyethylene alkyl ether sulfate, polyoxyethylene alkylphenyl ether sulfate, polyoxyethylene styrylphenyl ether sulfate, alkylbenzene sulfonate, lignin sulfonate, alkyl sulfosuccinate, naphthalene sulfonate, alkylnaphthalene sulfonate, salt of formalin condensate of naphthalene sulfonic acid, salt of formalin condensate of alkylnaphthalene sulfonic acid, fatty acid salt, polycarboxylate, N-methyl-fatty acid sarcosinate, resin acid salt, polyoxyethylene alkyl ether phosphate, and polyoxyethylene alkyl phenyl ether phosphate; cationic surfactants such as laurylamine hydrochlorides, stearylamine hydrochlorides, oleylamine hydrochlorides, stearylamine acetates, stearylaminopropylamine acetates, alkyltrimethylammonium chlorides, and alkylamine salts such as alkyldimethylbenzalconium chlorides; amphoteric surfactants such as those of betaine type like dialkyldiaminoethyl betaine and alkyldimethylbenzyl betaine, and those of amino acid type like dialkylaminoethylglycine and alkyldimethylbenzylglycine, and the like.

Examples of binders and tackifiers include carboxymethyl cellulose and its salts, dextrin, water-soluble starch, xanthan gum, guar gum, sucrose, polyvinylpyrrolidone, gum arabic, polyvinyl alcohol, polyvinyl acetate, sodium polyacrylate, polyethylene glycol, polyethylene oxide, natural phospholipids (for example, cepharic acid, lecithin, etc.), and the like.

Examples of thickeners include water-soluble polymer such as xanthan gum, guar gum, carboxymethyl cellulose, polyvinylpyrrolidone, carboxyvinyl polymer, acrylic polymer, starch derivative, and polysaccharide; inorganic fine powder such as highly pure bentonite and white carbon; organic fine powder such as organic bentonite, and the like.

Examples of colorants include inorganic pigments such as iron oxide, titanium oxide, and prussian blue; organic dyes such as alizarin dyes, azo dyes, and metallic phthalocyanine dyes.

Examples of spreading agents include silicone-based surfactants, cellulose powder, dextrins, modified starche, polyaminocarboxylic acid chelate compounds, crosslinked polyvinylpyrrolidone, maleic acid / styrene copolymer, methacrylic acid copolymer, half ester of polyhydric alcohol polymer and dicarboxylic acid anhydride, water-soluble salt of polystyrene sulfonic acid, polyoxyethylene alkanediols, polyoxyethylene alkyne diols, alkyne diols, and the like.

Examples of adhensive spreading agents include various surfactants such as sodium dialkyl sulfosuccinate, polyoxyethylene alkyl ether, polyoxyethylene alkyl phenyl ether, polyoxyethylene fatty acid ester; paraffin, terpene, polyamide resin, polyacrylic acid salt, polyoxyethylene, wax, polyvinyl alkyl ether, alkylphenol formalin condensate, synthetic resin emulsion, and the like.

Examples of antifreezing agents include polyhydric alcohols such as ethylene glycol, diethylene glycol, propylene glycol, and glycerin.

Examples of anticaking agents include polysaccharides such as starch, alginic acid, mannose, and galactose; polyvinylpyrrolidone, white carbon, ester gum, petroleum resin, and the like.

Examples of disintegrants include sodium tripolyphosphate, sodium hexametaphosphate, metal stearic acid salt, cellulose powder, dextrin, copolymer of methacrylic acid ester, polyvinylpyrrolidone, polyaminocarboxylic acid chelate compound, sulfonated styrene / isobutylene / maleic anhydride copolymer, starch / polyacrylonitrile graft copolymer, and the like.

Examples of decomposition inhibitors include desiccants such as zeolite, quicklime, and magnesium oxide; antioxidants such as phenol-based, amine-based, sulfur-based, and phosphoric acid-based ones; UV absorbers such as salicylic acid-based and benzophenone-based ones.

Examples of preservatives include potassium sorbate, 1,2-benzothiazole-3 (2H) -one, and the like.

Examples of plant pieces include sawdust, palms, corn cobs, tobacco stems, and the like.

On the other hand, when the above-mentioned additive components are contained in the pest control agents of the present invention, their content ratio on a mass basis is usually selected from the range of 5 to 95%, preferably 20 to 90% for carriers such as solid carriers or liquid carriers, the range of 0.1% to 30%, preferably 0.5 to 10% for surfactants, and the range of 0.1 to 30%, preferably 0.5 to 10% for other additives.

For use, the pest control agents of the present invention are formulated into any dosage form of, for example, powder, powder granule, granule, wettable powder, water soluble, water dispersible granule, tablets, jumbo, emulsifiable concentrate, oil, liquid, suspension concentrate, emulsion, micro emulsion, suspo emulsion, micro-spray solution, microcapsule, smoke, aerosol, bait, paste, and the like.

In the actual use, these formulations can be used as they are, or diluted before use to a predetermined concentration with a diluent such as water. Application of various formulations containing the compounds of the present invention or dilutions thereof can be carried out by commonly used application methods, such as spraying (for example, spraying, misting, atomizing, spreading powder, spreading granule, applying on water surface, applying in box, etc.), soil application (for example, mixing, irrigating, etc.), surface application (for example, swabbing, powder coating, covering, etc.), seed treatment (for example, smearing, powder coating treatment, etc.), soaking, poison bait, smoke application, and the like. Besides, the active ingredients can also be mixed with forage to feed the livestock to control the outbreak and growth of harmful insects, particularly harmful insects, in the excrement.

The method for controlling pests of the present invention can be carried out by using an effective amount of the active ingredient of the heterocyclic compounds represented by the general formula [I] or agriculturally acceptable salts thereof of the present invention in the above-mentioned application methods.

The compounding ratio (mass%) of the active ingredients in the pest control agents of the present invention is appropriately selected as necessary, and is appropriately selected, for example, from the range of 0.01 to 20%, preferably 0.05 to 10% in case of formulating into powder, powder granule, or microgranule, etc., from the range of 0.1 to 30%, preferably 0.5 to 20% in case of formulating into granule, etc., from the range of 1 to 70%, preferably 5 to 50% in case of formulating into wettable powder, water dispersible granule, etc., from the range of 1 to 95%, preferably 10 to 80% in case of formulating into water soluble agents, liquid, etc., from the range of 5 to 90%, preferably 10 to 80% in case of formulating into emulsifiable concentrate, etc., from the range of 1 to 50%, preferably 5 to 30% in case of formulating into oil, etc., from the range of 5 to 60%, preferably 10 to 50% in case of formulating into suspension concentrate, etc., from the range of 5 to 70%, preferably 10 to 60% in case of formulating into emulsion, micro emulsion, suspo emulsion,

etc., from the range of 1 to 80%, preferably 5 to 50% in case of formulating into tablets, bait, paste, etc., from the range of 0.1 to 50%, preferably 1 to 30% in case of formulating into smoke, etc., and from the range of 0.05 to 20%, preferably 0.1 to 10% in case of formulating into aerosol, etc.

These formulations are diluted to an appropriate concentration and sprayed, or applied directly.

The pest control agents of the present invention are generally applied at active ingredient concentration of 0.1 to 5000 ppm when diluted with a diluent. When a formulation is used as it is, the amount of application per unit area is 0.1 to 5000 g of the active ingredient compound per ha. However, it is not limited thereto.

Needless to say, the pest control agents of the present invention are sufficiently effective even when the compounds of the present invention are used alone as active ingredients. However, if necessary, it can be mixed or used with other fertilizers and pesticides, such as insecticides, acaricides, nematicides, synergists, fungicides, antivirals, attractants, herbicides, plant growth regulators, and the like, wherein a better effect may be exhibited.

Next, known insecticides (insecticidal active ingredients), acaricides (acaricidal active ingredients), nematicides (nematicidal active ingredients), and synergistic compounds (synergistic active ingredients) that may be mixed or used in combination are exemplified below.

Insecticidal active ingredients, acaricidal active ingredients, nematicidal active ingredients, and synergistic active ingredients:

Acrinathrin, azadirachtin, azamethiphos, acynonapyr, azinphos-ethyl, azinphos-methyl, acequinocyl, acetamiprid, acetoprole, acephate, azocyclotin, abamectin, afidopyropen, afoxolaner, amidoflumet, amitraz , alanycarb, aldicarb, aldoxycarb, allethrin, (including d-cis-trans-form and d-trans-form), isazophos, isamidofos, isocarbophos, isoxathion, isocycloseram, isofenphos-methyl, isoprocarb, epsilon-metofluthrin, epsilon-momfluorothrin, ivermectin, imicyafos, imidacloprid, imiprothrin, indazapyroxamet, indoxacarb, esfenvalerate, ethiofencarb, ethion, ethiprole, ethylene dibromide, etoxazole, etofenprox, ethoprophos, etrimfos, emamectin, emamectin benzoate, endosulfan, empenthrin, oxazosulfyl, oxamyl, oxydemeton-methyl, oxydeprofos, omethoate, Nuclear polyhedrosis virus, cadusafos, kappa-tefluthrin, kappa-bifenthrin, karanjin, cartap, Granulosis virus, carbaryl, carbosulfan, carbofuran, gamma-BHC, xylylcarb, quinalphos, kinoprene, chinomethionat, Entero virus, coumaphos, cryolite, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, chloroprallethrin, Entomopoxi virus, Irido virus, cyazypyr, cyanophos, diafenthiuron, diamidafos, cyantraniliprole, cyetpyrafen, dienochlor, cyenopyrafen, dioxabenzofos, diofenolan, Sigma virus, cyclaniliprole, cycloxaprid, dicrotophos, dichlofenthion, cyclobutrifluram, cycloprothrin, dichlorvos, dicloromezotiaz, dicofol, dicyclanil, disulfoton, dinotefuran, dinobuton, cyhalodiamide, cyhalothrin (including gamma-form and lambda-form), cyphenothrin, (including (1R)-trans-form), cyfluthrin (including beta-form), diflubenzuron, cyflumetofen, diflovidazin, cyproflanilide, cyhexatin, cypermethrin (including alpha-form, beta-form, theta-form, and zeta-form), dimpropyridaz, dimethyl-2,2,2-trichloro-1-hydroxyethylphosphonate (DEP), dimethylvinphos, dimethoate, dimefluthrin, jasmone, cis-jasmone, jasmonic acid, methyl jasmonate, silafluofen, cyromazine, Steinemema carpocapsae, Steinernema kushidai, Steinernema glaseri, spidoxamat, spinetoram, spinosad, spirodiclofen, spirotetramat, spiropidion, spiromesifen, sulcofuron-sodium, sulfluramid, sulfoxaflor, sulfotep, diazinon, thiacloprid, thiamethoxam, tioxazafen, thiodicarb, thiocyclam, thiosultap, thionazin, thiofanox, thiometon, tyclopyrazoflor, tetrachlorantraniliprole, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos, trichlorfon, Trichoderma asperellum, Trichoderma paecilomyces, Trichoderma harzianum, triflumuron, trifluenfuronate, triflumezopyrim, trimethacarb, tolfenpyrad, naled, nicotine, nicofluprole, nitenpyram, nemadetin, Denso virus, novaluron, noviflumuron, paecilomyces lilacinus, Barkholderia cepacia, Barkholderia rinojensis, Verticillium lecanii, hydroprene, Pasteuria nishizawae, Pasteuria penetrans, Bacillus amyloliquefaciens, Bacillus firmus, Bacillus sphaericus, Bacillus subtillis, Bacillus thuringiensis, insect toxin producing Bacillus thuringiensis, Bacillus thuringiensis subsp. Aizawai, Bacillus thuringiensis subsp. Israelensis, Bacillus thuringiensis subsp. Kurstaki, Bacillus thuringiensis subsp. Tenebrionis, Bacillus popilliae, Bacillus licheniformis, vamidothion, parathion, parathion-methyl, halfenprox, halofenozide, bioallethrin, bioallethrin S-cyclopentenyl, bioresmethrin, bis- (2-chloro-1-methylethyl ether (DCIP), bistrifluron, hydramethylnon, bifenazate, bifenthrin, pyflubumide, piperonyl butoxide, pymetrozine, pyraclofos, pyrafluprole, pyridaphenthion, pyridaben, pyridalyl, pyrifluquinazon, pyriprole, pyriproxyfen, pirimicarb, pyrimidifen, pyriminostrobin, pirimiphos-methyl, pyrethrine, famphur, fipronil, fenazaquin, fenamiphos, fenitrothion, fenoxycarb, fenothiocarb, phenothrin (including (1R)-trans-form), fenobucarb, fenthion, fenthoate, fenvalerate, fenpyroximate, fenbutatin oxide, fenpropathrin, fonofos, sulfuryl fluoride, butocarboxim, butoxycarboxim, prallethrin, fluacrypyrim, fluazaindolizine, fluazuron, fluensulfone, fluopyram, sodium fluoroacetate, fluxametamide, flucycloxuron, flucythrinate, flusulfamide, fluthrin, fluvalinate (including tau-form), flupyradifurone, flupyrazofos, flupyrimin, flufiprole, flufenerim, flufenoxystrobin, flufenoxuron, fluhexafon, flubendiamide, flupentiofenox, flumethrin, fluralaner, flurimfen, prothiofos, protrifenbute, flonicamid, propaphos, propargite, prohydrojasmon, profenofos, broflanilide, profluthrin, propetamphos, propoxur, flometoquin, bromopropylate, hexythiazox, hexaflumuron, Pacilimyces tenuipes, Paecilomyces fumosoroceus, Paecilomyces lilacinus, heptafluthrin, heptenophos, permethrin, benclothiaz, benzpyrimoxan, bensultap, benzoximate, bendiocarb, benfuracarb, Pochonia chlamydosporia, Beauveria tenella, Beauveria

bassiana, Beauveria brongniartii, phoxim, phosalone, fosthiazate, fosthietan, phosphamidon, phosmet, polynactins, formetanate, phorate, machine oil, malathion, milbemectin, mecarbam, mesulfenfos, methomyl, metaldehyde, metaflumizone, methamidophos, metham, methiocarb, methidathion, methyl isothiocyanate, methyl bromide, methoxychlor, methoxyfenozide, methothrin, metofluthrin, methoprene, metolcarb, mevinphos, meperfluthrin, Monacrosporium phymatophagum, Monacrosporium phymatophagum, monocrotophos, momfluorothrin, Trichoderma harzianum, litlure-A, litlure-B, aluminium phosphide, zinc phosphide, phosphine, lufenuron, rescalure, resmethrin, lepimectin, rotenone, Cytoplasmic polyhedrosis virus, fenbutatin oxide, calcium cyanide, organotins, nicotine-sulfate, (Z)-11-tetradecenyl acetate, (Z)-11-hexadecenal, (Z)-11-hexadecenyl acetate, (Z)-9,12-tetradecadienyl acetate, (Z)-9-tetradecen-1-ol, (Z, E)-9,11-tetradecadienyl acetate, (Z, E)-9,12-tetradecadienyl acetylate, 1,1,1-trichloro-2,2-bis (4-chlorophenyl ethane (DDT), 1,3-dichloropropene, 2,4-dichloro-5-{2-[4-(trifluoromethyl) phenyl]ethoxy}phenyl 2,2,2-trifluoroethyl sulfoxide (chemical name, CAS registry number: 1472052-11-1), 2,4-dimethyl-5-[6-(trifluoromethylthio)hexyloxy]phenyl-2,2,2-trifluoroethyl sulfoxide (chemical name, CAS registry number: 1472050-34-2), 2-{2-fluoro-4-methyl-5-[(2,2,2-trifluoroethyl)sulfinyl] phenoxy}-5-(trifluoromethyl)pyridine (chemical name, CAS registry number: 1448758-62-0), 3-chloro-2-{2-fluoro-4-methyl-5-[(2,2,2-trifluoroethyl) sulfinyl] phenoxy}-5-(trifluoromethyl)pyridine (chemical name, CAS registry number: 1448761-28-1), 4,6-dinitro-o-cresol (DNOC), 4-fluoro-2-methyl-5-(5,5-dimethylhexyloxy)phenyl 2,2,2-trifluoroethyl sulfoxide (chemical name, CAS registry number: 1472047-71-4), Bt protein (CrylAb, CrylAc, CrylFa, Cry2Ab, mCry3A, Cry3Ab, Cry3Bb, and Cry34 / 35Abl), methyl eugenol, 4-(p-acetooxyphenyl)-2-butanone, (Z)-10-tetradecenyl acetate, (E, Z)-4,10-tetradecadiniel acetate, (Z)-8-dodecenyl acetate, (Z)-11-tetradecenyl acetate, (Z)-13-icosen-10-on, 14-methyl-1-octadesen, AKD-1193 (code number), BCS-AA10147 (code number), CL900167 (code number), NA-85 (code number), NI-30 (code number), O,O-diethyl-O-[4-(dimethylsulfamoyl) phenyl]-phosphorothionate (DSP), O-ethyl-O-4-(nitrophenyl)phenylphosphonothioate (EPN), RU15525 (code number), XMC (XMC), Z-13-icosen-10-on, ZXI8901 (code number), and F4260 (code number).

Next, known compounds of herbicides, herbicidal active ingredients, or compounds of plant growth regulators which may be mixed or used in combination are exemplified below. However, they are not limited to thereto.

Herbicide compound or herbicidal active ingredient:

Ioxynil (including lithium salt, sodium salt, and salts with octanoic acid, etc.), aclonifen, acrolein, azafenidin, acifluorfen (including sodium salt, etc.), azimsulfuron, asulam, acetochlor, atrazine, anilofos, amicarbazone, amidosulfuron, amitrole, aminocyclopyrachlor, aminopyralid, amiprofos-methyl, ametryn, Araujia Mosaic Virus, alachlor, Alternaria destruens, alloxydim (including sodium salt, etc.), ancymidol, isouron, isoxachlortole, isoxaflutole, isoxaben, Isodecylalkoholethoxylate, isoproturon, ipfencarbazone, imazaquin, imazapic (including salt with amine, etc.), imazapyr (including salts such as isopropylamine), imazamethabenz, imazamethabenz-methyl, imazamox, imazethapyr, imazosulfuron, indaziflam, indanofan, eglinazine-ethyl, esprocarb, ethametsulfuron-methyl, ethalfluralin, ethidimuron, ethoxysulfuron, ethoxyfen, ethoxyfen-ethyl, ethofumesate, etobenzanid, epyrifenacil, endothal-disodium, oxadiazon, oxadiargyl, oxaziclomefone, oxasulfuron, oxyfluorfen, oryzalin, Obuda Pepper Virus, orthosulfamuron, orbencarb, oleic acid, cafenstrole, caprylic acid, capric acid, carfentrazone-ethyl, karbutilate, carbetamide, quizalofop, quizalofop-ethyl, quizalofop-P-ethyl, quizalofop-P-tefuryl, Xanthomonas campestris, quinoclamine, quinclorac, quinmerac, citric acid, cumyluron, clacyfos, glyphosate (including sodium, potassium, amine, propylamine, isopropylamine, ammonium, isopropylammonium, guanidine, monoethanolamine, choline, BAPMA (N,N-bis- (aminopropyl) methylamine, dimethylamine, or trimesium salts, etc.), glufosinate (including amine or sodium salts, etc.), glufosinate-P, glufosinate-P-sodium, clethodim, clodinafop, clodinafop-propargyl, clopyralid (including monoethanolamine salt), clomazone, chlomethoxyfen, clomeprop, cloransulam-methyl, chloramben, chloridazon, chlorimuron, chlorimuron-ethyl, chlorsulfuron, chlorthal-dimethyl, chlorthiamid, chlorphthalim, chlorflurenol-methyl, chlorpropham, chlorbromuron, chloroxuron, chlorotoluron, ketospiradox (including sodium, calcium, or ammonium salts, etc.), Colletotrichum orbiculare, Colletotrichum gloeosporioides, Colletotrichum truncatum, Chondrostercum purpureum, saflufenacil, sarmentine, cyanazine, cyanamide, diuron, diethatyl-ethyl, dioxopyritrione, dicamba (including salts with amine, diethylamine, isopropylamine, diglycolamine, dimethylammonium, diolamine, isopropylammonium, auramine, potassium, trollamine, BAPMA (N,N-bis- (aminopropyl) methylamine), choline, sodium, or lithium, etc., or esters such as methyl esters), cycloate, cycloxydim, diclosulam, cyclosulfamuron, cyclopyranil, cyclopyrimorate, dichlobenil, dichlofop, diclofop-P-methyl, diclofop-methyl, dichlorprop, dichlorprop-P (including salts with dimethylammonium, potassium, sodium, and choline, etc., or esters such as butothyl ester, 2-ethylhexyl ester, isoctyl ester, and methyl ester, etc.), diquat, diquat dibromide, dithiopyr, siduron, dinitramine, cinidon-ethyl, cinosulfuron, dinoseb (including acetate), dinoterb, cyhalofop, cyhalofop-butyl, cypyrafluone, diphenamid, difenzoquat, diflufenican, diflufenzopyr, simazine, dimesulfazet, dimethachlor, dimethametryn, dimethenamid, dimethenamid-P, simetryn, dimepiperate, dimefuron, Pseudomonas fluorescens, cinmethylin, swep, sulcotrione, sulfentrazone , sulfosate, sulfosulfuron, sulfometuron-methyl, sethoxydim, Scelerothinia minor, terbacil, daimuron, thaxtomin A, Tobacco Mild Green Mosaic Tobamovirus, Tobacco Rattle Virus, dalapon, thiazopyr, tiafenacil, thiencarbazone (including sodium salt, and methyl ester, etc.), tiocarbazil, thiobencarb, thidiazimin, thidiazuron, thifensulfuron, thifensulfuron-methyl, desmedipham, desmetryne, tetflupyrolimet, thenylchlor, tebutam, tebuthiuron, tepraloxydim, tefuryltrione, terbuthylazine, terbutryn, terbumeton, tembotrione, topramezone, tralkoxydim, triaziflam, triasulfuron, triafamone, tri-allate, trietazine, triclopyr, triclopyr-butotyl, triclopyr-triethylammonium, tritosulfuron, tripyrasulfone, trifludimoxazin, triflusulfuron-methyl, trifluralin, trifloxysulfuron (including sodium salt etc.), tribenuron-methyl, tolpyralate, naptalam (including sodium salt etc.), naproanilide, napropamide, napropamide-M, nicosulfuron, lactic acid, neburon, norflurazon, Barkholderia rinojensis, vernolate, paraquat, paraquat dichloride, halauxifen, halauxifen-benzyl, halauxifen-methyl, Burkholderia rinojensis, haloxyfop, haloxyfop-P, haloxyfop-etotyl, haloxyfop-P-methyl, halosafen, halosulfuron-methyl, bixlozone, picloram (including salts with dichloroammonium, trollamine, etc.), picolinafen, bicyclopyrone, bispyribac-sodium, pinoxaden, bipyrazone, bifenox, piperophos, pyraclonil, pyrasulfotole, pyrazoxyfen, pyrazosulfuron-ethyl, pyrazolynate, bilanafos, pyraflufen, pyraflufen-ethyl, pyridafol, pyrithiobac-sodium, pyridate, pyriftalid, pyributicarb, pyribenzoxim, pyrimisulfan, pyriminobac-methyl, pyroxasulfone, pyroxsulam, Phytophthora palmivora, phenisopham, fenuron, fenoxasulfone, fenoxaprop (including methyl, ethyl, isopropyl esters), fenoxaprop-P (including methyl, ethyl, isopropyl esters), fenquinotrione,fenthiaprop-ethyl, fentrazamide, fenpyrazone,phenmedipham, Phoma chenopodicola, Phoma herbarum, Phoma macrostoma, butachlor, butafenacil, butamifos, butylate, Puccinia canaliculata, Puccinia thlaspeos, butenachlor, butralin, butroxydim, flazasulfuron, flamprop (including methyl, ethyl, isopropyl esters), flamprop-M (including methyl, ethyl, isopropyl esters), primisulfuron, primisulfuron-methyl, fluazifop-butyl, fluazifop-P, fluazifop-P-butyl, fluazolate, fluometuron, fluoroglycofen-ethyl, flucarbazone-sodium, fluchloralin, flucetosulfuron, fluthiacet-methyl, flupyrsulfuron-methyl (including sodium, calcium, or ammonium salts, etc.), flufenacet, flufenpyr-ethyl, flupropanate (including sodium salt), flupoxame, flumioxazin, flumiclorac-pentyl, flumetsulam, fluridone, flurtamone, fluroxypyr (including butomethyl and meptyl esters, or sodium, calcium and ammonium salts, etc.), flurochloridone, pretilachlor, procarbazone (including sodium salt etc.), prodiamine, prosulfuron, prosulfocarb, propaquizafop, propachlor, propazine, propanil, propyzamide, propisochlor, propyrisulfuron, propham, profluazol, prohexadione-calcium, propoxycarbazone, propoxycarbazone-sodium, profoxydim, bromacil, brompyrazon, prometryn, prometon, bromoxynil (including esters of butyric acid, octanic acid, or heptanoic acid, etc.), bromofenoxim, bromobutide, florasulam, florpyrauxifen, florpyrauxifen-benzyl, hexazinone, pethoxamid, benazolin, benazolin-ethyl, penoxsulam, Pepino Mosaic Virus, heptamaloxyloglucan, beflubutamid, beflubutamid-M, pebulate, pelargonic acid, bencarbazone, benquitrione, benzfendizone, bensulide, bensulfuron, bensulfuron-methyl, benzobicyclon, benzofenap, bentazone, pentanochlor, pendimethalin, pentoxazone, benfluralin, benfuresate, fosamine, fomesafen, foramsulfuron, forchlorfenuron, mecoprop (including sodium, potassium, isopropylamine, triethanolamine, dimethylamine, diolamine, trollamine, and choline salts, etc., or ethadyl ester, 2-ethylhexyl ester, isoctyl ester, and methyl ester, etc.), mecoprop-P-potassium, mesosulfuron (including methyl ester, etc.), mesotrione, metazachlor, metazosulfuron, methabenzthiazuron, metamitron, metamifop, metam (including sodium salt, etc.), disodium methanearsonate (DSMA), methizolin, methyldymuron, metoxuron, metosulam, metsulfuron-methyl, metobromuron, metobenzuron, metolachlor, metribuzin, mepiquat chloride, mefenacet, monosulfuron (including methyl, ethyl, and isopropyl ester), monolinuron, molinate, iodosulfuron, iodosulfuron-methyl-sodium, iofensulfuron, iofensulfuron-sodium, lactofen, lancotrione, linuron, rimisoxafen, rimsulfuron, lenacil, 2,2,2-trichloroacetic acid (TCA) (including sodium, calcium, or ammonium salts, etc.), 2,3,6-trichlorobenzoic acid (2,3,6-TBA), 2,4,5-trichlorophenoxyacetic acid (2,4,5-T), 2,4-dichlorophenoxyacetic acid (2,4-D), (including salts of amines, diethylamine, triethanolamine, isopropylamine, dimethylammonium, diolamine, dodecylammonium, heptylammonium, tetradecylammonium, triethylammonium, tris (2-hydroxypropyl) ammonium, trollamine, choline, sodium or lithium, or esters such as buttotyl ester, 2-butoxypropyl ester, 2-ethylhexyl ester, methyl ester, ethyl ester, butyl ester, isobutyl ester, octyl ester, pentyl ester, propyl ester, isoctyl ester, isopropyl ester, meptyl ester, and tefryl ester, etc.), 2,4-dichlorophenoxybutyric acid (2,4-DB) (including salts of amines, diethylamines, triethanolamines, isopropylamine, dimethylammonium, choline, sodium or lithium, or esters such as isoctyl ester), 2-amino-3-chloro-1,4-naphthoquinone (ACN), 2-methyl-4-chlorophenoxyacetic acid (MCPA) (including salts of sodium, dimethylammonium, choline, etc., or esters such as 2-ethylhexyl ester, isoctyl ester, ethyl ester, etc.), 2-methyl-4-chlorophenoxybutyric acid (MCPB) (including sodium salt, and ethyl ester, etc.), 4- (2,4-dichlorophenoxybutyric acid (2,4-DB), 4,6-dinitro-O-cresol (DNOC) (including amine or sodium salt, etc.), (SS)-3-(3,5-difluorophenyl)-N- [rel- (3R, 5R) -5-(trifluoromethylsulfonylcarbamoyl) tetrahydrofuran-3-yl]-5-vinyl-4H-isoxazole-5-carboxamid (chemical name, CAS registry number: 2266183-40-6) (international publication number: 2018/228986), international publication number: 2020/114934), N4-(2,6-difluorophenyl) -6- (1-fluoro-1-methylethyl) -1,3,5-triazine-2,4-diamine (chemical name, CAS registry number: 1606999-43-2) (international publication number: 2014/064094, international publication number: 2015/162164), (5S) -3- (3,5-difluorophenyl) -N- [(3R) -5- (methylsulfonylcarbamoyl) -2,3-dihydrofuran-3-yl] -5-vinyl-4H-isoxazole-5-carboxamid (chemical name, CAS registry number: 2266190-06-9) (international publication number: 2018/228986, international publication number: 2020/114934), (SR)-3-(3,5-difluorophenyl) -5-methyl-N- [rel- (3R, 5R) -5-(methylsulfonylcarbamoyl) tetrahydrofuran-3-yl] -4H-isoxazole-5-carboxamid (chemical name, CAS registry number: 2266164-36-5) (international publication number: 2018/228986, international publication number: 2020/114934), (5R) -3-(3,5-difluorophenyl) -N-[(3R) -5-(methoxycarbamoyl) -2,3-dihydrofuran-3-yl] -5-methyl-4H-isoxazole-5-carboxamid (chemical name, CAS registry number: 2266170-31-2) (international publication number: 2018/228986, international publication number: 2020/114934), 2- [2- (3,4-dimethoxyphenyl) -6-methyl-3-oxo-pyridazine-4-carbonyl] cyclohexane-1,3-dione (chemical

name, CAS registry number: 2138855-12-4) (international publication number: 2017/178582, international publication number: 2018/015476), 4-hydroxy-1-methyl-3- [4- (trifluoromethyl) -2-pyridyl] imidazolidin-2-one (chemical name, CAS registry number: 1708087-22-2) (international publication number: 2015/059262, international publication number: 2018/015476), 6-(1-fluorocyclopendyl) -N4-(2,3,5,6-tetrafluolophenyl) -1,3,5-triazine-2,4-diamine (chemical name, CAS registry number: 182807-75-7) (international publication number: 2015/162164), 6- (1-fluoro-1-methylethyl) -N4-(2,3,5,6-tetrafluoropheneyl) -1,3,5-triazine-2,4-diamine (chemical name, CAS registry number: 1606999-21-6) (international publication number: 2014/064094, international publication number: 2015/162164), (5S)-3-(3-fluoro-5-methylphenyl)-N-[rel-(3R, 5R) -5-(methoxycarbamoyl) tetrahydrofuran-3-yl] -5-vinyl-4H-isoxazole-5 -carboxamid (chemical name, CAS registry number: 2266292-43-5) (international publication number: 2018/228986, international publication number: 2020/114934), 6- (1-methylcyclobutyl) -N4- (2,3,5,6-tetrafluoropheneyl) -1,3,5-triazine-4,4-diamine (chemical name, CAS registry number: 1607001-97-7) (international publication number: 2014/064094, international publication number: 2015/162164), AE-F-150944 (code number), F9960 (code number), IR-6396 (code number), MCPA-thioethyl, NC-656 (code number), SYP-298 (code number), SYP-300 (code number), S-ethyl dipropylthiocarbamate (EPTC), S-metolachlor, S-9750 (code number), MSMA, and HW-02 (code number).

Plant growth regulators:

1-naphthylcatemide, 1-methylcyclopropene, 1,3-diphenylurea, 2,3,5-triiodobenzoic acid, 2-methyl-4-chlorophenoxybutyric acid (MCPB) (including sodium salt and ethyl ester, etc.), 2-(naphthalen-1-yl) acetamide, 2,6-diisopropylnaphthalene), 3-[(6-chloro-4-phenylquinazolin-2-yl) amino] propan-1-ol, 4-oxo-4-(2-phenylethyl) aminobutyric acid (chemical name, CAS registry number: 1083-55-2), 4-chlorophenoxyacetic acid (4-CPA), 5-aminolevulinic acid hydrochloride, methyl 5-(trifluoromethyl) benzo [b] thiofen-2-carboxylate, AVG (aminoethoxyvinylglycine, n-decanol, anisiflupurin, aviglycine, ancymidol, abscisic acid, isoprothiolane, inabenfide, indole acetic acid, indole butyric acid, uniconazole, uniconazole-P, Ecolyst, ethychlozate, ethephon, epocholeone, calcium chloride, choline chloride, oxine-sulfate, opabactin, kinetin, calcium peroxide, carvone, quinabactin, calcium formate, cloxyfonac, cloxyfonac-potassium, cloprop, chlormequat, chlormequat-chloride, chlorpropham, choline, cytokinins, oxydized glutathione, cyanamide, sodium cyanate, cyclanilide, dichlorprop (including dimethylammonium, potassium, sodium, and choline salts, etc., or esters such as butothyl ester, 2-ethylhexyl ester, isoctyl ester, and methyl ester, etc.), dichlorprop-P (including sodium, potassium, and dimethylammonium salts, etc., or 2-ethylhexyl ester), diquat, diquat dibromide, dikegulac, gibberellinic acid, gibberellin A4, gibberellin A7, dimethipin, sintofen, jasmone, cis-jasmone, jasmonic acid, methyl jasmonate, streptomycin, calcium polysulfide, daminozide, calcium carbonate, thidiazuron, decan-1-ol, triacontanol, triapenthenol, trinexapac-ethyl, tribufos, paclobutrazol, paraffin, bispyribac-sodium, hymexazol, butralin, fluthiacet-methyl, pyraflufen-ethyl, flumetralin, flurprimidol, flurenol, pronitridine, prohydrojasmon, prohexadione-calcium, heptamaloxyloglucan, 6-benzylaminopurine, pendimethalin, forchlorfenuron, formononetin, maleic hydrazide, mepiquat chloride, mefluidide, lipochitooligosaccharides such as lipochitooligosaccharides SP104, and calcium sulfate.

Next, known compounds for reducing chemical damage that may be mixed or used in combination are exemplified.

Compounds for reducing chemical damage:
Isoxadifen, isoxadifen-ethyl, oxabetrinil, octane-1,8-diamine, cloquintocet, cloquintcet-mexyl, dietholate, cyometrinil, dichlormid, dicyclonone, cyprosulfamide, daimuron, 1,8-Naphthalic Anhydride, fenchlorazole, fenchlorazole-O-ethyl, fenchlorim, furilazole, fluxofenim, flurazole, benoxacor, metcamifen, mephenate, mefenpyr, mefenpyr-ethyl, mefenpyr-diethyl, lower alkyl substituted benzoic acid, 2,2-dichloro-N-(1,3-dioxolan-2-ylmethyl) -N- (2-propenyl) acetamide (PPG-1292), 2-dichloromethyl-2-methyl-1,3-dioxane (MG-191), 3-dichloroacetyl-2,2,5-trimethyl-1,3-oxazolidine (R-29148), 4-dichloroacetyl-1-oxa-4-azaspiro [4.5] decane (AD-67), 4-carboxy-3,4-dihydro-2H-1-benzopyran-4-acetic acid (CL-304415, code number), MON4660 (code number), metcamifen, N1,N2-diallyl-N2-dichloroacetylglycinamide (DKA-24, code number), 1-bromo-4-[(chloromethyl) sulfonyl] benzene (CSB), 2-propenyl 1-oxa-4-azaspiro [4,5] decane-4-carbodithioate (MG-838, code number), 3-(dichloroacetyl)-2,2-dimethyl-1,3-oxazolidine (R-28725, code number), R-29148 (code number), and 1-(dichloroacetyl) azepane (TI-35, code number).

Next, known bacteriocides (bactericidal active ingredients) or compounds of disease control agents that may be mixed or used in combination are exemplified below.

Bactericidal active ingredients or compounds of disease control agents: Agrobacterim radiobacter, azaconazole, acibenzolar-S-methyl, azoxystrobin, anilazine, amisulbrom, aminopyrifen, ametoctradin, aldimorph, isotianil, isopyrazam, isofetamid, isoflucypram, isoprothiolane, ipconazole, ipflufenoquin, ipfentrifluconazole, iprodione, iprovalicarb, iprobenfos, imazalil, iminoctadine-albesilate, iminoctadine-triacetate, imibenconazole, inpyrfluxam, imprimatin A, imprimatin B, edifenphos, etaconazole, ethaboxam, ethirimol, ethoxyquin, etridiazole, enestroburin, enoxastrobin, epoxiconazole, organic oils, oxadixyl, oxazinylazole, oxathiapiprolin, oxycarboxin, oxine-copper, oxytetracycline, oxpoconazole-fumarate, oxolinic acid, copper dioctanoate, octhilinone, ofurace, orysastrobin, o-phenylphenol, kasugamycin, captafol, carpropamid, carbendazim, carboxin, carvone, Candida oleophila, Candida saitoana, quinoxyfen, quinofumelin, chinomethionat, captan, quinconazole, quintozene, guazatine, cufraneb, coumethoxystrobin, coumoxystrobin, Gliocradium catenulatum, Cryptococcus albidus, kresoxim-methyl, clozylacon, Clonostachys rosea, chlozolinate, chloroinconazide, chlorothalonil, chloroneb, Chaetomium cupreum, Coniothyrium minitans, cyazofamid, diethofencarb, diclocymet, dichlofluanid, dichlobentiazox, diclomezine, dicloran, dichlorophen, dithianon, diniconazole, diniconazole-M, zineb, dinocap, dipymetitrone, diphenylamine, difenoconazole, cyflufenamid, diflumetorim, cyproconazole, cyprodinil, simeconazole, dimethirimol, dimethyl disulfide, dimethomorph, cymoxanil, dimoxystrobin, Pseudozyma flocculosa, Pseudomonas aureofaciens, Pseudomonas chlororaphis, Pseudomonas syringae, Pseudomonas flurorescens, Pseudomonas rhodesiae, ziram, silthiofam, Zucchini yellow mosaic virus, streptomycin, Streptomyces griseoviridis, Streptomyces lygicus, spiroxamine, sedaxane, seboctylamine, zoxamide, solatenol, dazomet, Talaromyces flavus, tiadinil, thiabendazole, thiram, thiophanate, thiophanate-methyl, thifluzamide, thiram, tecnazene, tecloftalam, tetraconazole, debacarb, tebuconazole, tebufloquin, terbinafine, dodine, dodemorph, triadimenol, triadimefon, triazoxide, trichlamide, triclopyricarb, Trichoderma asperellum, Trichoderma atroviride, Trichoderma gamsii, Trichoderma stromaticum, Trichoderma paecilomyces, Trichoderma harzianum, Trichoderma viride, Trichoderma virens, Trichoderma polysporum, Trichoderma harzianum rifai, Trichoderma lignorum, tricyclazole, triticonazole, tridemorph, triflumizole, trifloxystrobin, triforine, tolylfluanid, tolclofos-methyl, tolnifanide, tolprocarb, nabam, natamycin, naftifine, nitrapyrin, nitrothal-isopropyl, nuarimol, copper nonyl phenol sulphonate, Paenibacillus polymyxa, Barkholderia cepacia, Bacillus amyloliquefaciens, Bacillus simplex, Bacillus subtilis, Bacillus pumilus, Bacillus licheniformis, harpin protein, Variovorax paradoxus, validamycin, valifenalate, Pantoea agglomerans, picarbutrazox, bixafen, picoxystrobin, Pythium oligandrum, pydiflumetofen, bitertanol, binapacryl, hinokitiol, Erwinia carotovora, Rhizobium vitis, biphenyl, piperalin, hymexazol, pyraoxystrobin, pyraclostrobin, pyraziflumid, pyrazophos, pyrapropoyne, pyrametostrobin, pyriofenone, pyrisoxazole, pyridachlometyl, pyrifenox, pyributicarb, pyribencarb, pyrimethanil, pyroquilon, vinclozolin, ferbam, famoxadone, phenazine oxide, fenamidone, fenaminstrobin, fenarimol, fenoxanil, ferimzone, fenpiclonil, fenpicoxamid, fenpyrazamine, fenbuconazole, fenfuram, fenpropidin, fenpropimorph, fenhexamid, folpet, phthalide, Fusarium oxysporum, bupirimate, fuberidazole, blasticidin-S, furametpyr, furalaxyl, furancarboxylic acid, fluazinam, fluindapyr, fluoxastrobin, fluoxapiprolin, fluoxytioconazole, fluopiclide, fluopimomide, fluopyram, fluoroimide, fluxapyroxad, fluquinconazole, furconazole, furconazole-cis, fludioxonil, flusilazole, flusulfamide, flutianil, flutolanil, flutriafol, flufenoxadiazam, flufenoxystrobin, flubeneteram, flumetylsulforim, flumetover, flumorph, Phlebiopsis gigantea, proquinazid, prochloraz, procymidone, prothiocarb, prothioconazole, bronopol, propamocarb-hydrochloride, propiconazole, propineb, probenazole, bromuconazole, flometoquin, florylpicoxamid, hexaconazole, benalaxyl, benalaxyl-M, benodanil, benomyl, pefurazoate, penconazole, pencycuron, benzovindiflupyr, benthiazole, benthiavalicarb-isopropyl, penthiopyrad, penflufen, boscalid, fosetyl (including aluminum, calcium, sodium salts, etc.), polyoxin, polycarbamate, Bordeaux mixture, mancopper, mancozeb, mandipropamide, mandestrobin, maneb, myclobutanil, mineral oils, mildiomycin, methasulfocarb, metam, metalaxyl, metalaxyl-M, metarylpicoxamid, metiram, metyltetraprole, metconazole, metominostrobin, metrafenone, mepanipyrim, mefentrifluconazole, meptyldinocap, mepronil, iodocarb, laminarin, phosphorous acid and salts, copper oxychloride, silver, copper (II) sulfate, cuprous oxide, copper hydroxide, potassium bicarbonate, sodium bicarbonate, sulfur, oxyquinoline sulfate, copper sulfate, (3,4-dichloroisothiazol-5-yl) methyl 4-(tert-butyl) benzoic acid ester (chemical name, CAS registry number: 1231214-23-5), BAF-045 (code number), BAG-010 (code number), UK-2A (code number), dodecylbenzene sulfonic acid bisethylenediamine copper complex salt [II] (DBEDC), MIF-1002 (code number), NF-180 (code number), triphenyltin acetate (TPTA), triphenyltin chloride (TPTC), triphenyltin hydroxide (TPTH), and F9650 (code number).

Next, known biopesticides that may be mixed or used in combination are exemplified.

Biopesticide:

Haplothrips brevitubus, Franklinothrips vespiformis, Diglyphus isaea, Encarsia formosa, Amblyseius cucumeris, Pseudaphycus malinus, Amblyseius womersleyi, Aphidius colemani, Eretmocerus eremicus, Aphidoletes aphidimyza, Amblyseius swirskii, Orius strigicollis, Phytoseiulus persimilis, Amblyseius degenerans, Phytoseiulus persimilis, Orius sauteri, Dacnusa sibirica, Amblyseius californicus, Chrysoperla nipponensis, and Anicetus beneficus.

Next, known agricultural materials that may be mixed or used in combination are exemplified.

Agricultural materials:

Ethylene, hypochlorite water (limited to those obtained by electrolyzing aqueous solutions of hydrochloric acid or potassium chloride), sodium bicarbonate, vinegar, humus, humic acid, fulvic acid, seaweed extract, polysaccharides, amino acids, microorganisms materials, functional ingredients derived from animals and plants, microbial metabolites, microbial activators, soil spreading agents, materials for regulating soil permeability, materials for retaining water in soil, etc., and biostimulants.

Next, known ingredients of agricultural fertilizer that may be mixed or used in combination are exemplified. Fertilizers include inorganic and organic ones

Ingredients of agricultural fertilizer:

Ammonium chloride, ammonium sulfate, ammonium nitrate, ammonium dihydrogen phosphate, urea ammonium nitrate, urea, lime nitrogen, potassium nitrate, lime superphosphate, lime superphosphate, potassium dihydrogen phosphate, potassium chloride, potassium sulfate, potassium carbonate, potassium silicate, potassium phosphite, oil cake, fish flour, rice bran, bat guano, fertilized chicken manure.

The pest control agents of the present invention configured as described above exhibit excellent control effects against harmful organisms, such as Orthoptera pests, Thysanoptera pests, Hemiptera pests, Coleoptera pests, Diptera pests, Lepidoptera pests, Hymenoptera pests, Collembola pests, Thysanura pests, Cockroach pests, Psocoptera pests, Mallophaga pests, Phthiraptera pests, plant-parasitic Acari, plant-parasitic Caenorhabditis, plant-parasitic Mollusca, other harmful animals, unpleasant animals, sanitary pests, parasites, and the like. As such harmful organisms, following biological species can be exemplfied.

As Orthoptera pests, *Ruspolia lineosa, Teleogryllus emma, Truljalia hibinonis, Gryllotalpa orientalis, Oxya hyla intricate, Locusta migratoria, Melanoplus sanguinipes, Melanoplus differentialis, Melanoplus femurrubrum, Atractomorpha lata, Euscyrtus japonicus, Xya japonicus,* and the other species can be exemplified.

As Thysanoptera pests, *Frankliniella intonsa, Frankliniella occidentalis, Scirtothrips dorsalis, Thrips palmi, Thrips tabaci, Thrips setosus, Heliothrips haemorrhoidalis, Stenchaetothrips biformis, Ponticulothrips diospyrosi, Liothrips wasabiae, Haplothrips aculeatus,* and the other species can be exemplified.

As Hemiptera pests, *Mogannia minuta, Aphrophora intermedia, Mahanarva fimbriolata,* ., *Machaerotypus sibiricus, Arboridia apicalis, Empoasca onukii, Nephotettix cincticeps, Nephotettix malayanus, Nephotettix virescens, Nephotettix nigropictus, Recilia dorsalis, Amrasca biguttula, Idioscopus nitidulus, Idioscopus clypealis, Amritodus atkinsoni, Empoasca fabae, Dalbulus maidis, Pentastiridius apicalis, Laodelphax striatellus, Nilaparvata lugens, Sogatella furcifera, Peregrinus maidis, Nisia nervosa, Kamendaka saccharivora, Achilus flammeus, Orosanga Japonicus, Mimophantia maritima,* ., *Cacopsylla pyrisuga, Diaphorina citri, Calophya mangiferae,Daktulosphaira vitifoliae, Adelges laricis, Adelges tsugae, Acyrthosiphon pisum, Aphis gossypii, Aphis spiraecola, Lipaphis erysimi, Brevicoryne brassicae, Myzus persicae, Schizaphis graminum, Rhopalosiphum padi, Toxoptera aurautii, Aulacorthum solani, Macrosiphum euphorbiae, Nasonovia ribisnigri, Sitobion avenae, Aphis glycines, Aleurocanthus camelliae, Aleurocanthus spiniferus, Bemisia tabaci, Bemisia argentifolii, Trialeurodes vaporariorum, Drosicha corpulenta, Icerya purchasi, Dysmicoccus brevipes, Pianococcus citri, Pseudococcus comstocki, Ceroplastes ceriferus, Ceroplastes rubens, Aclerda takahashii, Aonidiella aurantii, Diaspidiotus pemiciosus, Pseudaulacaspis pentagon, Unaspis yanonensis, Lygus lineolaris, Trigonotylus caelestialium, Apolygus lucorum, Nesidiocoris tenuis, Halticus bractatus, Stephanitis pyrioides, Stephanitis nashi, Eurydema rugosa, Eysarcoris lewisi, Eysarcoris aeneus, Lagynotomus elongatus, Nezara viridula, Plautia crossota, Nezara antennata, Eushistus heros, Piezodorus guildini, Tibraca limbativentris, Dichelops furcatus, Megacopta cribraria, Urochela luteovoria, Cavelerius saccharivorus, .Malcus japonicus, Dysdercus cingulatus, Leptocorisa acuta, Leptocorisa chinensis, Anacanthocoris striicornis, Rhopalus maculatus, Scaptocoris castanea, Cimex lectularis,* and the other species can be exemplified.

As Coleoptera pests, *Anomara cuprea, Anomara rufocuprea, Popillia japonica, Oxycetonia jucunda, Anomala geniculata, Oryctes rhinoceros, Heptophylla picea, Phyllophaga cuyabana, Agriotes ogurae, Agriotes lineatus, Agriotes obscurus, Melanotus okinawensis, Melanotus fortnumi, Anthrenus verbasci, Heterobostrychus hamatipennis, Stegobium paniceum, Pitinus clavipes, Tenebroides mauritanicus, Necrobia rufipes, Carpophilus hemipterus, Meligethes aeneus, Ahasverus advena, Cryptolestes ferrugineus, Epilachna varivestis, Henosepilachna vigintioctopunctata, Tenebrio molitor, Tribolium castaneum, Epicauta gorhami, Anoplophora glabripennis, Xylotrechus pyrrhoderus, Monochamus alternatus, Dectes texanus, Callosobruchus chinensis, Leptinotarsa decemlineata, Diabrotica virgifera virgifera, Diabrotica barberi, Diabrotica undecimpunctata howardi, Aulacophora femoralis, Phaedon brassicae, Cassida nebulosa, Oulema oryzae, Epilachna varivestis, Phyllotreta striolata, Demotina fasciculata, Psylliodes chrysocephala, Cerotoma trifurcate, Colaspis brunnea, Colaspis crinnicomis, Odontota homi, Chaetocnema pulicaria, Diabrotica balteata, Cylasformicarius, Hypera postica, Listroderes costirostris, Euscepes postfasciatus, Curculio sikkimensis, Anthonomus grandis, Sternechus subsignatus, Echinocnemus bipunctatus, Lissorhoptrus oryzophilus, Oryzophagus oryzae, Sitophilus zeamais, Sphenophrus venatus, Sphenophorus levis, Tomicus piniperda, Crossotarsus niponicus, Lyctus brunneus,* and the other species can be exemplified.

As Diptera pests, *Tipula aino, Plecia nearctica, Exechia shiitakevora, Pnyxia scabiei, Bradysia agrestis, Asphondylia yushimai, Mayetiola destructor, Dasineura oxycoccana, Aedes aegypti, Culex pipiens pallens, Simulium takahashii, Chironomus oryzae, Chrysops suavis, Tabanus trigonus, Eumerus strigatus, Bactrocera dorsalis, Euphranta japonica, Ceratitis capitata, Liriomyza trifolii, Liriomyza sativae, Agromyza oryzae, Liriomyza bryoniae, Chromatomyia horticola, Liriomyza chinensis, Meromyza nigriventris, Drosophila suzukii, Drosophila melanogaster, Hydrellia griseola, Hippobosca equina, Parallelpmma sasakawae, Delia antiqua, Delia platura, Fannia canicularis, Musca domestica, Stomoxys calcitrans, Sarcophaga peregrina, Gasterophilus intestinalis, Hypoderma lineatum, Oestrus ovis,* and the other species can be exemplified.

As Lepidoptera pests, *Endoclita excrescens, Antispila ampelopsia, Zeuzera leuconotum, Cossus insularis, Archips fuscocupreanus, Adoxophyes orana fasciata, Grapholita molesta, Homona magnanima, Leguminivora glycinivorella, Cydia pomonella, Lobesia botrana, Eupoecilia ambiguella, Bambalina* sp., *Eumeta minuscula, Nemapogon granella, Tinea translucens, Bucculatrix pyrivorella, Lyonetia clerkella, Lyonetia prunifoliella malinella, Caloptilia theivora, Phyllonorycter ringoniella, Phyllocnistis citrella, Acrolepiopsis sapporensis, Plutella xylostella, Yponomeuta orientalis, Argyresthia conjugella, Nokona regalis, Synanthedin hector, Phthorimaea operculella, Sitotroga cerealella, Pectinophora gossypiella, Tuta absoluta, Carposina sasakii, Illiberis pruni, Monema flavescens, Ancylolomia japonica, Chilo suppressalis, Cnaphalocrocis medinalis, Ostrinia furnacalis, Hellulla undalis, Conogethes punctiferlis, Diaphania indica, Parapediasia teterrella, Ostrinia nubilalis, Diatraea saccharalis, Cadra cautella, Galleria mellonella, Nippoptilia vitis, Papilio xuthus, Pieris rapae, Pamara guttata, Ascotis selenaria, Dendrolimus spectabilis, Malacosoma neustrium testaceum, Agrius convolvuli, Arna pseudoconspersa, Orygia recens approximans, Lymantria dispar, Hyphantria cunea, Agrotis ipsilon, Agrotis segetum, Autographa nigrisigna, Helicoverpa armigera, Helicoverpa zea, Heliothis virescens, Spodoptera exigua, Spodoptera litura, Chrysodeixis includens, Spodoptera frugiperda, Nephelodes minians,* and the other species can be exemplified.

As Hymenoptera pests, *Arge pagana, Apethymus kuri, Athalia rosae ruficornis, Dryocosmus kuriphilus, Vespa simillima xanthoptera, Solenopsis invicta, Linepithema humile, Megachile nipponica,* and the other species can be exemplified.

As Collembola pests, *Bourletiella hortensis,* and the other species can be exemplified.

As Thysanura pests, *Lepisma saccharina, Ctenolepisma villosa,* and the other speciescan be exemplified.

As Cockroach pests, *Periplaneta americana, Blattella germanica, Odontotermesformosanus, Incisitermes minor, Cryptotermes domesticus, Coptotermes formosanus, Reticulitermes speratus,* and the other species can be exemplified.

As Psocoptera pests, *Trogium pulsatorium, Liposcelis corrodens,* and the other species can be exemplified.

As Dermaptera pests, *Labodura riparia* and the other species can be exemplified.

As Mallophaga pests, *Lipeurus caponis, Damalinia bovis,* and the other species can be exemplified.

As Phthiraptera pests, *Haematopinus suis, Pediculus humanus, Linognathus setosus, Pthirus pubis,* and the other species can be exemplified.

As Acari pests, *Penthaleus major, Phytonemus pallidus, Polyphagotarsonemus latus, Siteroptes sp., Brevipalpus lewisi, Tuckerella pavoniformis, Eotetranychus boreus, Panonychus citri, Panonychus ulmi, Tetranychus urticae, Tetranychus kanzawai, Trisetacus pini, Aculops pelekassi, Epitrimerus pyri, Phyllocoptruta oleivora, Aculops lycopersici, Diptacus crenatae, Aleuroglyphus ovatus, Tyrophagus putrescentiae, Rhizoglyphus robini, Varroa jacobsoni, Dermanyssus gallinae, Omithonyssus sylviarum, Boophilus microplus, Rhipicephalus sanguineus, Haemaphysalis longicomis, Sarcoptes scabiei,* and the other species can be exemplified.

As Plant-parasitic *Caenorhabditis, Xiphinema index, Paratrichodorus minor, Rhabditella* sp., *Aglenchus* sp., *Cephalenchus* sp., *Nothotylenchus acris, Ditylenchus destructor, Rotylenchulus reniformis, Helicotylenchus dihystera, Paratylenchus curvitatus, Meloidogyne incognita, Meloidogyne hapla, Meloidogyne javanica, Meloidogyne chitwoodi, Meloidogyne fallax, Globodera rostochiensis, Globodera pallida, Heterodera glycines, Heterodera Schachtii, Tylenchorhynchus claytoni, Psilenchus* sp., *Criconemoides* sp., *Tylenchulus semipenetrans, Sphaeronema camelliae, Radopholus citrophilus, Radopholus similis, Nacobbus aberrans, Pratylenchus penetrans, Pratylenchus coffeae, Pratylenchus zeae, Pratylenchus brachyurus, Iotonchium ungulatum, Aphelenchus avenae, Aphelenchoides besseyi, Aphelenchoides fragariae, Bursaphelenchus xylophilus,* and the other species can be exemplified.

As plant-parasitic Mollusca, *Pomacea canaliculata, Leavicaulis alte, Achatina fulica, Meghimatium bilineatum,* Succinea lauta, *Discus pauper, Zonitoides yessoensis, Limax flavus, Lehmannia valentiana, Deroceras reticulatum, Parakaliella harimensis, Acusta despecta sieboldiana, Bradybaena similaris,* and the other species can be exemplified.

As other harmful organisms such as harmful animals, unpleasant animals, sanitary pests, pests in livestock, parasites, and the like, *Procambarus clarkii, Porcellio scaber, Armadillidium vulgare,* Chilopoda pests such as *Thereuonema tuberculata* and *Scolopendra subspinipes,* Diplopoda pests such as *Oxidus gracilis, Theridiidae hasseltii, Cheiracanthium japonicum, Androctonus crassicauda,* parasites inside of nematodes such as *Ascaris lumbricoides, Syphacia* sp., and *Wuchereria bancrofti,* parasites inside of flatworms such as *Distomum* sp., *Paragonimus westermanii, Metagonimus yokokawai, Schistosoma japonicum, Taenia solium, Taeniarhynchus saginatus, Echinococcus* sp., *Diphyllobothrium latum,* and the other species can be exemplified.

The pest control agents of the present invention also exhibit control effects on the pests exemplified above and the like, which have acquired resistance to the existing pest control agents. In addition, the pest control agents of the present invention can also be used for the plants that have acquired characteristics such as pest resistance, disease resistance, and herbicide resistance by genetic recombination, artificial mating, and the like.

The "plants to which resistance has been given by breeding methods or genetic recombination techniques" of the present invention includ not only those to which resistance has been given by classical cultivar mating and genetic recombination techniques, but also those to which resistance has been given by new plant breeding techniques (NBTs)

which combine techniques of molecular biology with conventional mating techniques. The new plant breeding techniques (NBT) are described in the book "Let's Understand New Plant Breeding Technology" (from International Literature Company, by Ryo Osawa and Hiroshi Egami), the review "Genome Editing Tools in Plants" (Genes 2017, 8, 399, by Tapan Kumar Mohanta, Tufail Bashir, Abeer Hashem, Elsayed Fathi Abd_Allah, and Hanhong Bae), and the like.

## Examples

Next, the methods for producing the compounds of the present invention, the formulation methods, and the use thereof will be described in detail in the following examples. However, the present invention is not limited to these examples. As physical properties of the compounds of the present invention, the melting point was measured with a Yanaco MP-500 V micro melting point apparatus, the refractive index was measured with an Abbe refractometer manufactured by Atago, and the $^1$H-NMR spectra were measured with a JNM-ECS300 (300 MHz) manufactured by JEOL using tetramethylsilane (TMS) as an internal standard.

In addition, methods for producing intermediates of the compounds of the present invention ar also described.

Example 1

Production of 1-[3-(ethylthio)-6-(trifluoromethyl)imidazo[1,2-a]pyridin-2-yl]-1H-pyrazol-4-ol (Compound number of the present invention: A-0479)

1) 2-[4-(benzyloxy)-1H-pyrazol-1-yl]-6-(trifluoromethyl) imidazo [1,2-a] pyridine

2-bromo-6-(trifluoromethyl)imidazo[1,2-a]pyridine (1.0 g, 3.8 mmol) which was synthesized according to International Publication No.2019/124548, 4-(benzyloxy)-IH-pyrazole (0.66 g, 3.8 mmol), copper (I) iodide (0.36 g, 1.9 mmol), N,N'-dimethylethylenediamine (0.17 g, 1.9 mmol), and tripotassium phosphate n-hydrate (1.6 g, 7.5 mmol as anhydrate) were sequentially added to N-methyl-2-pyrrolidone (7.5 mL), and further stirred in a sealed tube at 140°C for 8 hours. The reaction solution was poured into dilute aqueous ammonia. Ethyl acetate was added, and the mixture was filtered through celite. The organic layer was washed with water and saturated brine. The organic layer was dried over anhydrous magnesium sulfate, and the solvent was distilled off under reduced pressure. The residue was purified by silica gel column chromatography, and 2-[4-(benzyloxy)-1H-pyrazol-1-yl]-6-(trifluoromethyl)imidazo[1,2-a]pyridine (0.40 g, yield: 30%) was obtained.

$^1$H-NMR data (300 MHz, $CDCl_3$/TMS $\delta$ (ppm)): 5.03 (2H, s), 7.31-7.46 (6H, m), 7.53 (1H, s), 7.64 (1H, d), 7.88 (1H, s), 8.02 (1H, s), 8.49 (1H, br s)

2) 2-[4-(benzyloxy)-1H-pyrazol-1-yl]-3-iodo-6-(trifluoromethyl)imidazo[1,2-a]pyridine

2-[4-(benzyloxy)-1H-pyrazol-1-yl]-6-(trifluoromethyl)imidazo[1,2-a]pyridine (0.40 g, 1.1 mmol) was dissolved in acetonitrile (11 mL). The mixture was added with N-iodosuccinimide (0.30 g, 1.3 mmol), and further stirred at room temperature overnight. The reaction solution was poured into an aqueous solution of sodium thiosulfate and extracted with ethyl acetate. The organic layer was washed successively with aqueous solutions of potassium carbonate and sodium thiosulfate, and the organic layer was dried over anhydrous magnesium sulfate. The solvent was distilled off under reduced pressure and 2-[4-(benzyloxy)-1H-pyrazol-1-yl]-3-iodo-6-(trifluoromethyl)imidazo[1,2-a]pyridine (0.46 g, yield: 85%) was obtained.

$^1$H-NMR data (300 MHz, $CDCl_3$ / TMS $\delta$ (ppm)): 5.04 (2H, s), 7.32-7.48 (6H, m), 7.63 (1H, d), 7.64 (1H, d), 8.03 (1H, d), 8.62-8.63 (1H, m)

3) 1-[3-(ethylthio)-6-(trifluoromethyl)imidazo[1,2-a]pyridin-2-yl]-1H-pyrazol-4-ol (Compound number of the present invention: A-0479)

2-[4-(benzyloxy)-1H-pyrazol-1-yl]-3-iodo-6-(trifluoromethyl)imidazo[1,2-a]pyridine (0.46 g, 0.95 mmol), ethanethiol (0.69 mL, 9.6 mmol), N,N-diisopropylethylamine (0.15 g, 1.2 mmol), tris(dibenzylideneacetone)dipalladium (0) (0.043 g, 0.047 mmol), and Xantphos (0.055 g, 0.095 mmol) were sequentially added to 1,4-dioxane (9.5 mL), and the mixture was further stirred under heating under reflux for 3 hours. The reaction solution was concentrated. The residue was purified by silica gel column chromatography, and 2-[4-(benzyloxy)-1H-pyrazol-1-yl]-3-(ethylthio)-6-(trifluoromethyl)imidazo[1,2-a]pyridine was obtained. The obtained 2-[4-(benzyloxy)-1H-pyrazol-1-yl]-3-(ethylthio)-6-(trifluoromethyl)imidazo[1,2-a]pyridine was dissolved in nitromethane (9.5 mL). The mixture was added with aluminum chloride (0.29 g, 2.2 mmol), and further stirred at room temperature for 1 hour. The reaction solution was poured into dilute hydrochloric acid and extracted with ethyl acetate. The organic layer was dried over anhydrous magnesium sulfate, and

the solvent was distilled off under reduced pressure. The residue was washed with a mixed solvent of hexane / diisopropyl ether (1:1), and 1-[3-(ethylthio)-6-(trifluoromethyl)imidazo[1,2-a]pyridin-2-yl]-1H-pyrazol-4-ol (0.27 g, yield: 86%) was obtained.

$^1$H-NMR data (300 MHz, DMSO-$d_6$/TMS δ (ppm)): 1.06 (3H, t), 2.91 (2H, q), 7.52 (1H, d), 7.71 (1H, dd), 7.85 (1H, d), 7.90 ( 1H, d), 8.97 (1H, br s), 9.08 (1H, s)

Melting point: 199-202°C

Example 2

Production of 3-(ethylsulfonyl)-2-[4-(2,2,3,3,4,4,4-heptafluorobutoxy)-1H-pyrazol-1-yl]-6-(trifluoromethyl)imidazo[1,2-a]pyridine (Compound number of the present invention: A-0046)

1-[3-(ethylthio)-6-(trifluoromethyl)imidazo[1,2-a]pyridin-2-yl]-1H-pyrazol-4-ol (0.27 g, 0.82 mmol), potassium carbonate (0.17 g, 1.2 mmol), and 2,2,3,3,4,4,4-heptafluorobutyl nonafluorobutanesulfonate (0.44 g, 0.91 mmol) were added sequentially to N,N-dimethylformamide (8.2 mL), and the mixture was further stirred overnight at room temperature. The reaction solution was poured into water and extracted with ethyl acetate. The organic layer was washed with water and saturated brine, and the organic layer was dried over anhydrous magnesium sulfate. The solvent was distilled off under reduced pressure, and 3-(ethylthio)-2-[4-(2,2,3,3,4,4,4-heptafluorobutoxy)-1H-pyrazol-1-yl]-6-(trifluoromethyl)imidazo[1,2-a]pyridine was obtained. The obtained 3-(ethylthio)-2-[4-(2,2,3,3,4,4,4-heptafluorobutoxy)-1H-pyrazol-1-yl]-6-(trifluoromethyl)imidazo[1,2-a]pyridine was dissolved in dichloromethane (7.3 mL). The mixture was added with m-chloroperbenzoic acid (content: 77%, 0.35 g, 1.6 mmol), and further stirred at room temperature for 3 hours. The reaction solution was poured into an aqueous solution of potassium carbonate and extracted with ethyl acetate. The organic layer was dried over anhydrous magnesium sulfate, and the solvent was distilled off under reduced pressure. The residue was purified by silica gel column chromatography and 3-(ethylsulfonyl)-2-[4-(2,2,3,3,4,4,4-heptafluorobutoxy)-1H-pyrazol-1-yl]-6-(trifluoromethyl)imidazo[1,2-a] pyridine (0.26 g, yield: 66%) was obtained.

$^1$H-NMR data (300 MHz, $CDCl_3$ / TMS δ (ppm)): 1.42 (3H, t), 3.91 (2H, q), 4.44 (2H, tt), 7.66 (1H, dd), 7.67 (1H, d), 7.77 (1H, d), 8.06 (1H, d), 9.63-9.65 (1H, m)
Melting point: 134-136°C

Example 3

Production of 2- [4-(benzyloxy)-1H-pyrazol-1-yl]-6-bromo-3- (ethylsulfonyl) imidazo [1,2-a] pyridine (Compound number of the present invention: A-0612)

1) 6-Bromo-2-chloro-3-(ethylthio) imidazo [1,2-a] pyridine

6-Bromo-2-chloroimidazo [1,2-a] pyridine (3.50 g, 15 mmol), which was synthesized according to the description of the specification of international publication No. 2009/144395, diethyl disulfide (2.77 g, 23 mmol), and iodobenzene diacetate (7.31 g, 23 mmol) were added sequentially to acetonitrile (30 mL), and the mixture was further stirred under heating and reflux for 4 hours. The reaction solution was poured into an aqueous solution of sodium thiosulfate and extracted with ethyl acetate. The organic layer was washed with water and saturated brine. The organic layer was dried over anhydrous magnesium sulfate, and the solvent was distilled off under reduced pressure. The residue was purified by silica gel column chromatography. 6-Bromo-2-chloro-3- (ethylthio) imidazo [1,2-a] pyridine (3.81 g, yield: 86%) was obtained.

$^1$H-NMR data (300 MHz, $CDCl_3$/ TMS δ (ppm)): 1.23 (3H, t), 2.75 (2H, q), 7.37 (1H, dd), 7.47 (1H, dd), 8.51 (1H, d)

2) 6-Bromo-2-chloro-3-(ethylsulfonyl) imidazo [1,2-a] pyridine

6-Bromo-2-chloro-3-(ethylthio) imidazo [1,2-a] pyridine (3.81 g, 13 mmol) was dissolved in chloroform (65 mL). M-chloroperbenzoic acid (content: 70%, 8.05 g, 33 mmol) was added to the mixture under ice-cooling, and further stirred at room temperature for 3 hours. The reaction solution was poured into an aqueous solution of potassium carbonate. An aqueous solution of sodium thiosulfate was added, and extracted with chloroform. The organic layer was washed with an aqueous solution of potassium carbonate and saturated brine, and dried over anhydrous magnesium sulfate. The solvent was distilled off under reduced pressure. 6-Bromo-2-chloro-3-(ethylsulfonyl) imidazo [1,2-a] pyridine (4.20 g, yield: 99%) was obtained.

$^1$H-NMR data (300 MHz, $CDCl_3$ / TMS δ (ppm)): 1.36 (3H, t), 3.37 (2H, q), 7.58 (2H, m), 9.16 (1H, d)

3) 2-[4-(Benzyloxy)-1H-pyrazol-1-yl]-6-bromo-3-(ethylsulfonyl) imidazo [1,2-a] pyridine

(Compound number of the present invention: A-0612)

6-Bromo-2-chloro-3-(ethylsulfonyl)imidazo [1,2-a] pyridine (4.13 g, 13 mmol), 4-(benzyloxy) -1H-pyrazole (2.45 g, 14 mmol), and cesium carbonate (6.24 g, 19 mmol) were added sequentially to dimethyl sulfoxide (26 mL) and further stirred at 100°C for 8 hours. The reaction solution was poured into water and extracted with ethyl acetate. The organic layer was washed with saturated brine, dried over anhydrous magnesium sulfate, and the solvent was distilled off under reduced pressure. The residue was washed with a mixed solvent of acetone / diisopropyl ether (1:1). 2-[4-(Benzyloxy)-1H-pyrazol-1-yl]-6-bromo-3-(ethylsulfonyl)imidazo [1,2-a] pyridine (2.68 g, yield: 46%) was obtained.

$^1$H-NMR data (300 MHz, DMSO-$d_6$/ TMS δ (ppm)): 1.30 (3H, t), 3.90 (2H, q), 5.09 (2H, s), 7.32-7.49 (5H, m), 7.78-7.87 (3H, m), 8.16 (1H, d), 9.17 (1H, d)

Example 4

Production of 1-[6-bromo-3-(ethylsulfonyl) imidazo [1,2-a] pyridin-2-yl]-1H-pyrazol-4-ol (Compound number of the present invention: A-0476)

2-[4-(Benzyloxy)-1H-pyrazol-1-yl]-6-bromo-3-(ethylsulfonyl)imidazo[1,2-a] pyridine (2.68 g, 5.8 mmol) was dissolved in nitromethane (29 mL) and dichloromethane (29 mL). Aluminum chloride (1.55 g, 12 mmol) was added to the mixture, and further stirred at room temperature for 1 hour. Dilute hydrochloric acid was poured into the reaction solution, and diisopropyl ether was added to precipitate crude crystals. The crude crystals were washed with diisopropyl ether. 1-[6-Bromo-3-(ethylsulfonyl) imidazo [1,2-a] pyridin-2-yl]-1H-pyrazol-4-ol (1.72 g, yield: 80%) was obtained.

$^1$H-NMR data (300 MHz, DMSO-$d_6$ / TMS δ (ppm)): 1.29 (3H, t), 3.90 (2H, q), 7.54 (1H, d), 7.77-7.85 (3H, m), 9.17 (1H, d), 9 .17 (1H, br s)

Example 5

Production of 6-bromo-3-(ethylsulfonyl)-2-[4-(2,2,3,3,4,4,4-heptafluorobutoxy)-1H-pyrazol-1-yl] imidazo [1,2-a] pyridine (Compound number of the present invention: A-0039)

1-[6-Bromo-3-(ethylsulfonyl)imidazo [1,2-a] pyridin-2-yl]-1H-pyrazol-4-ol (1.58 g, 4.3 mmol), potassium carbonate (0.88 g, 6.4 mmol) and 2,2,3,3,4,4,4-heptafluorobutyl nonafluorobutanesulfonate (2.26 g, 4.7 mmol) were added sequentially to acetonitrile (21 mL), and was further stirred at 80°C for 4 hours. The reaction solution was concentrated and the residue was purified by silica gel column chromatography. 6-Bromo-3-(ethylsulfonyl)-2-[4-(2,2,3,3,4,4,4-heptafluorobutoxy)-1H-pyrazol-1-yl] imidazo [1,2-a] pyridine (2.13 g, yield: 90%) was obtained.

$^1$H-NMR data (300 MHz, $CDCl_3$ / TMS δ (ppm)): 1.40 (3H, t), 3.87 (2H, q), 4.43 (2H, tt), 7.53-7.65 (3H, m), 8.01 (1H, d), 9.38 (1H, d)
Melting point: 171-174°C

Example 6

Production of 6-cyclopropyl-3-(ethylsulfonyl)-2-[4-(2,2,3,3,4,4,4-heptafluorobutoxy)-1H-pyrazol-1-yl] imidazo [1,2-a] pyridine (Compound number of the present invention: A-0049)

6-Bromo-3-(ethylsulfonyl)-2-[4-(2,2,3,3,4,4,4-heptafluorobutoxy)-1H-pyrazol-1-yl] imidazo [1,2-a] pyridine (4.25 g, 7.7 mmol), cyclopropylboronic acid (1.32 g, 15 mmol), tripotassium phosphate n-hydrate (4.89 g, 23 mmol as anhydrate), palladium (II) acetate (0.086 g, 0.38 mmol), and 2-dicyclohexylphosphino-2',6'-dimethoxybiphenyl (0.32 g, 0.78 mmol) were added sequentially to 1,4-dioxane (40 mL) and water (4 mL), and further stirred under heating and reflux for 6 hours. The reaction solution was poured into water and extracted with ethyl acetate. The organic layer was washed with water and saturated brine, dried over anhydrous magnesium sulfate, and the solvent was distilled off under reduced pressure. The residue was purified by silica gel column chromatography. 6-Cyclopropyl-3-(ethylsulfonyl)-2-[4-(2,2,3,3,4,4,4-heptafluorobutoxy)-1H-pyrazol-1-yl] imidazo [1,2-a] pyridine (2.02 g, yield: 52%) was obtained.

$^1$H-NMR data (300 MHz, $CDCl_3$ / TMS δ (ppm)): 0.76 (2H, m), 1.06 (2H, m), 1.39 (3H, t), 1.99 (1H, m), 3.81 (2H, q), 4.42 (2H, tt), 7.21 (1H, dd), 7.55 (1H, dd), 7.62 (1H, d), 7.98 (1H, d), 9.01 (1H, d)
Melting point: 117-120°C

The values of physical property of compound [I] of the present invention synthesized according to the above-mentioned examples are shown in Tables 24 to 28 below, including the values in the above-mentioned examples. The

compound numbers and symbols in the table have the same meanings as described above.

Table 24

| compound no. | physical property | |
|---|---|---|
| | melting point (°C) | refractive index ($n_D^{20}$) |
| A-0003 | 103-105 | |
| A-0005 | 92-95 | |
| A-0006 | 93-96 | |
| A-0038 | 176-179 | |
| A-0039 | 171-174 | |
| A-0042 | 235-238 | |
| A-0044 | 126-129 | |
| A-0045 | 111-114 | |
| A-0046 | 134-136 | |
| A-0047 | 127-130 | |
| A-0049 | 117-120 | |
| A-0056 | 125-128 | |
| A-0058 | 127-129 | |
| A-0060 | 184-187 | |
| A-0075 | 195-197 | |
| A-0162 | 137-140 | |
| A-0164 | 150-153 | |
| A-0183 | 160-163 | |
| A-0184 | 230-233 | |
| A-0185 | 156-159 | |
| A-0189 | 160-162 | |
| A-0191 | 150-153 | |
| A-0192 | 229-231 | |
| A-0193 | 210-213 | |
| A-0195 | 222-224 | |
| A-0199 | 185-188 | |
| A-0207 | 130-132 | |
| A-0209 | 66-68 | |
| A-0210 | 113-116 | |
| A-0213 | 118-121 | |
| A-0218 | 124-126 | |
| A-0256 | 203-206 | |
| A-0259 | 135-136 | |
| A-0274 | 132-134 | |
| A-0423 | 157-159 | |

(continued)

| compound no. | physical property | |
|---|---|---|
| | melting point (°C) | refractive index ($n_D^{20}$) |
| A-0426 | 103-106 | |
| A-0458 | 170-171 | |
| A-0479 | 199-202 | |
| A-0579 | 94-97 | |
| A-0580 | 113-115 | |
| A-0581 | 135-138 | |
| A-0582 | 128-131 | |
| A-0583 | 182-184 | |

Table 25

| compound no. | physical property | |
|---|---|---|
| | melting point (°C) | refractive index ($n_D^{20}$) |
| A-0584 | 154-156 | |
| A-0585 | 148-151 | |
| A-0587 | 197-200 | |
| A-0589 | 283-286 | |
| A-0590 | 203-206 | |
| A-0591 | 167-169 | |
| A-0592 | 178-180 | |
| A-0593 | 114-117 | |
| A-0594 | 175-178 | |
| A-0595 | 115-118 | |
| A-0596 | 152-155 | |
| A-0597 | 190-193 | |
| A-0598 | 215-218 | |
| A-0599 | 257-260 | |
| A-0600 | 192-194 | |
| A-0602 | 196-197 | |
| A-0603 | 173-176 | |
| A-0604 | 155-158 | |
| A-0605 | 161-163 | |
| A-0606 | 178-181 | |
| A-0607 | 136-138 | |
| A-0608 | 170-173 | |
| A-0609 | 185-188 | |
| A-0610 | 240-243 | |

(continued)

| compound no. | physical property | |
|---|---|---|
| | melting point (°C) | refractive index ($n_D^{20}$) |
| A-0611 | 134-137 | |
| A-0612 | 182-185 | |

Table 26

| compound no. | physical property $^1$H-NMR data [TMS δ(ppm)] | |
|---|---|---|
| A-0050 | 300 MHz, $CDCl_3$ | 1.39 (3H, t), 1.58-1.68 (2H, m), 1.85-1.91 (2H, m), 2.25-2.30 (2H, m), 3.85 (2H, q), 4.24 (1H, dt), 4.43 (2H, tt), 7.08 (1H, dd), 7.64-7.69 (2H, m), 8.02 (1H, d), 9.23 (1H, dd) |
| A-0068 | 300 MHz, $CDCl_3$ | 1.42 (3H, t), 3.89 (2H, q), 4.48 (2H, tt), 7.43-7.77 (4H, m), 8.05 (1H, d), 8.71 (1H, dd), 8.88 (1H, d), 9.45 (1H, d) |

Table 27

| compound no. | physical property | |
|---|---|---|
| | melting point (°C) | refractive index ($n_D^{20}$) |
| C-0025 | 157-158 | |
| C-0026 | 107-110 | |

Table 28

| compound no. | physical property | |
|---|---|---|
| | melting point (°C) | refractive index ($n_D^{20}$) |
| D-0006 | 157-159 | |
| D-0021 | 135-138 | |

Next, formulation examples of the pest control agents of the present invention using the heterocyclic compounds produced as described above or agriculturally acceptable salts thereof will be specifically described. However, the types and blending ratios of the compounds and additives are not limited thereto and can be changed in a wide range. Further, in the following description, the "portion" means a mass portion.

[Formulation example 1] Emulsifiable concentrate

| | |
|---|---|
| A compound listed in Tables 1 to 28 | 10 portions |
| Cyclohexanone | 30 portions |
| Polyoxyethylene alkylaryl ether | 11 portions |
| Calcium alkylbenzene sulfonate | 4 portions |
| Methyl naphthalene | 45 portions |

The above ingredients were uniformly dissolved to obtain an emulsifiable concentrate.

[Formulation example 2] Wettable powder

| | |
|---|---|
| A compound listed in Tables 1 to 28 | 10 portions |

(continued)

| [Formulation example 2] | Wettable powder |
|---|---|
| Naphthalene sulfonic acid formalin condensate sodium salt | 0.5 portions |
| Polyoxyethylene alkylaryl ether | 0.5 portions |
| Diatomaceous earth | 24 portions |
| Clay | 65 portions |

The above ingredients were uniformly mixed and pulverized to obtain a wettable powder.

| [Formulation example 3] | Powder |
|---|---|
| A compound listed in Tables 1 to 28 | 2 portions |
| Diatomaceous earth | 5 portions |
| Clay | 93 portions |

The above ingredients were uniformly mixed and pulverized to obtain a powder.

| [Formulation example 4] | Granule |
|---|---|
| A compound listed in Tables 1 to 28 | 5 portions |
| Sodium salt of lauryl alcohol sulfate ester | 2 portions |
| Sodium lignin sulfonate | 5 portions |
| Carboxymethyl cellulose | 2 portions |
| Clay | 86 portions |

The above ingredients were uniformly mixed and pulverized. An amount equivalent to 20 portions of water was added to the mixture, kneaded, processed into granule of 14 to 32 mesh using an extrusion granulator, and then dried to obtain a granule.

| [Formulation example 5] | Suspension concentrate |
|---|---|
| A compound listed in Tables 1 to 28 | 20 portions |
| Polyoxyethylene styrenated phenyl ether sulfate | 4 portions |
| Ethylene glycol | 7 portions |
| Silicone AF-118N (manufactured by Asahi Kasei) | 0.02 portions |
| Water | 68.98 portions |

The above ingredients were mixed with a high-speed stirrer for 30 minutes and then pulverized with a wet pulverizer to obtain a suspension concentrate.

| [Formulation example 6] | Water dispersible granule |
|---|---|
| A compound listed in Tables 1 to 28 | 10 portions |
| Sodium lignin sulfonate | 5 portions |
| Polyoxyethylene alkylaryl ether | 1 portion |
| Sodium polycarboxylate | 3 portions |
| White carbon | 5 portions |
| Pregelatinized starch | 1 portion |
| Calcium carbonate | 65 portions |
| Water | 10 portions |

The above ingredients were mixed, kneaded, pressed and granulated. The obtained granules were dried in a fluidized bed dryer to obtain a water dispersible granule.

Next, the effects of the pest control agents of the present invention will be described with reference to test

examples.

[Test example 1] Test on insecticidal activity in *Plutella xylostella*

A wettable powder prepared according to formulation example 2 was diluted with water to a concentration of 500ppm as the active ingredient. Some cabbage leaves were dipped in the drug solution, air-dried, and then placed in a plastic cup. Ten second-instar larvae of *Plutella xylostella* were put on the treated leaves, and the cup was covered with a lid. Then, it was placed in a room at constant temperature of 25°C. The number of dead insects was investigated at 6 days after treatment. Mortality rate was calculated using the formula 1. The number of replication was one in each test.

[Formula 1]

$$\text{Mortality (\%)} = \left(1 - \frac{\text{Number of insects survived}}{\text{Number of insects provided for test}}\right) \times 100$$

The compound numbers that showed a mortality of 90% or more in the test are listed below. A-0003, A-0005, A-0006, A-0038, A-0039, A-0042, A-0044, A-0045, A-0046, A-0049, A-0050, A-0056, A-0058, A-0060, A-0068, A-0075, A-0162, A-0183, A-0184, A-0185, A-0189, A-0192, A-0193, A-0195, A-0199, A-0207, A-0209, A-0210, A-0213, A-0218, A-0256, A-0259, A-0274, A-0423, A-0426, A-0458, A-0579, A-0580, A-0581, A-0582, A-0583, A-0584, A-0585, A-0587, A-0589, A-0590, A-0591, A-0592, A-0593, A-0594, A-0595, A-0596, A-0597, A-0598, A-0599, A-0600, A-0602, A-0603, A-0604, A-0605, A-0606, A-0607, A-0608, A-0610, A-0611, D-0006, and D-0021.

[Test example 2] Test on insecticidal activity in *Helicoverpa armigera*

A wettable powder prepared according to formulation example 2 was diluted with water to a concentration of 500ppm as the active ingredient. Some cabbage leaves were dipped in the drug solution, air-dried, and then placed in a plastic cup. Five hatching larvae of *Helicoverpa armigera* were put on the treated leaves, and the cup was covered with a lid. Then, it was placed in a room at constant temperature of 25°C. The number of dead insects was investigated at 6 days after the treatment. Mortality rate was calculated using the formula 1. The number of replication was two in each test.

The compound numbers that showed a mortality of 90% or more in the test are listed below. A-0003, A-0005, A-0006, A-0038, A-0039, A-0044, A-0046, A-0049, A-0058, A-0183, A-0207, A-0209, A-0210, A-0213, A-0218, A-0259, A-0274, A-0423, A-0426, A-0458, A-0583, A-0584, A-0587, A-0589, A-0591, D-0006, and D-0021.

[Test example 3] Test on insecticidal activity in *Nilaparvata lugens*

A wettable powder prepared according to formulation example 2 was diluted with water to a concentration of 500ppm as the active ingredient. Some rice sprouts were dipped in the drug solution and placed in a plastic cup. Ten second-instar larvae of *Nilaparvata lugens* were put on the treated leaves, and the cup was covered with a lid. Then, it was placed in a constant room temperature of 25°C. The number of dead insects was investigated at 6 days after the treatment. Mortality rate was calculated using the formula 1. The number of replication was one in each test.

The compound numbers that showed a mortality of 90% or more in the test are listed below. A-0003, A-0005, A-0006, A-0038, A-0039, A-0044, A-0045, A-0046, A-0049, A-0050, A-0056, A-0058, A-0162, A-0183, A-0184, A-0210, A-0213, A-0218, A-0579, A-0580, A-0584, A-0592, A-0593, A-0594, A-0595, A-0600, A-0605 and A-0611.

[Test example 4] Test on pest control effect in *Aphis gossypii*

A wettable powder prepared according to formulation example 2 was diluted with water to a concentration of 500ppm as the active ingredient. Some nursery plant of cucumber previously inoculated with larvae of *Aphis gossypii* were dipped in the drug solution and air-dried. The treated nursery plants of cucumber were placed in a room at constant temperature of 25°C. The number of surviving insects was counted at 3 days after the treatment. Mortality rate was calculated using the formula 1. The number of replication was one in each test.

The compound numbers that showed a mortality of 90% or more in the test are listed below. A-0003, A-0005, A-0006, A-0038, A-0039, A-0042, A-0044, A-0045, A-0046, A-0049, A-0050, A-0056, A-0058, A-0162, A-0183, A-0184, A-0185, A-0189, A-0195, A-0199, A-0207, A-0210, A-0213, A-0218, A-0259, A-0274, A-0423, A-0426, A-0579, A-0580, A-0581, A-0582, A-0584, A-0585, A-0589, A-0590, A-0591, A-0592, A-0593, A-0594, A-0595, A-0596, A-0597, A-0598, A-0599, A-0600, A-0603, A-0604, A-0605, A-0607, A-0608, A-0610, A-0611, D-0006, and D-0021.

## Industrial applicability

The present invention provides novel compounds having excellent insecticidal activity, production intermediates thereof, and pest control agents containing the same as active ingredients. It is useful in the fields of pesticides and agriculture, and has the potential for industrial use.

## Claims

1. A compound represented by the general formula [I] or a salt thereof, wherein,

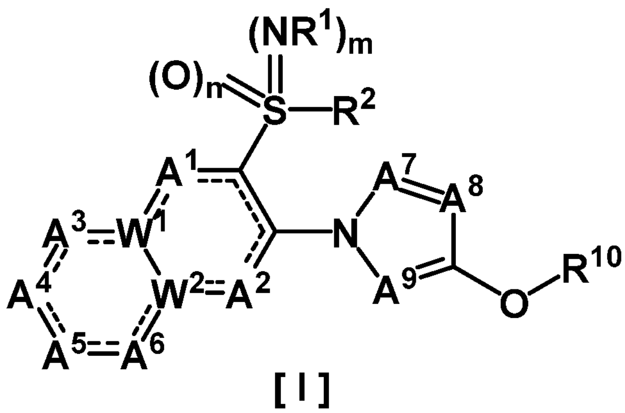

[ I ]

each of m and n independently represents an integer of 0, 1 or 2, and the sum thereof is an integer in the range of 0 to 2,
$R^1$ represents a hydrogen atom, cyano group, or $C_1$-$C_7$ acyl group which is unsubstituted or substituted with a halogen atom,
$R^2$ represents a $C_1$-$C_6$ alkyl group, $C_1$-$C_6$ alkoxy $C_1$-$C_6$ alkyl group, $C_1$-$C_6$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_3$-$C_6$ cycloalkyl group, $C_3$-$C_6$ halocycloalkyl group, $C_3$-$C_6$ cycloalkyl $C_1$-$C_6$ alkyl group, $C_3$-$C_6$ halocycloalkyl $C_1$-$C_6$ alkyl group, cyano $C_1$-$C_6$ alkyl group, $C_7$-$C_{14}$ aralkyl group which is unsubstituted or optionally substituted with $R^{11}$, or $C_6$-$C_{14}$ heteroaralkyl group which is unsubstituted or optionally substituted with $R^{11}$,
$W^1$ represents a nitrogen atom,
$W^2$ represents a carbon atom, or
$W^1$ represents a carbon atom,
$W^2$ represents a nitrogen atom,
$A^1$ represents a nitrogen atom, a carbon atom substituted with $R^a$, or a bond,
$A^2$ represents a nitrogen atom, a carbon atom substituted with $R^b$, or a bond,
$A^3$ represents a nitrogen atom, a carbon atom substituted with $R^3$, or a bond,
each of $A^4$, $A^5$, $A^6$, $A^7$, $A^8$ and $A^9$ independently represents a nitrogen atom, or a carbon atom substituted with $R^4$, $R^5$, $R^6$, $R^7$, $R^8$ and $R^9$ respectively,
each of $R^a$, $R^b$, $R^3$, $R^4$, $R^5$, $R^6$, $R^7$, $R^8$ and $R^9$ independently represents a hydrogen atom, halogen atom, azido group, cyano group, nitro group, $C_1$-$C_6$ alkyl group, cyano $C_1$-$C_6$ alkyl group, hydroxy $C_1$-$C_6$ alkyl group, $C_1$-$C_6$ haloalkyl group, $C_2$-$C_6$ alkenyl group, $C_2$-$C_6$ alkynyl group, $C_3$-$C_6$ cycloalkyl group, cyano $C_3$-$C_6$ cycloalkyl group, hydroxy $C_3$-$C_6$ cycloalkyl group, $C_3$-$C_6$ halocycloalkyl group, $C_3$-$C_6$ cycloalkyl $C_1$-$C_6$ alkyl group, $C_3$-$C_6$ cycloalkyl $C_2$-$C_6$ alkenyl group, $C_3$-$C_6$ cycloalkyl $C_2$-$C_6$ alkynyl group, $C_3$-$C_6$ halocycloalkyl $C_1$-$C_6$ alkyl group, heterocyclic group which is unsubstituted or optionally substituted with $R^{11}$, $C_6$-$C_{12}$ aryl group which is unsubstituted or optionally substituted with $R^{11}$, $C_5$-$C_{12}$ heteroaryl group which is unsubstituted or optionally substituted with $R^{11}$, $C_7$-$C_{14}$ aralkyl group which is unsubstituted or optionally substituted with $R^{11}$, $C_6$-$C_{14}$ heteroaralkyl group which is unsubstituted or optionally substituted with $R^{11}$, hydroxy group, $C_1$-$C_6$ alkoxy group, $C_1$-$C_6$ haloalkoxy group, cyano $C_1$-$C_6$ alkoxy group, hydroxy $C_1$-$C_6$ alkoxy group, $C_1$-$C_6$ haloalkoxy $C_1$-$C_6$ alkoxy group, $C_1$-$C_6$ alkoxy $C_1$-$C_6$ haloalkoxy group, $C_1$-$C_6$ haloalkoxy $C_1$-$C_6$ haloalkoxy group, $C_3$-$C_6$ cycloalkyloxy group, $C_3$-$C_6$ cycloalkyl $C_1$-$C_6$ alkoxy group, $C_3$-$C_6$ halocycloalkyloxy group, cyano $C_3$-$C_6$ cycloalkyloxy group, $C_3$-$C_6$ halocycloalkyl $C_1$-$C_6$ alkoxy group, (cyano $C_3$-$C_6$ cycloalkyl) $C_1$-$C_6$ alkoxy group, $C_1$-$C_6$ haloalkylthio $C_1$-$C_6$ alkoxy group, $C_1$-$C_6$ haloalkylsulfinyl $C_1$-$C_6$ alkoxy group, $C_1$-$C_6$ haloalkylsulfonyl $C_1$-$C_6$ alkoxy group,

$C_7$-$C_{14}$ aralkyloxy group which is unsubstituted or optionally substituted with $R^{11}$, $C_6$-$C_{14}$ heteroaralkyloxy group which is unsubstituted or optionally substituted with $R^{11}$, $C_6$-$C_{12}$ aryloxy group which is unsubstituted or optionally substituted with R 11, $C_5$-$C_{12}$ heteroaryloxy group which is unsubstituted or optionally substituted with R 11, sulfanyl group, pentahalosulfanyl group, $C_1$-$C_6$ alkylthio group, $C_1$-$C_6$ alkylsulfinyl group, $C_1$-$C_6$ alkylsulfonyl group, $C_1$-$C_6$ haloalkylthio group, $C_1$-$C_6$ haloalkylsulfinyl group, $C_1$-$C_6$ haloalkylsulfonyl group, $C_3$-$C_6$ cycloalkylthio group, $C_3$-$C_6$ cycloalkylsulfinyl group, $C_3$-$C_6$ cycloalkylsulfonyl group, $C_3$-$C_6$ halocycloalkylthio group, $C_3$-$C_6$ halocycloalkylsulfinyl group, $C_3$-$C_6$ halocycloalkylsulfonyl group, $C_3$-$C_6$ cycloalkyl $C_1$-$C_6$ alkylthio group, $C_3$-$C_6$ cycloalkyl $C_1$-$C_6$ alkylsulfinyl group, $C_3$-$C_6$ cycloalkyl $C_1$-$C_6$ alkylsulfonyl group, $C_3$-$C_6$ halocycloalkyl $C_1$-$C_6$ alkylthio group, $C_3$-$C_6$ halocycloalkyl $C_1$-$C_6$ alkylsulfinyl group, $C_3$-$C_6$ halocycloalkyl $C_1$-$C_6$ alkylsulfonyl group, group-S ($R^{12}$) = NH, group-S ($R^{12}$) = N-C=N, group-S ($R^{12}$) = N-C (= O) $R^{13}$, group-S ($R^{12}$) (= O) = NH, group-S ($R^{12}$) (= O) = N-C=N, group-S ($R^{12}$) (= O) = N-C (= O) $R^{13}$, $C_6$-$C_{12}$ arylthio group, $C_6$-$C_{12}$ arylsulfinyl group, $C_6$-$C_{12}$ arylsulfonyl group, $C_5$-$C_{12}$ heteroarylthio group, $C_5$-$C_{12}$ heteroarylsulfinyl group, $C_5$-$C1_2$ heteroarylsulfonyl group, amino group, mono(Ci-$C_6$ alkyl)amino group, di($C_1$-$C_6$ alkyl) amino group, (di($C_1$-$C_6$ alkyl)sulfinylidene)amino group, di ($C_1$-$C_6$ alkyl) phosphoryl group, $C_1$-$C_7$ acyl group, $C_1$-$C_7$ haloacyl group, hydroxyimino $C_1$-$C_6$ alkyl group, hydroxyimino(amino) $C_1$-$C_7$ alkyl group, $C_1$-$C_6$ alkoxyimino $C_1$-$C_6$ alkyl group, $C_1$-$C_6$ haloalkoxyimino $C_1$-$C_6$ alkyl group, carboxyl group, $C_1$-$C_6$ alkoxycarbonyl group, aminocarbonyl group, mono($C_1$-$C_6$ alkyl)aminocarbonyl group, di($C_1$-$C_6$ alkyl)aminocarbonyl group, mono($C_1$-$C_6$ haloalkyl)aminocarbonyl group, mono($C_3$-$C_6$ cycloalkyl)aminocarbonyl group, mono (cyano $C_3$-$C_6$ cycloalkyl) aminocarbonyl group, mono ($C_1$-$C_6$ alkoxyimino $C_1$-$C_6$ alkyl) aminocarbonyl group, di ($C_1$-$C_6$ alkyl) aminomethylidene aminocarbonyl group, aminothiocarbonyl group, mono($C_1$-$C_6$ alkyl)aminothiocarbonyl group, di($C_1$-$C_6$ alkyl)aminothiocarbonyl group, or N-($C_1$-$C_6$ alkoxy)-N-($C_1$-$C_6$ alkyl)aminocarbonyl group,

$R^{10}$ represents a hydrogen atom, $C_1$-$C_{12}$ alkyl group, $C_1$-$C_6$ alkoxy $C_1$-$C_6$ alkyl group, $C_1$-$C_{12}$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_2$-$C_6$ alkenyl group, $C_2$-$C_6$ haloalkenyl group, $C_2$-$C_6$ alkynyl group, $C_3$-$C_6$ cycloalkyl $C_2$-$C_6$ alkynyl group, $C_2$-$C_6$ haloalkynyl group, $C_3$-$C_6$ cycloalkyl group, $C_3$-$C_6$halocycloalkyl group, $C_3$-$C_6$ cycloalkyl $C_1$-$C_6$ alkyl group, $C_3$-$C_6$ halocycloalkyl $C_1$-$C_6$ alkyl group, heterocyclyl $C_1$-$C_6$ alkyl group which is unsubstituted or optionally substituted with $R^{11}$, $C_7$-$C_{14}$ aralkyl group which is unsubstituted or optionally substituted with $R^{11}$, $C_6$-$C_{14}$ heteroaralkyl group which is unsubstituted or optionally substituted with $R^{11}$, $C_6$-$C_{12}$ aryl group which is unsubstituted or optionally substituted with R 11, or $C_5$-$C_{12}$ heteroaryl group which is unsubstituted or optionally substituted with R 11, and

$R^{11}$ represents a halogen atom, azido group, cyano group, nitro group, $C_1$-$C_6$ alkyl group, $C_1$-$C_6$ haloalkyl group, $C_1$-$C_6$ alkoxy group, $C_1$-$C_6$ haloalkoxy group, $C_3$-$C_6$ cycloalkyl group, pentahalosulfanyl group, $C_1$-$C_6$ alkylthio group, $C_1$-$C_6$ alkylsulfinyl group, $C_1$-$C_6$ alkylsulfonyl group, $C_1$-$C_6$ haloalkylthio group, $C_1$-$C_6$ haloalkylsulfinyl group, $C_1$-$C_6$ haloalkylsulfonyl group, amino group, mono ($C_1$-$C_6$ alkyl) amino group, or di($C_1$-$C_6$ alkyl) amino group,

$R^{12}$ and $R^{13}$ independently represent a $C_1$-$C_6$ alkyl group, $C_1$-$C_6$ alkoxy $C_1$-$C_6$ alkyl group, $C_1$-$C_6$ haloalkyl group, $C_1$-$C_6$ alkoxy $C_1$-$C_6$ haloalkyl group, $C_1$-$C_6$ haloalkoxy $C_1$-$C_6$ alkyl group, $C_1$-$C_6$ haloalkoxy $C_1$-$C_6$ haloalkyl group, $C_3$-$C_6$ cycloalkyl group, $C_3$-$C_6$ halocycloalkyl group, $C_3$-$C_6$ cycloalkyl $C_1$-$C_6$ alkyl group, $C_3$-$C_6$ halocycloalkyl $C_1$-$C_6$ alkyl group, cyano $C_1$-$C_6$ alkyl group, $C_7$-$C_{14}$ aralkyl group which is unsubstituted or optionally substituted by $R^{11}$, or $C_6$-$C_{14}$ heteroaralkyl group which ais unsubstituted or optionally substituted by $R^{11}$.

**2.** The compound or salt thereof according to claim 1, wherein $A^7$ represents a nitrogen atom, and each of $A^8$ and $A^9$ represents a carbon atom substituted with $R^8$ and $R^9$, respectively.

**3.** The compound or salt thereof according to claim 1, wherein $A^1$ represents a bond,

each of $A^2$ and $A^7$ represents a nitrogen atom,
$W^1$ represents a nitrogen atom,
$W^2$ represents a carbon atom, and
each of $A^3$, $A^4$, $A^5$, $A^6$, $A^8$ and $A^9$ represents a carbon atom substituted with $R^3$, $R^4$, $R^5$, $R^6$, $R^8$, and $R^9$ respectively.

**4.** The compound or salt thereof according to claim 3, wherein each of $R^3$, $R^4$, $R^5$, $R^6$, $R^8$ and $R^9$ independently represents a hydrogen atom, halogen atom, $C_1$-$C_6$ alkyl group, $C_1$-$C_6$ haloalkyl group, cyano $C_3$-$C_6$ cycloalkyl group, $C_1$-$C_6$ haloalkoxy group, or cyano $C_1$-$C_6$ alkoxy group.

**5.** The compound or salt thereof according to any one of claims 1 to 4, wherein $R^2$ represents a $C_1$-$C_6$ alkyl group.

**6.** The compound or salt thereof according to any one of claims 1 to 5, wherein $R^{10}$ represents a $C_1$-$C_{12}$ haloalkyl group.

**7.** The compound or salt thereof according to claim 1, wherein the compound represented by the general formula [I] is a compound represented by the general formula [Ia],

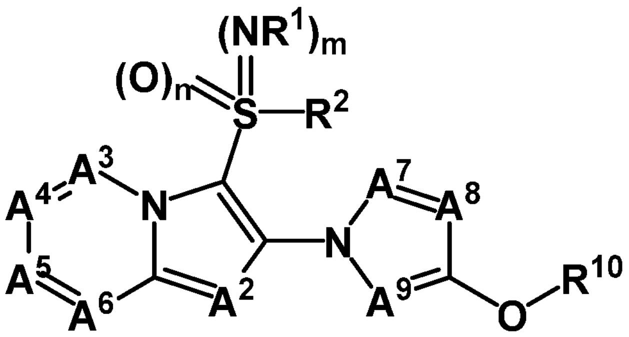

[ Ia ]

wherein $A^2$, $A^3$, $A^4$, $A^5$, $A^6$, $A^7$, $A^8$, $A^9$, m, n, $R^1$, $R^2$ and $R^{10}$ have the same meaning as defined in claim 1.

**8.** The compound or salt thereof according to claim 1, wherein the compound represented by the general formula [I] is a compound represented by the general formula (A),

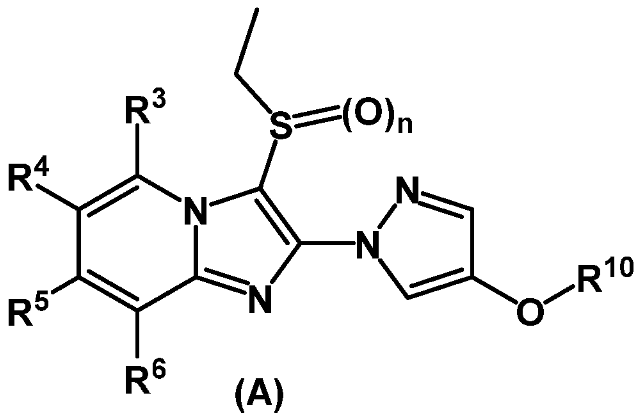

(A)

wherein n, $R^3$, $R^4$, $R^5$, $R^6$ and $R^{10}$ have the same meaning as defined in claim 1.

**9.** The compound or salt thereof according to claim 8, wherein n is 2, each of $R^3$, $R^5$ and $R^6$ is a hydrogen atom, $R^4$ is a $C_1$-$C_6$ haloalkyl group, cyano $C_3$-$C_6$ cycloalkyl group, $C_1$-$C_6$ haloalkoxy group, or cyano $C_1$-$C_6$ alkoxy group, and $R^{10}$ is a $C_1$-$C_{12}$ haloalkyl group.

**10.** The compound or salt thereof according to claim 1, wherein the compound represented by the general formula [I] is a compound represented by the general formula (B),

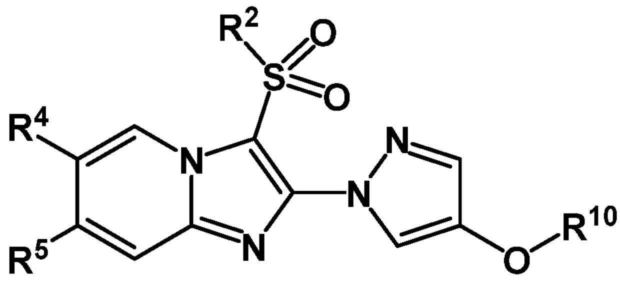

(B)

wherein $R^2$, $R^4$, $R^5$ and $R^{10}$ have the same meaning as defined in claim 1.

**11.** The compound or salt thereof according to claim 1, wherein the compound represented by the general formula [I] is a compound represented by the general formula (C),

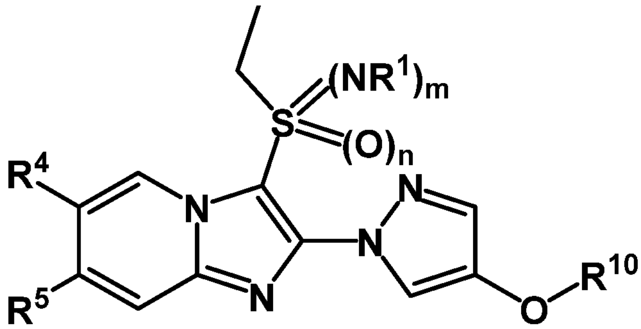

(C)

wherein m, n, $R^1$, $R^4$, $R^5$ and $R^{10}$ have the same meaning as defined in claim 1.

**12.** The compound or salt thereof according to claim 1, wherein the compound represented by the general formula [I] is a compound represented by the general formula (D),

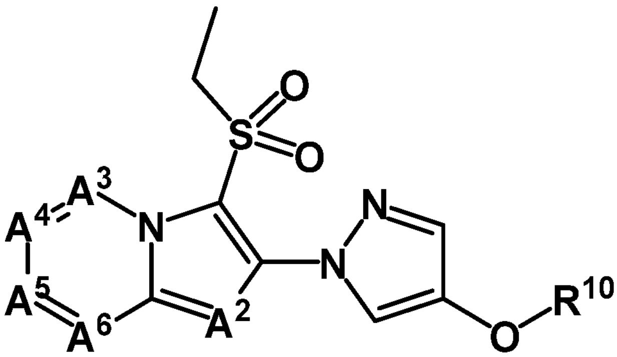

(D)

wherein $A^2$, $A^3$, $A^4$, $A^5$, $A^6$ and $R^{10}$ have the same meaning as defined inclaim 1.

**13.** The compound or salt thereof according to claim 12, wherein $A^2$ represents a nitrogen atom or a carbon atom substituted with $R^b$, $A^3$ represents a nitrogen atom or a carbon atom substituted with $R^3$, and each of $A^4$, $A^5$ and $A^6$

independently represents a nitrogen atom or a carbon atom substituted with $R^4$, $R^5$ and $R^6$, respectively.

**14.** A pesticide composition comprising the compound or salt thereof according to any one of claims 1 to 13 as an active ingredient.

**15.** The pesticide composition according to claim 14, wherein the pesticide composition further comprisess a surfactant.

**16.** A pest control agent comprising the compound or salt thereof according to any one of claims 1 to 13 as an active ingredient.

**17.** The pest control agent according to claim 16, wherein the pest control agent is an insecticide, a nematicide, or an acaricide.

**18.** The pest control agent according to claim 17, which has control effects against pests in paddy fields, farmland, turf, orchards, non-agricultural land, greenhouses, nursery facilities, and plant factories for cultivating agricultural and horticultural plants.

**19.** The pest control agent according to claim 18, wherein the agricultural and horticultural plants are plants to which resistance has been given by breeding methods or gene recombination techniques.

**20.** A method for controlling pests using an effective amount of the compound or salt thereof according to any one of claims 1 to 13.

**21.** A method for controlling pests by allowing a pesticide composition comprising the compound or salt thereof according to any one of claims 1 to 13 as an active ingredient to act at the same time or in a divided manner on places where agricultural or horticultural crops are to be grown or growing.

**22.** The method for controlling pests according to claim 20 or 21, wherein the places where the pest control agents are applied are paddy fields, farmland, turf, orchards, non-agricultural land, greenhouses, nursery facilities, or plant factories.

**23.** The method for controlling pests according to any one of claims 20 to 22, wherein the compound or salt thereof according to any one of claims 1 to 13 is used as an insecticide, a nematicide, or an acaricide.

**24.** A use of the pest control agent according to any one of claims 16 to 19 for controlling pests on agricultural and horticultural crops.

**25.** The compound represented by the general formula [I] or salt thereof according to claim 1, wherein $R^{10}$ is a hydrogen atom in the general formula [I].

**26.** A method for producing the compound represented by the general formula [I] according to claim 1, comprising using the compound or salt thereof according to claim 25 as an intermediate.

**INTERNATIONAL SEARCH REPORT**

International application No.
**PCT/JP2022/016390**

**A. CLASSIFICATION OF SUBJECT MATTER**

***C07D 471/04***(2006.01)i; ***A01P 7/04***(2006.01)i; ***C07D 487/04***(2006.01)i; ***A01N 43/90***(2006.01)i; ***A01N 47/02***(2006.01)i
FI: C07D471/04 104Z; A01N43/90 103; A01N47/02; A01N43/90 104; A01N43/90 102; A01P7/04; C07D471/04 CSP; C07D487/04 144; C07D487/04 140; C07D471/04 108Q

According to International Patent Classification (IPC) or to both national classification and IPC

**B. FIELDS SEARCHED**

Minimum documentation searched (classification system followed by classification symbols)

C07D471/04; A01P7/04; C07D487/04; A01N43/90; A01N47/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2021-024859 A (NIPPON SODA CO., LTD.) 22 February 2021 (2021-02-22) | 1-26 |
| A | JP 2020-079324 A (SUMITOMO CHEMICAL CO., LTD.) 28 May 2020 (2020-05-28) | 1-26 |
| A | JP 2020-105188 A (SUMITOMO CHEMICAL CO., LTD.) 09 July 2020 (2020-07-09) | 1-26 |
| P, A | WO 2021/085370 A1 (KUMIAI CHEMICAL INDUSTRY CO., LTD.) 06 May 2021 (2021-05-06) | 1-26 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 June 2022** | **21 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/016390**

| Patent document cited in search report | Publication date (day/month/year) | Patent family member(s) | Publication date (day/month/year) |
|---|---|---|---|
| JP 2021-024859 A | 22 February 2021 | (Family: none) | |
| JP 2020-079324 A | 28 May 2020 | (Family: none) | |
| JP 2020-105188 A | 09 July 2020 | (Family: none) | |
| WO 2021/085370 A1 | 06 May 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017061497 A
- WO 2018052119 A
- WO 2019124548 A
- WO 2016204270 A
- JP 2021024859 A
- WO 03048081 A
- WO 2018228986 A
- WO 2020114934 A
- WO 2014064094 A
- WO 2015162164 A
- WO 2017178582 A
- WO 2018015476 A
- WO 2015059262 A
- WO 2009144395 A


### Non-patent literature cited in the description


- *CHEMICAL ABSTRACTS,* 1472052-11-1
- *CHEMICAL ABSTRACTS,* 1472050-34-2
- *CHEMICAL ABSTRACTS,* 1448758-62-0
- *CHEMICAL ABSTRACTS,* 1448761-28-1
- *CHEMICAL ABSTRACTS,* 1472047-71-4
- *CHEMICAL ABSTRACTS,* 2266183-40-6
- *CHEMICAL ABSTRACTS,* 1606999-43-2
- *CHEMICAL ABSTRACTS,* 2266190-06-9
- *CHEMICAL ABSTRACTS,* 2266164-36-5
- *CHEMICAL ABSTRACTS,* 2266170-31-2
- *CHEMICAL ABSTRACTS,* 2138855-12-4
- *CHEMICAL ABSTRACTS,* 1708087-22-2
- *CHEMICAL ABSTRACTS,* 182807-75-7
- *CHEMICAL ABSTRACTS,* 1606999-21-6
- *CHEMICAL ABSTRACTS,* 2266292-43-5
- *CHEMICAL ABSTRACTS,* 1607001-97-7
- *CHEMICAL ABSTRACTS,* 1083-55-2
- *CHEMICAL ABSTRACTS,* 1231214-23-5
- **RYO OSAWA ; HIROSHI EGAMI.** Let's Understand New Plant Breeding Technology. International Literature Company
- **TAPAN KUMAR MOHANTA ; TUFAIL BASHIR ; ABEER HASHEM ; ELSAYED FATHI ABD_ALLAH ; HANHONG BAE.** Genome Editing Tools in Plants. *Genes,* 2017, vol. 8, 399